(19) Europäisches Patentamt
European Patent Office
Office européen des brevets

(11) Numéro de publication: **0 420 716 A2**

(12) # DEMANDE DE BREVET EUROPEEN

(21) Numéro de dépôt: **90402555.8**

(22) Date de dépôt: **17.09.90**

(51) Int. Cl.5: **C07D 471/06**, C07D 487/06, A61K 31/55, //(C07D471/06, 243:00,221:00),(C07D487/06, 243:00,209:00),(C07D487/06, 243:00,223:00)

(30) Priorité: **28.09.89 FR 8912700**

(43) Date de publication de la demande: **03.04.91 Bulletin 91/14**

(84) Etats contractants désignés: **AT BE CH DE DK ES FR GB GR IT LI LU NL SE**

(71) Demandeur: **JOUVEINAL S.A.**
**Tour Maine Montparnasse 33 Avenue du Maine**
**F-75755 Paris Cedex 15(FR)**

(72) Inventeur: **Calvet, Alain Pierre**
**Résidence Ronsard, 3, rue de Gatine**
**F-94240 L'Hay Les Roses(FR)**
Inventeur: **Junien, Jean-Louis**
**36 Avenue Eiffel**
**F-92310 Sèvres(FR)**
Inventeur: **Pascal, Yves Robert Alain**
**16, rue Georges Tournier**
**F-92500 Reuil Malmaison(FR)**
Inventeur: **Pascaud, Xavier Bernard Louis**
**41, rue de Charenton**
**F-75012 Paris(FR)**
Inventeur: **Roman, François Joseph**
**11 Allée Pierre Fresnay**
**F-94400 Vitry Sur Seine(FR)**

(74) Mandataire: **Bourgognon, Jean-Marie et al**
**Cabinet Flechner 22, Avenue de Friedland**
**F-75008 Paris(FR)**

(54) **Benzodiazépines, leur procédé et intermédiaires de préparation et leur application en thérapeutique.**

(57) Benzodiazépines de formule

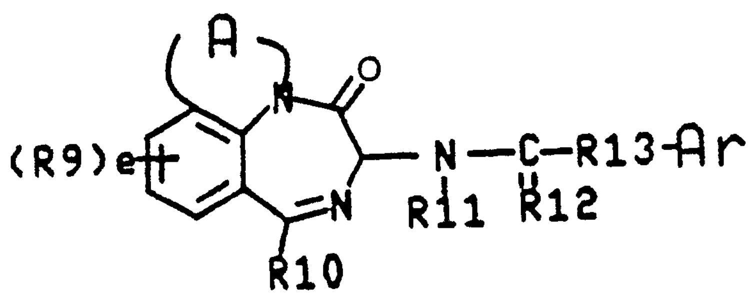

(I)

dans laquelle
R9 est hydrogène ou chlore, R10 est phényle éventuellement substitué, R11 est hydrogène, R12 est oxygène ou soufre, R13 est un groupe NH, Ar est aromatique ou hétéroaromatique comportant un ou deux cycles et dans laquelle, notamment, A est un pont éthylène ou triméthylène ; e est égal à 1 ou 2 ; qui sont antagonistes de la gastrine et ou antagonistes centraux de la cholecystokinine.

EP 0 420 716 A2

# BENZODIAZÉPINES LEUR PROCÉDÉ ET INTERMÉDIAIRES DE PRÉPARATION ET LEUR APPLICATION EN THÉRAPEUTIQUE.

La présente invention est relative aux benzodiazépines, à leur procédé et intermédiaires de préparation et à leur application en thérapeutique.

La cholécystokinine, abrégée ci-dessous en CCK, est un peptide comportant trente-trois aminoacides dans la forme isolée à l'origine. Mais il circule dans l'organisme des formes actives comportant trente-neuf, douze et huit aminoacides. La forme comportant les huit aminoacides de l'extrémité acide carboxylique du peptide est le plus court enchaînement d'aminoacides présentant l'activité. Elle est désignée ci-dessous par les abréviations CCK-8, CCK-8 SO4 ou CCK-8 sulfatée ; ces deux dernières abréviations signifiant que le groupe phénol de la tyrosine en position 27 de la cholecystokinine est estérifié par un groupe -SO3H, ce qui est le cas dans la forme naturelle. Des études biochimiques ont mis en évidence l'existence de deux types de récepteurs de la cholecystokinine dans l'organisme des mammifères, le premier ci-après dénommé récepteur CCK A présentant une localisation préférentiellement périphérique et le second ci-après dénommé CCK B présentant une localisation préférentiellement centrale. D'autre part, la gastrine qui est une hormone intervenant dans la régulation du système gastro-intestinal, présente des similitudes de structure avec la cholecystokinine.

La demande de brevet européen N° 88302141.2 déposée par la société Merck & co décrit des benzodiazépines de formule :

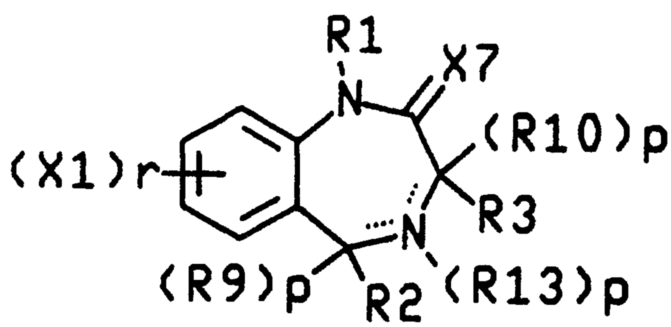

(XX)

notée dans le présent mémoire formule (XX),
dans laquelle :
R1 est hydrogène, alkyle inférieur linéaire ou ramifié, alcènyle inférieur, alcynyle inférieur, -X12-COOR6, -X11-cycloalkyle inférieur, -X12-nR4R5, -X12-C(O)-NR4R5, -X12-CN ou X11-C(X10)3
R2 est hydrogène ; phényle éventuellement substitué par un ou deux groupes choisis parmi halogène, alkyle inférieur, alkyloxy inférieur, alkylthio inférieur, carboxyle, alkyloxycarbonyle inférieur, nitro, trifluorométhyle ou hydroxy ; 2-, 3-, 4-pyridyle ;

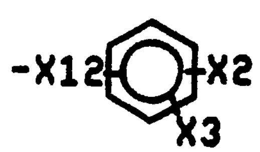

, -X12-SCH3

-X12-SOCH3 , -X12-SO2CH3 ou -X12-COOR-

R3 est -X11NR18(CH2)qR16, -X11NR18C(=O)X11R7, -NH(CH2)3NHR7, -NH(CH2)3NHCOR7, -X11C(=O)-X9X11R7, -X11X9C(=O)C(CH6CH2R7)-NHCOOR14, -X11NR18C(=O)-X9aX11R7, -X11X9C(=O)-CH(NH2)-CH2R7,

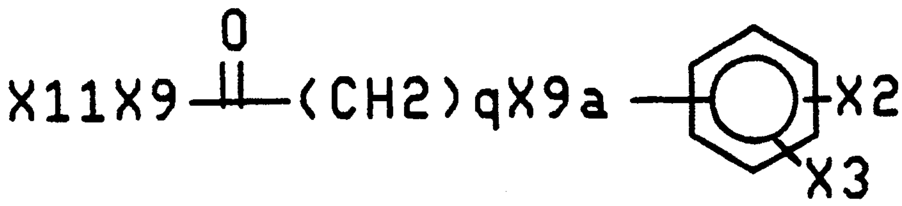

-X11NR12SO2(CH2)qR7 ou -X11C(=O)R7, sous réserve que R10 n'est ni hydrogène ni méthyle lorsque R3 est -X11COR7.

R4 et R5 sont indépendamment égaux à R6 ou forment avec l'atome d'azote du groupe NR4R5 un hétérocycle de 4 à 7 chaînons non substitué ou mono ou disubstitué saturé ou insaturé ; un hétérocycle de 4 à 7 chainons condensé avec un cycle benzène ou l'un des deux systèmes décrits ci-dessus comprenant en plus un second hétéroatome choisi entre 0 et NCH3 et dont le ou les substituants sont des alkyles inférieurs.

R6 est hydrogène, alkyle ou cycloalkyle inférieur, phényle éventuellement substitué ou phényl-alkyle inférieur éventuellement substitué, les substituants sur le phényle ou le phényl-alkyle inférieur pouvant être au nombre de 1 ou 2 et choisis parmi halogène, alkyle inférieur, alkyloxy inférieur, nitro ou trifluorométhyle.

R7 est alpha ou béta naphtyle ; phényle éventuellement mono ou di substitué par halogène, nitro,hydroxy, -X11NR4R5, alkyle inférieur, CF3, CN, SCF3, CH=CH, CH2SCF3, OC(=O)CH3, OCHF2, SH, SPh, PO3H, alkyloxy inférieur, alkylthio inférieur ou COOH ; 2-, 3-,4-pyridyle,

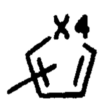
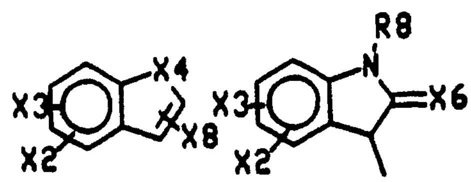

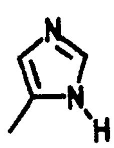
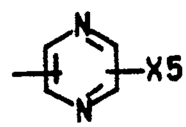
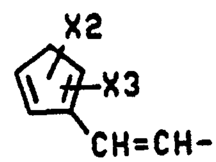

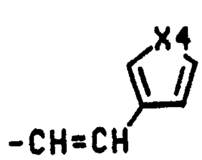
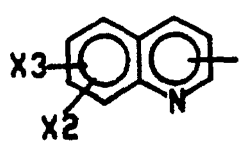
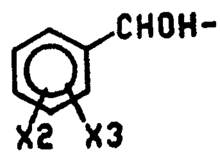

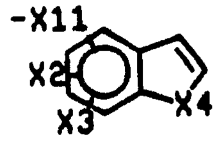

R8 est hydrogène, alkyle inférieur, cycloalkyle inférieur, -X12CONH2, -X12COOR6, -X12-cycloalkyle inférieur, -X12NR4R5, -COCH(NH2)-CH2R12, -C-=O)-CH (CH2R12)-NHCOOR11,

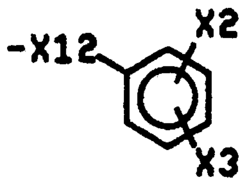

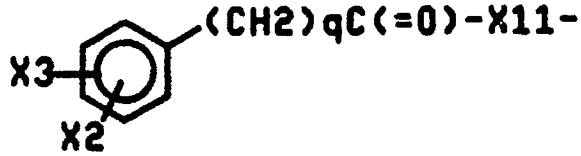

R9 et R10 sont indépendamment H, OH ou CH3.

R11 et R12 sont indépendamment alkyle inférieur ou cycloalkyle inférieur.

R13 est H, O, alkyle inférieur, cycloalkyle inférieur ou acyle.

R14 est alkyle inférieur ou phénylalkyle inférieur.

R15 est H, -NH2, alkyle inférieur ou

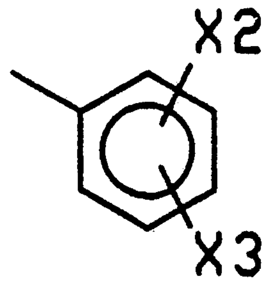

R16 est alpha ou béta naphtyle ou 2-indolyle.

R18 est H ou alkyle inférieur.

p est zéro quand la double liaison optionnelle adjacente est insaturée et un quand elle est saturée sauf quand R13 est 0, auquel cas p = 1 et la double liaison optionnelle adjacente est insaturée.

q est 1-4.

r est 1 ou 2.

X1 est H, -NO2, CF3, CN, OH, halogène,alkyle inférieur,alkoxy inférieur, alkylthio inférieur, -X11COOR6 ou -X11NR4R5.

X2 et X3 sont indépendamment H, -NO2, OH, halogène, alkyle inférieur, alkoxy inférieur, alkylthio inférieur.

X4 est S, O, CH2 ou NR8.

X5 est H, CF3, CN, -COOR6, -NO2 ou halogène.

X6 est O ou HH.

X7 est O, S, HH ou NR15 sous réserve que X7 ne peut être NR15 que lorsque R1 n'est pas H.

X8 est H ou alkyle inférieur.

X9 et X9a sont indépendamment NR18 ou 0.

X10 est F, Cl ou Br.

X11 est absent ou alkylidène linéaire ou ramifié comportant 1 à 4 atomes de carbone.

X12 est alkylidène linéaire ou ramifié comportant 1 à 4 atome de carbone.

sous réserve que lorsque (X1)r est un chlore en position 7, R1 est H et R2 est phényle non substitué alors R3 n'est pas NHC(O)-(CH2)2-C6H5 ou NHC(O)-C6H5.

En outre, la préparation et les propriétés pharmacologiques de ces benzodiazépines ont été publiées aux références d'articles indiqués ci-dessous :

J. Med. Chem. 1989, 32, 13-16

J. Med. Chem. 1988, 31, 2235-2246

European Journal of Pharmacology 162 (1989) 273-280

Ces publications mettent notamment en évidence un composé énantiomère de formule (XXa)

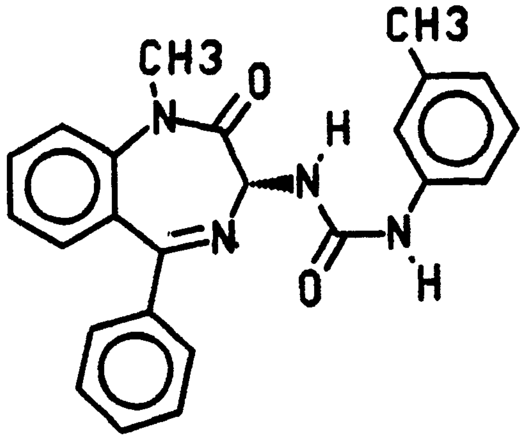

(XXa)

pour lequel "in vitro" il est montré une affinité sélective aussi bien pour les récepteurs B de la cholecystokinine que pour les récepteurs de la gastrine par rapport aux récepteurs A de la cholecystokinine. Les résultats présentés montrent que le rapport d'activité CCK-A/gastrine est de 290/1,9 soit environ 150 fois favorable à l'activité du composé sur les récepteurs de la gastrine. La demanderesse a reproduit ces expériences et a trouvé un rapport de 1419/4,7 soit environ 300 fois favorable à l'activité sur les récepteurs de la gastrine.

Par ailleurs, à la demande de brevet européen n° 89116504.5 déposée par la société Fujisawa le 07.09.89 et publiée postérieurement à la date de priorité dont bénéficie la présente demande, il est décrit des composés tricycliques d'activité antagoniste de la cholecystokinine (CCK) sans autre présicion de leur spécificité d'action sur les récepteurs précédemment précisés.

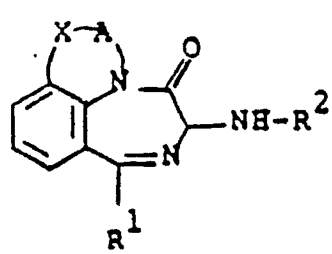

dans laquelle,
R1 est aryle éventuellement substitué,
X représente -O- ou -CH(R3)- dans lequel R3 est l'hydrogène ou alkyle inférieur,
A est une liaison de valence ou alkylène inférieur pouvant comporter un ou plusieurs groupes alkyle inférieur,
R2 est l'hydrogène ou un groupe acyle.

Or, on a maintenant trouvé des benzodiazépines qui, même sous forme racémique, présentent à la fois une activité comparable ou meilleure que celle de l'art antérieur sur les récepteurs de la gastrine et/ou sur les récepteurs B de la cholecystokinine et ont pour certains composés une activité notablement plus faible sur les récepteurs A de la cholecystokinine. Cette propriété permet l'utilisation des produits de l'invention dans le traitement des maladies dépendant du récepteur central de la cholecystokinine ou du récepteur de la gastrine avec peu ou pas d'effet secondaire sur les systèmes dépendant du récepteur périphérique de la cholecystokinine, et ceci chez les mammifères et en particulier chez l'homme. C'est ainsi notamment que les benzodiazépines de l'invention permettent d'inhiber la sécrétion gastrique sans provoquer à titre d'effet secondaire d'augmentation de l'évacuation gastrique. Les produits de l'invention peuvent donc être utile dans le traitement de la douleur, l'hyperplasie du pancréas et du colon, le syndrome de Zollinger-Ellison, les ulcères gastro-duodénaux et plus généralement dans les phénomènes régulés par la gastrine, notamment la motricité digestive.

L'invention a donc pour objet des benzodiazépines de formule (I) :

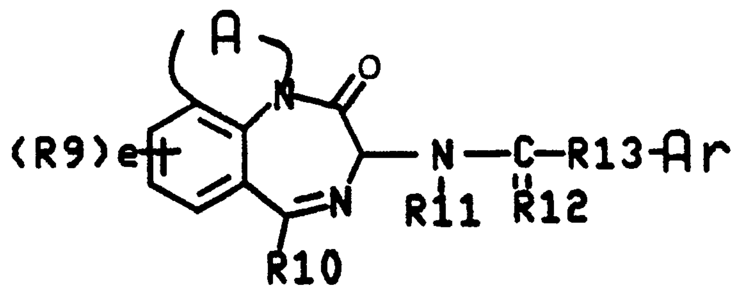

(I)

Dans laquelle,

R9 est hydrogène, nitro, halogène, cyano, trifluorométhyle, hydroxy, alkyle inférieur, alkoxy inférieur, -COOR20, -N(R21)-R22

, R10 est un phényle éventuellement mono à tri substitué par des groupes identiques ou différents choisis parmi halogène, alkyle inférieur, alkoxy inférieur, cyano, nitro ou trifluorométhyle ;

R11 est hydrogène ou alkyle inférieur ;

R12 est un atome d'oxygène ou un atome de soufre ;

R13 est un groupe -N(R24)- ;

R20 est hydrogène ou alkyle inférieur ;

R21 et R22 sont identiques ou différents et peuvent être l'hydrogène ou un groupe alkyle inférieur;

R24 est hydrogène ou alkyle inférieur ;

Ar est un groupe aromatique hydrocarboné ou hétéroaromatique, comportant un cycle ou deux cycles condensés, chacun des cycles comportant de 5 à 7 atomes parmi lesquels figurent de zéro à deux hétéroatomes d'azote ; ce groupe aromatique ou hétéro aromatique étant éventuellement mono ou di substitué par des groupes identiques ou différents choisis parmi alkyle inférieur, carboxyle, alkoxycarbonyle inférieur, sulfonyle, sulfamoyle, cyano, nitro, trifluorométhyle ou, sous réserve que R12 soit un atome de soufre, par des groupes halogène ou alkoxy inférieur;

A est égal à [-CH(R1)-]a dans lequel R1 est hydrogène ou alkyle inférieur;

a est égal à deux, trois ou quatre ;

e est égal à un ou deux ;

Dans ce qui précède ainsi que dans ce qui suit :

- par halogène on entend le fluor, le chlore, le brome ou l'iode ;

- par pseudohalogène on entend les groupes qui réagissent de manière analogue aux halogènes notamment dans les réactions de substitutions nucléophiles où ils se comportent comme des groupes nucléofuges. Des exemples de tels groupes sont le méthanesulfonate, le benzènesulfonate, le paratoluènesulfonate, l'azoture.

- par alkyle inférieur on entend les groupes alkyles linéaires ou ramifiés comportant de un à sept atomes de carbone comme par exemple : méthyle, éthyle, propyle, isopropyle, butyle, isobutyle, tertiobutyle, et les divers isomères des groupes pentyle, hexyle et heptyle.

- par alkoxy inférieur on entend un groupe (-O-Alkyle) dans lequel le groupe alkyle est alkyle inférieur tel que défini ci-dessus.

- par alkoxycarbonyle inférieur on entend un groupe (Alkyle-O-C(=O)-) dans lequel le groupe alkyle est un groupe alkyle inférieur tel que défini ci-dessus.

L'invention comprend les benzodiazépines de formule (I), sous forme racémique aussi bien que sous forme d'énantiomères purs.

L'invention comprend également les sels pharmacologiquement acceptables des composés de formule (I) lorsque ceux-ci présentent une portion basique ou acide.

Par sel pharmacologiquement acceptable d'un composé de formule (I) présentant une portion basique on entend les sels d'addition des composés de formule (I) que l'on forme à partir d'acides minéraux ou organiques non toxiques comme par exemple les sels d'acides bromhydrique, chlorhydrique, sulfurique, sulfamique, phosphorique, nitrique, acétique, propionique, succinique, glycolique, stéarique, lactique, malique, tartrique, citrique, mucique, ascorbique, pamoïque, maléique, hydroxymaléique, phénylacétique, glutamique, benzoïque, salicylique, sulfanilique, acétoxybenzoïque, fumarique, toluènesulfonique, éthanedisulfonique, oxalique, isethionique et autres. Les divers sels d'ammonium quaternaires des dérivés (I) sont également inclus dans cette catégorie des composés de l'invention.

Par sel pharmacologiquement acceptable d'un composé de formule (I) présentant une portion acide on entend les sels usuels des composés de formule (I) que l'on forme à partir de bases minérales ou organiques non toxiques comme par exemple les hydroxydes des métaux alcalins et alcalino-terreux

(lithium, sodium, potassium, magnésium et calcium), les amines (dibenzyléthylènediamine, triméthylamine, piperidine, pyrrolidine, benzylamine et autres) ou encore les hydroxydes d'ammoniums quaternaires comme l'hydroxyde de tétraméthylammonium.

Parmi ces benzodiazépines nouvelles, on préfère celles dans lesquelles R12 est atome de soufre. Egalement on préfère lorsque le groupe Ar comporte un seul cycle qu'il soit un groupe benzène substitué par un méthyle, et, sous réserve que R12 soit le soufre on préfère également que ce groupe benzène soit substitué par un atome de brome. Lorsque le groupe Ar comporte deux cycles, on préfère que ce soit le naphtalène et, lorsque Ar comporte deux cycles dont un est hétérocycle on préfère que celui-ci ne comporte qu'un atome d'azote et dans ce cas, on préfère tout particulièrement les benzodiazépines suivant l'invention dans lesquelles le groupe Ar est la quinoléine.

Parmi ces benzodiazépines nouvelles on préfère celles dans lesquelles a est égal à 2 ou 3 et que R1 soit hydrogène pour que A soit un pont éthylène (-CH2-CH2-) ou triméthylène (-CH2-CH2-CH2-).

Parmi les benzodiazépines de l'invention on préfère également :

- celles dans lesquelles R9 est un atome d'hydrogène ou un atome de chlore en position para par rapport à l'atome d'azote commun au cycle diazépine et au cycle contenant le groupe A et celles dans lesquelles e = 1.
- celles dans lesquelles R10 est un groupe phényle, ou un groupe phényle substitué par un atome de fluor en position ortho, ou par un radical méthyle en position para par rapport à l'atome reliant R10 au cycle diazépine.
- celles dans lesquelles R11 est hydrogène.
- celles dans lesquelles R13 est N(R24), le groupe R24 préféré étant l'hydrogène.

La configuration absolue du carbone du cycle diazépine portant le substituant -N(R11)-C(=R12)-R13-Ar, est importante pour la sélectivité de la benzodiazépine de formule (I) pour l'un ou l'autre des récepteurs CCK-A, CCK-B, ou gastrine et de manière générale on préfère les isomères optiques des benzodiazépines de l'invention. En particulier si l'on désire un composé présentant une sélectivité pour le récepteur B de la cholecystokinine on préfère généralement l'isomère dont le carbone asymétrique du cycle diazépine présente la configuration absolue (R) selon la nomenclature de Cahn, Ingold et Prelog.

Plus particulièrement les benzodiazépines préférées sont :

La (4R,S)-N-(céto-3-phényl-6-tétrahydro-1,2,3,4-pyrrolo[3,2,1-j,k]benzodiazépin-[1,4]-yl-4)-N′-(méthyl-3-phényl-)-urée.

La (4R)-N-(céto-3-phényl-6-tétrahydro-1,2,3,4-pyrrolo[3,2,1-j,k]benzodiazépin-[1,4]-yl-4)-N′-(méthyl-3-phényl-)-urée.

La (5R,S)-N-(céto-4-phényl-7-hexahydro -1,2,3,3a,4,5-pyrido[3,2,1-j,k]benzodiazépin-[1,4]-yl-5)-N′-(méthyl-3-phényl-)-urée.

La (5R)-N-(céto-4-phényl-7-hexahydro-1,2,3,3a,4,5-pyrido[3,2,1-j,k]benzodiazépin-[1,4]-yl-5)-N′-(méthyl-3-phényl-)-urée.

La (5R,S)-N-(céto-4-phényl-7-hexahydro-1,2,3,3a,4,5-pyrido[3,2,1-j,k]benzodiazépin-[1,4]-yl-5)-N′-(nitro-4-phényl-)-urée et ses isomères .

La (5R,S)-N-(céto-4-phényl-7-hexahydro-1,2,3,3a,4,5-pyrido[3,2,1-j,k]benzodiazépin-[1,4]-yl-5)-N′-(bromo-4-phényl-)-thiourée et ses isomères .

La (5R,S)-N-(céto-4-phényl-7-hexahydro-1,2,3,3a,4,5-pyrido[3,2,1-j,k]benzodiazépin-[1,4]-yl-5)-N′-(naphtyl-1)-urée et ses isomères .

La (5R,S)-N-(céto-4-phényl-7-hexahydro-1,2,3,3a,4,5-pyrido[3,2,1-j,k]benzodiazépin-[1,4]-yl-5)-N′-(quinoléinyl-3)-urée et ses isomères .

L'invention vise également un procédé de préparation des benzodiazépines de formule (I), procédé illustré par les schémas 1 et 2 et qui consiste

a) Dans le cas d'une benzodiazépine de formule (I) sous forme racémique (R,S) :

i) dans le cas où R11 est l'hydrogène, R13 est NH et R12 est l'oxygène,

à faire réagir une amine racémique de formule (II) dans laquelle A, R9, R10 et e ont la signification définie plus haut avec un arylisocyanate de formule Ar-N=C=O, Ar ayant la signification définie plus haut pour obtenir une benzodiazépine racémique de formule (Ia), (schéma 1).

ii) dans le cas où R11 est l'hydrogène, R13 est N(R24), R24 étant alkyle inférieur et R12 est l'oxygène,

à faire réagir une amine racémique de formule (II) dans laquelle A, R9, R10 et e ont la signification définie plus haut avec un agent de protection des amines de formule (XII) : R25-CH2X, dans laquelle R25 est un benzène éventuellement mono di ou tri-substitué par des substituants identiques ou différents choisis parmi les halogènes et le groupe nitro et X est un halogène ou un groupe pseudohalogène qui ont la signification définie précédemment ou encore avec un agent de protection (XIIa) R25-CHO en opérant dans des conditions d'amination réductrice pour obtenir la benzylamine de formule (XIX) qui est mise à réagir avec

un arylisocyanate de formule Ar-N = C = 0 comme au paragraphe i) ci-dessus, pour obtenir l'urée de formule (XV).

L'urée de formule (XV) ainsi obtenue est alkylée avec un réactif de formule (XIV) : R24-R39 dans lequel R24 est alkyle inférieur et R39 est un halogène ou un pseudo halogène pour obtenir une urée tétrasubstituée de formule (XVI), puis à éliminer le groupe protecteur R25-CH2 par hydrogènolyse avec un réactif choisi parmi le sodium dans l'ammoniac liquide ou l'hydrogène en présence d'un catalyseur d'hydrogènation comme par exemple un métal finement divisé sur un support comme du carbone pour obtenir une benzodiazépine racémique de formule (Ib), (schéma 1).

iii) dans le cas où R11 est alkyle inférieur, R13 est NH et R12 est l'oxygène,

à faire réagir une amine racémique de formule (II) dans laquelle A, R9, R10 et e ont la signification définie plus haut avec un agent d'alkylation des amines de formule (XIII) : R11-R36 dans laquelle R11 est alkyle inférieur et R36 est un halogène ou un pseudo-halogène tels que précédemment définis, ou avec un aldéhyde de formule (XIIIa) R37-CHO dans laquelle R37 est l'hydrogène ou alkyle inférieur tel que (R37-CH2-) = (R11-) et dans des conditions d'amination réductrice c'est-à-dire en présence d'hydrogène et d'un catalyseur d'hydrogènation (homogène ou hétérogène) qui est choisi parmi les métaux nobles ou les oxydes de métaux nobles finement divisés et déposés sur un support finement divisé, les complexes solubles des métaux de transition ou en présence d'un agent réducteur comme le borohydrure de sodium ou et de préférence le cyanoborohydrure de sodium pour obtenir une amine de formule (IIa), que l'on fait réagir avec un arylisocyanate de formule Ar-N = C = O, comme au paragraphe i) ci-dessus pour obtenir une benzodiazépine racémique de formule (Ic), (schéma 1).

iv) dans le cas où R11 est alkyle inférieur, R13 est N-R24, R24 étant alkyle inférieur, et R12 est l'oxygène,

à faire réagir une benzodiazépine racémique de formule (Ic) dans laquelle A, R9, R10, R11, Ar et e ont la signification définie plus haut avec un réactif d'alkylation (XIV) pour obtenir une benzodiazépine racémique de formule (Id), (schéma 1).

v) dans le cas où R11 est l'hydrogène, R13 est NH et R12 est le soufre,

à faire réagir une amine racémique de formule (II) dans laquelle A, R9, R10 et e ont la signification définie plus haut avec un arylisothiocyanate de formule Ar-N = C = S, Ar ayant la signification définie plus haut pour obtenir une benzodiazépine racémique de formule (Ie), (schéma 2).

vi) dans le cas où R11 est l'hydrogène, R13 est N(R24), R24 étant alkyle inférieur et R12 est le soufre,

à faire réagir une benzylamine racémique de formule (XIX) dans laquelle A, R9, R10, R25 et e ont la signification définie plus haut avec un arylisothiocyanate de formule Ar-N = C = S, pour obtenir la thiourée de formule (XVII) qui est mise à réagir avec un réactif d'alkylation (XIV) défini plus haut pour obtenir une thiourée tétrasubstituée de formule (XVIII) puis à éliminer le groupe protecteur par hydrogènolyse par réaction avec le sodium dans l'ammoniac liquide pour obtenir une benzodiazépine racémique de formule (If), (schéma 2).

vii) dans le cas où R11 est alkyle inférieur, R13 est NH et R12 est le soufre,

à faire réagir une amine racémique de formule (IIa) dans laquelle A, R9, R10, R11 et e ont la signification définie plus haut avec un arylisothiocyanate de formule Ar-N = C = S, Ar ayant la signification définie plus haut pour obtenir une benzodiazépine racémique de formule (Ig), (schéma 2).

viii) dans le cas où R11 est alkyle inférieur, R13 est N-R24, R24 étant alkyle inférieur, et R12 est le soufre,

à faire réagir une benzodiazépine racémique de formule (Ig) dans laquelle A, R9, R10, R11, Ar et e ont la signification définie plus haut avec un réactif d'alkylation (XIV), pour obtenir une benzodiazépine racémique de formule (Ih), (schéma 2).

b) Dans le cas d'une benzodiazépine de formule (I) sous forme optiquement active (forme R ou S) :

à opérer avec des produits de départ (II) sous forme d'énantiomères appropriés et de manière identique à celle décrite dans chacun des huit cas précédents pour obtenir les énantiomères des benzodiazépines de formule (Ia), (Ib), (Ic), (Id), (Ie), (If), (Ig) ou (Ih).

Les sels pharmaceutiquement acceptables de la présente invention peuvent être préparés à partir des composés de formule (I) par des procédés classiques. Cette préparation consiste à faire réagir, d'une façon générale, la fonction réactive de la benzodiazépine de l'invention avec la quantité stoechiométrique ou un excès du réactif salifiant approprié dans un solvant ou un mélange de solvants.

## PREPARATION DES COMPOSES DE FORMULE I

La préparation des composés de formule (I) décrite ci-dessous se rapporte indifféremment aux

composés (I) racémiques ou à leurs énantiomères à partir d'amines de formule (II) ou (IIa) racémiques ou optiquement pures. Les synthèses sont résumées aux schémas 1 et 2 et détaillées ci-dessous. Les produits obtenus après réaction sont, si nécessaire, purifiés par chromatographie sur colonne de silice (chromatographie flash) ou par chromatographie liquide sous haute pression (HPLC) puis éventuellement par recristallisation.

i) Préparation des composés (Ia) et (Ic) (schéma 1) :

Dans le cas ou R13 est NH et R12 est un atome d'oxygène on peut notamment opérer de la manière suivante :

un composé de formule (II) ou (IIa) est dissout dans 5 à 50 volumes d'un solvant organique anhydre comme par exemple un hydrocarbure chloré comme du dichlorométhane ou du chloroforme, un éther-oxyde linéaire ou cyclique comme du diméthoxy-1,2-éthane, du tétrahydrofurane ou du dioxane, un solvant polaire aprotique tel que de la pyridine, du diméthylsulfoxyde ou du diméthylformamide ou tout autre solvant convenable pour y effectuer une réaction de condensation ou encore un mélange approprié de deux ou plusieurs de ces solvants et on y ajoute un équivalent d'un agent pouvant réagir avec la fonction amine du composé de formule (II) pour donner une urée, comme par exemple un composé de formule Ar-NH-CO-R51 dans lequel R51 est halogène, p.nitrophénoxy ou un groupe imidazolyle ou, de façon préférée avec un isocyanate d'aryle Ar-N=C=O éventuellement substitué. Dans ce cas, on agite entre -20°C et la température d'ébullition du mélange durant une période comprise entre dix minutes et plusieurs heures, une période comprise entre dix minutes à une heure étant en général suffisante pour assurer la

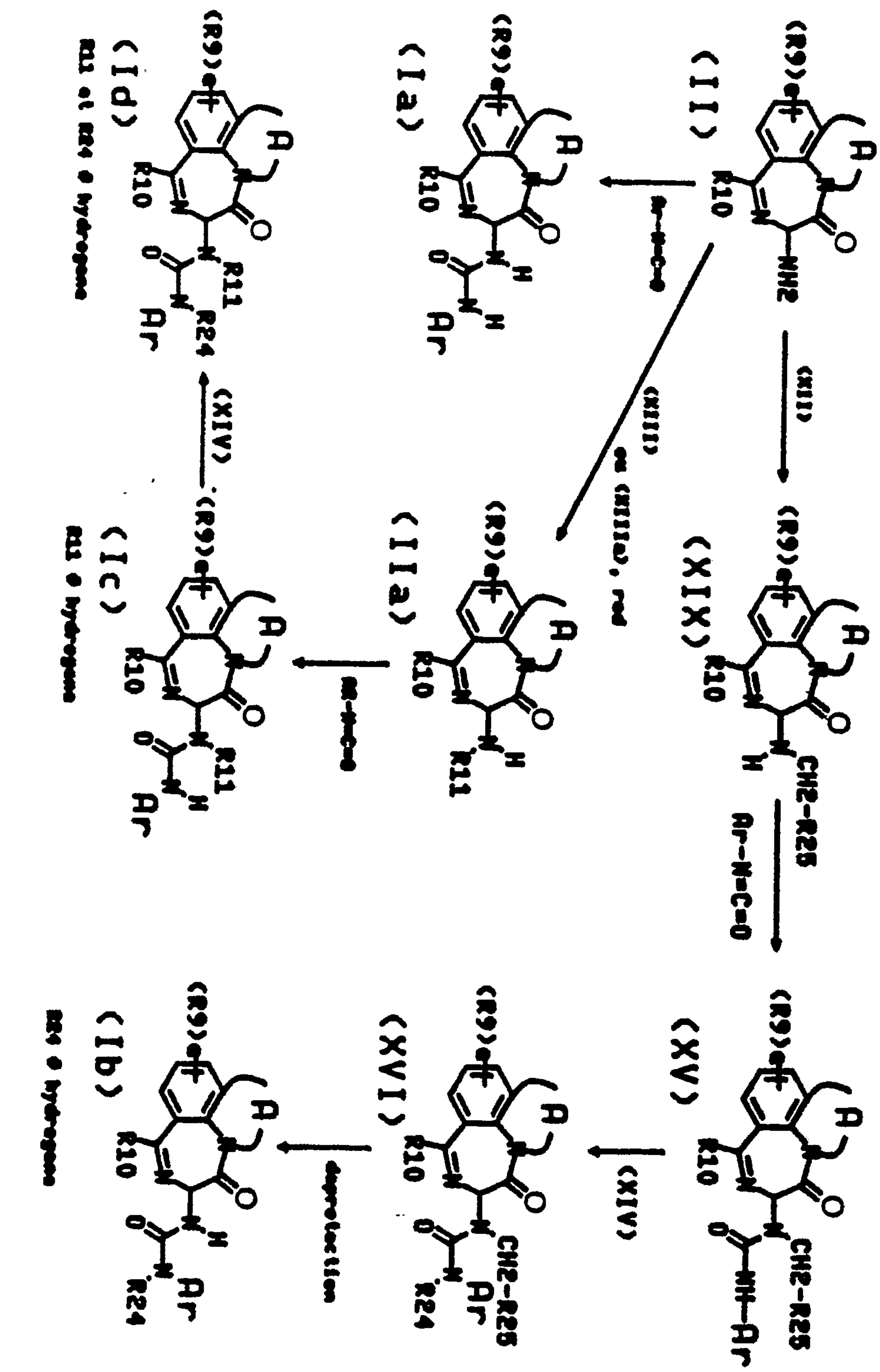
(II)
(XII)
(XIX)
Ar-N=C=O
(XV)
(XIV)
(XVI)
deprotection
(Ib)
R24 ≠ hydrogène
Ar-N=C=O
(Ia)
(XIII) ou (XIIIa), red
(IIa)
Ar-N=C=O
(Ic)
R11 ≠ hydrogène
(XIV)
(Id)
R11 et R24 ≠ hydrogène

SCHEMA 1

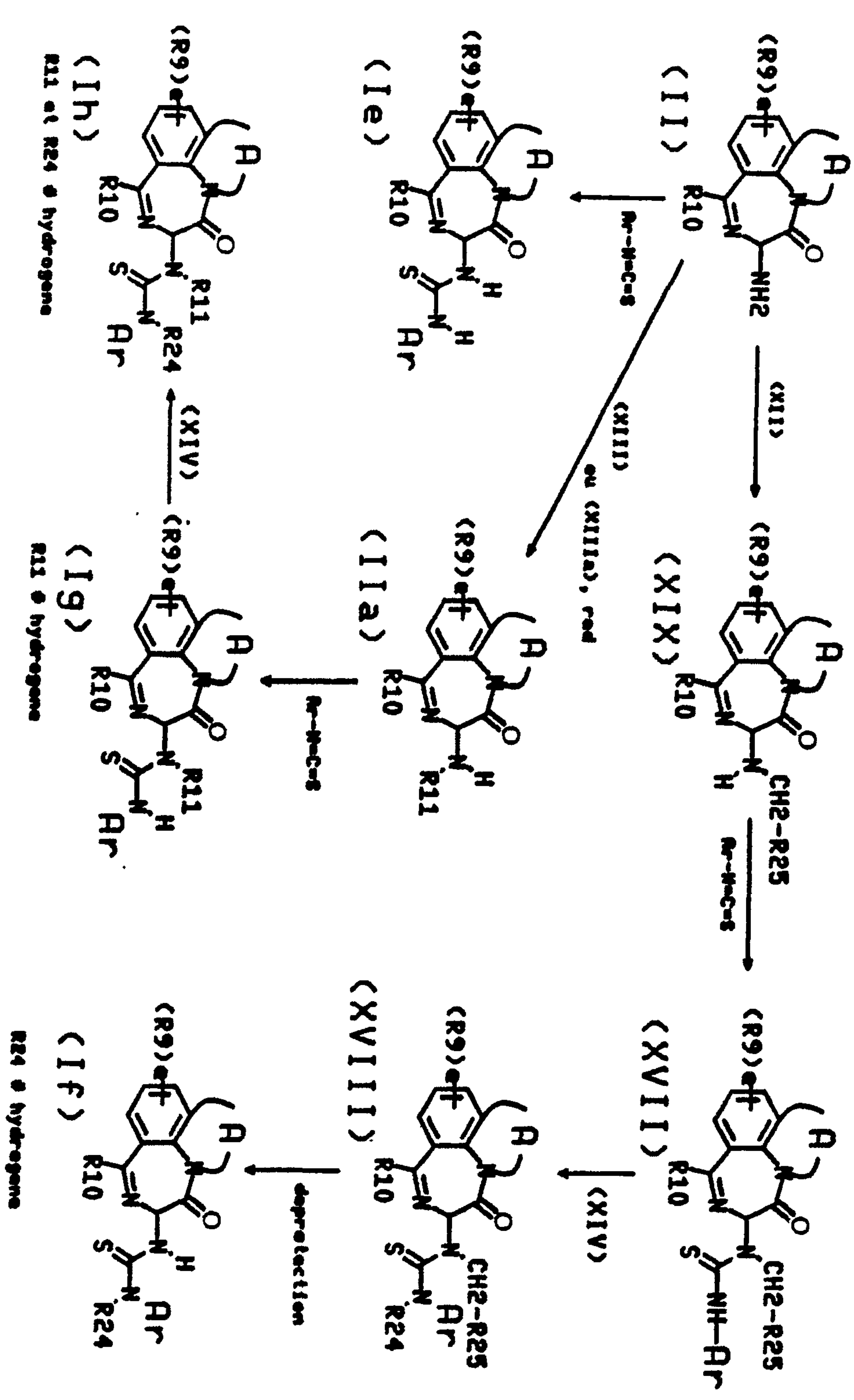


SCHEMA 2

complétion de la réaction. Le milieu réactionnel éventuellement dilué par un des solvants cités ci-dessus est alors évaporé et le produit brut résiduel est purifié.

ii) Préparation des composés (Ib) (schéma 1) :

Dans le cas où R11 est l'hydrogène, R13 est N(R24), R24 etant alkyle inférieur et R12 est l'oxygène, on peut opérer de la manière suivante :

Un composé de formule (II) est dissout dans 5 à 50 volumes d'eau ou d'un solvant organique comme par exemple un hydrocarbure chloré comme du dichlorométhane ou du chloroforme, un éther-oxyde linéaire ou cyclique comme du diméthoxy-1,2-éthane, du tétrahydrofurane ou du dioxane, un solvant polaire aprotique tel que de la pyridine, du diméthylsulfoxyde ou du diméthylformamide ou tout autre solvant convenable pour y effectuer une réaction de condensation ou encore un mélange approprié de deux ou plusieurs de ces solvants et on y ajoute un à plusieurs équivalents d'une base minérale comme un hydroxyde ou un carbonate alcalin ou alcalino-terreux ou d'une base organique comme une amine aliphatique tertiaire, de préférence de la triéthylamine, puis à une température comprise entre entre -20 °C et la température d' ébullition du mélange, on y ajoute une quantité inférieure à un équivalent d'un composé de formule (XII) R25-CH2X dans lequel X représente un halogène ou un pseudo-halogène. La durée de cette addition peut être comprise entre quelques minutes et quelques heures, une durée de une à deux heures étant généralement convenable. On agite entre -20 °C et la température d' ébullition du mélange durant une période comprise entre une et plusieurs heures, une période comprise entre deux et quatre heures étant en général suffisante pour assurer la complétion de la réaction. Le milieu réactionnel après filtration éventuelle des sels est dilué par de l'eau et le produit est extrait par un solvant organique non miscible à l'eau comme par exemple un hydrocarbure halogéné, un ether-oxyde, un hydrocarbure aliphatique ou aromatique ou un ester. La phase organique après lavages éventuels est concentrée sous pression réduite et le produit brut résiduel est purifié pour le séparer des sous produits de la réaction. On obtient ainsi la benzylamine de formule (XIX).

Une autre méthode pour préparer une benzylamine (XIX) à partir d'une amine (II) consiste à dissoudre le composé (II) dans 5 à 50 volumes d'un solvant organique protique comme par exemple un alcool, de préférence le méthanol et à y ajouter à une température comprise entre 0 ° et la température de reflux du mélange une quantité stoechiométrique de composé (XIIa) R25-CHO, précédemment définie plus haut. La suspension est agitée et on y ajoute une suspension ou une solution de cyanoborohydrure de sodium dans 5 à 50 volumes d'un solvant tel que décrit plus haut. Cette addition s'effectue sur une durée comprise entre quelques minutes et quelques heures, une durée de trente minutes étant généralement convenable. A la fin de cette addition le mélange est agité durant la période de temps suffisante pour assurer la complétion de la réaction, cette période étant déterminée par chromatographie sur couche mince, puis le mélange amené à une température proche de la température ambiante est filtré, concentré sous pression réduite, dilué par de l'eau et le produit est alors extrait par un solvant organique non miscible à l'eau, et après évaporation du solvant le produit brut résiduel est purifié.

Cette amination réductrice peut également être réalisée de la manière suivante : un composé (II) est dissout dans 5 à 50 volumes d'un solvant organique protique comme par exemple un alcool, de préférence le méthanol, on y ajoute une quantité stoechiométrique d'une base organique comme par exemple une amine tertiaire de préférence de la triéthylamine puis à une température comprise entre 0 °C et la température de reflux du mélange une quantité stoechiométrique de composé (XIIa) R25-CHO. La suspension est ramenée à une température proche de la température ambiante est tranférée dans un autoclave où elle est traitée à une température voisine de la température ambiante par l'hydrogène sous une pression comprise entre entre la pression atmosphérique et 50 atmosphères durant une période comprise entre 30 minutes et 24 heures en présence d'un catalyseur d'hydrogènation comme un métal noble finement divisé et déposé sur un support, un catalyseur comme du palladium (10%) sur du charbon finement divisé étant convenable. Le catalyseur est ensuite éliminé par filtration, le mélange concentré sous pression réduite, dilué par de l'eau et le produit est alors extrait par un solvant organique non miscible à l'eau, et après évaporation du solvant le produit brut résiduel est purifié.

Le composé de formule (XIX) est ensuite mis à réagir avec un isocyanate d'aryle de manière analogue au composé (I) ci-dessus dans le paragraphe i), et l'on obtient ainsi l'urée de formule (XV) qui est dissoute dans 10 à 100 volumes d'un solvant organique anhydre, comme par exemple un hydrocarbure aromatique ou un éther linéaire ou cyclique, comme par exemple le tétrahydrofurane ou dans un solvant polaire aprotique tel que le dimethylformamide, l'hexaméthylphosphorotriamide, le diméthylsulfoxyde, la N-méthylpyrrolidinone ou le sulfolane (tétraméthylènesulfone) ou encore dans un mélange de ces solvants. La solution est maintenue à une température comprise entre -50 et 0 °C et on ajoute 2 à 4 équivalents d'un agent basique susceptible de déplacer le proton situé sur l'azote de la fonction amide comme par exemple un alcoolate inférieur de métal alcalin de préférence du tertio-butylate ou du tertio-amylate de potassium. Le mélange est agité durant une période comprise entre 10 et 60 minutes et on y ajoute une quantité

stoechiométrique ou légérement supérieure d'un composé (XIV) R24-R39 dans laquelle R24 et R39 ont les significations définies précédemment puis on agite durant une période comprise entre 10 et 60 minutes. Le milieu réactionnel est alors concentré, les sels présents dans le milieu sont filtrés, le milieu est éventuellement dilué avec de l'eau et le produit extrait par un solvant organique non miscible à l'eau, et après évaporation du solvant l'urée de formule (XVI) est purifiée.

L'urée est ensuite dissoute dans 10 à 100 volumes d'ammoniac liquide éventuellement additionnée d'une quantité comprise entre 0 et 50 % d'un cosolvant comme un éther-oxyde, de préférence du tétrahydrofurane ou du dioxane, pour améliorer la solubilité de l'urée et on y ajoute par petites portions une quantité comprise entre 1 et 3 équivalents de sodium. Il se forme, après addition de chaque portion une coloration bleue qui disparaît rapidement (15 à 30 secondes). On arrête l'addition lorsque la coloration bleue persiste durant plus d'une minute. On ajoute alors un excès de chlorure d'ammonium puis dilue avec un solvant organique insoluble dans l'eau comme par exemple du chlorure de méthylène ou de l'acétate d'éthyle et de l'eau. Le produit est alors extrait dans le solvant organique, et après évaporation la benzodiazépine de formule (Ib) est purifiée.

iii) Préparation des composés (Id) (schéma 1) :

Dans le cas où R11 est alkyle inférieur, R13 est N-R24, R24 étant alkyle inférieur, et R12 est l'oxygène, on procède par alkylation d'une benzodiazépine (Ic) dont la préparation est décrite au paragraphe i) ci-dessus, de la même manière que pour la préparation d'une benzodiazépine (Ib) pour obtenir l'intermédiaire (XVI) par alkylation ducomposé (XV) tel que décrit au paragraphe ii) ci-dessus.

iv) Préparation des composés (Ie) et (Ig) (schéma 2) :

Dans le cas ou R13 est N(R24) et R12 est un atome de soufre on peut notamment opérer de la manière suivante:

un composé de formule (II) ou (IIa) est dissous dans 5 à 50 volumes d'un solvant organique anhydre comme par exemple un hydrocarbure chloré comme du dichlorométhane ou du chloroforme, un éther-oxyde linéaire ou cyclique comme du diméthoxy-1,2-éthane, du tétrahydrofurane ou du dioxane, un solvant polaire aprotique tel que de la pyridine, du diméthylsulfoxyde ou du diméthyl-formamide ou tout autre solvant convenable pour y effectuer une réaction de condensation ou encore un mélange approprié de deux ou plusieurs de ces solvants et on y ajoute un équivalent d'un agent pouvant réagir avec la fonction amine du composé (II) pour donner une thiourée, comme par exemple un isothiocyanate d'aryle éventuellement substitué. On agite entre -20° C et la température d'ébullition du mélange durant une période comprise entre dix minutes et seize heures, une période comprise entre dix minutes à une heure étant en général suffisante pour assurer la complétion de la réaction. Le milieu réactionnel éventuellement dilué par un des solvants cités ci-dessus est alors évaporé puis le produit est purifié.

v) Préparation des composés (If) (schéma 2) :

Dans le cas où R11 est l'hydrogène, R13 est N(R24), R24 étant alkyle inférieur et R12 est le soufre, on opère de la même manière que pour la préparation d'une benzodiazépine (Ib) mais en remplaçant l'isocyanate d'aryle par un isothiocyanate d'aryle, pour obtenir successivement une benzylamine (XIX), une thiourée (XVII), une thiourée tétrasubstituée (XVIII) puis après élimination du groupe protecteur R25-CH2- une benzodiazépine de formule (If). Il faut cependant noter que dans ce cas la première méthode d'élimination du groupe protecteur ne convient pas car le soufre empoisonnerait le catalyseur métallique.

vi) Préparation des composés (Ih) (schéma 2) :

Dans le cas où R11 est alkyle inférieur, R13 est N-R24, R24 étant alkyle inférieur, et R12 est le soufre on procède à partir d'une benzodiazépine de formule (Ig), de la manière décrite au paragraphe iii) ci-dessus pour la préparation d'une benzodiazépine (Id) à partir d'une benzodiazépine (Ic).

**PREPARATION D'UN COMPOSE DE FORMULE (II) ou (IIa) RACEMIQUE**

La préparation des composés de formule (II) et (IIa) racémiques est réalisée à partir de benzodiazépines-[1,4] de formule (III) est illustrée sur le schéma 3, et détaillée ci-dessous :

i) Lorsque R11 est l'hydrogène (formule (II)) :

On peut préparer l'amine (II) en aminant en la position alpha par rapport au carbonyle une benzodiazépine-[1,4]-one (III), (schéma 3) dans laquelle A, R9, R10 et e ont la signification définie précédemment par un dérive d'hydroxylamine ou par la chloramine.

On peut également aminer la benzodiazépine-[1,4]-one (III) en deux stades, le premier stade consistant à la faire réagir sur un réactif d'oximation de formule (XI) :

**R35-N(=0)h**

dans laquelle h est égal à un ou deux et R35 est alcoxy inférieur ou le chlore quand h est égal à 1 et est NO2 quand h est égal à 2, pour obtenir l'oxime de formule (IV), qui figure au schéma 3 et dans laquelle A, R9, R10 et e ont la signification définie précédemment, que l'on isole, et le second stade consistant à réduire l'oxime catalytiquement par de l'hydrogène en présence d'un catalyseur de réduction ou par réaction avec du zinc en présence d'acide acétique ou avec du chlorure stanneux en presence d'acide chlorhydrique pour obtenir le dérivé aminé (II).

On peut encore préparer l'amine (II) en mettant une benzodiazépine-1,4 one (III) à réagir en milieu basique sur un réactif approprié pour introduire une fonction azoture sur un carbanion afin obtenir un azoture de formule (V) (schéma 3) dans laquelle A, R9, R10 et e ont la signification définie précédemment ; que l'on isole, puis que l'on réduit par un agent réducteur.

Pour préparer un composé (II) en une étape, à partir d'un composé (III), on opère en dissolvant un composé (III) dans 10 à 100 volumes d'un solvant organique anhydre, comme par exemple un hydrocarbure aromatique ou un éther linéaire ou cyclique, comme par exemple le tétrahydrofurane ou dans un solvant polaire aprotique tel que le dimethylformamide, l'hexaméthylphosphorotriamide, le diméthylsulfoxyde, la N-méthylpyrrolidinone ou le sulfolane (tétraméthylène-sulfone) ou encore dans un mélange de ces solvants. La solution est maintenue à une température comprise entre -50 et 0° C et on ajoute 2 à 4 équivalents d'un agent basique susceptible de déplacer le proton en α du groupe carbonyle du cycle diazépine comme par exemple un alcoolate inférieur de métal alcalin de préférence du tertio-butylate ou du tertio-amylate de potassium. Le mélange est agité durant une période comprise entre 10 et 60 minutes et on y ajoute 3 à 20 équivalents d'un réactif d'amination tel que par exemple un dérivé d'hydroxylamine, l'O-(2,4-dinitrophényl)-hydroxylamine ou l'O-(diphényl-phosphinyl)-hydroxylamine ou l'O-(2,4,6-triméthylphényl-sulfonyl)-hydroxylamine ou la chloramine, puis on agite durant une période comprise entre 10 et 60 minutes. Le milieu réactionnel est alors concentré, les sels présents dans le milieu sont filtrés, le milieu est éventuellement dilué avec de l'eau et le produit extrait par un solvant organique non miscible à

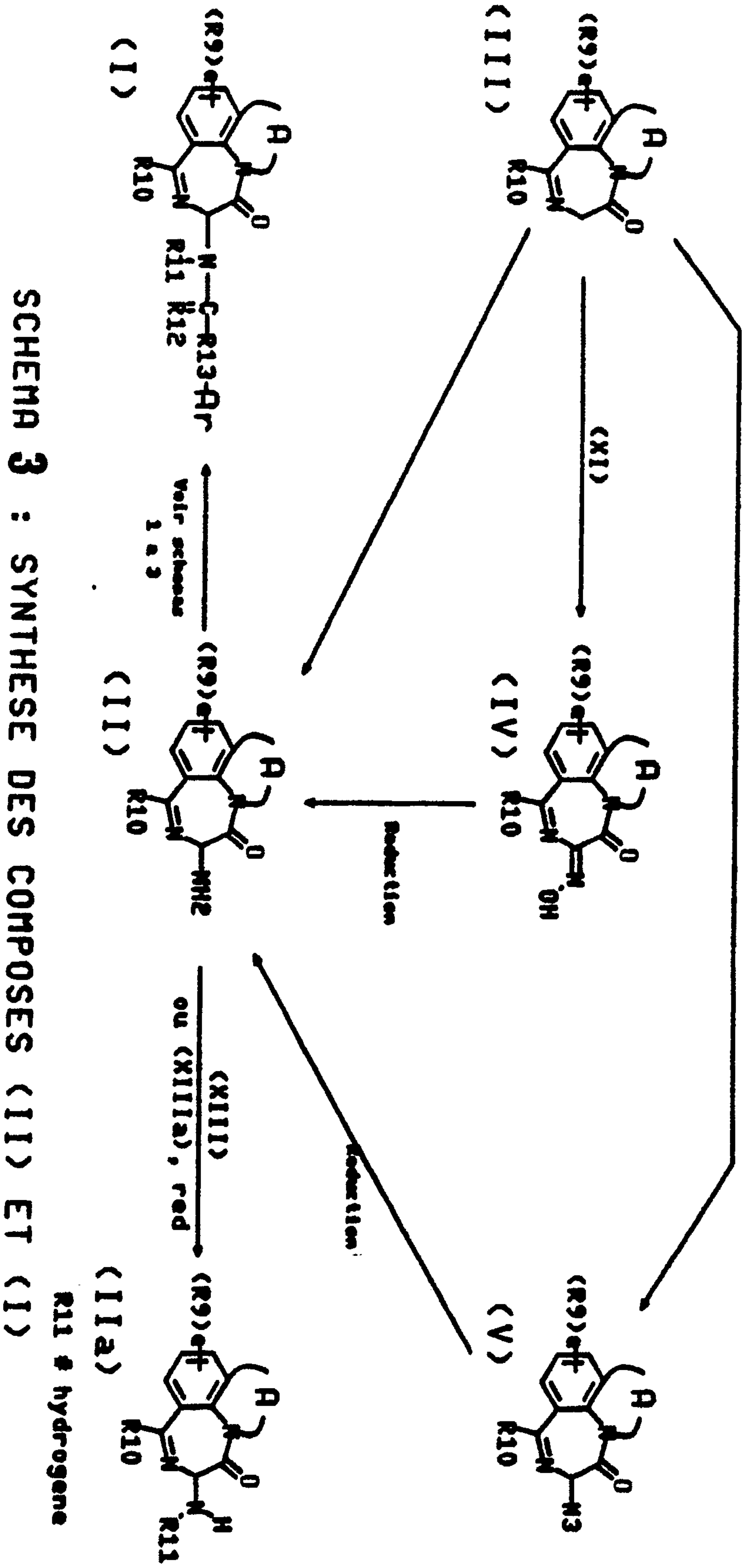


SCHEMA 3 : SYNTHESE DES COMPOSES (II) ET (I)

l'eau, et après évaporation du solvant le produit est purifié par les méthodes habituelles.

Pour préparer un composé (II) en deux étapes à partir d'un composé (III) en isolant un composé intermédiaire de formule (IV) comportant une fonction oxime on opère de la manière suivante (mode opératoire préféré) :

Un composé (III) est dissous dans 10 à 100 volumes d'un solvant organique anhydre comme par exemple un hydrocarbure aromatique ou un éther linéaire ou cyclique comme par exemple le tétrahydrofurane ou dans un solvant polaire aprotique tel que le dimethylformamide, l'hexaméthylphosphorotriamide, le diméthylsulfoxyde, la N-méthylpyrrolidone ou le sulfolane ou encore dans un mélange de ces solvants. La solution est maintenue à une température comprise entre -50 et 0 ° C et on ajoute 2 à 4 équivalents d'un agent basique susceptible de déplacer le proton en alpha du C=O, comme par exemple un alcoolate inférieur de métal alcalin de préférence du tertio-butylate ou du tertio-amylate de potassium. Le mélange est agité durant une période comprise entre 10 et 60 minutes et on y ajoute 3 à 20 équivalents d'un nitrite d'alkyle inférieur de préférence du nitrite d'isoamyle ou du chlorure de nitrosyle (NOCl) ou encore du tétroxyde d'azote (N2O4) et on laisse le mélange revenir à la température ambiante puis on agite durant une période comprise entre 10 et 60 minutes. Le milieu réactionnel est alors neutralisé par addition d'une solution au dixième d'acide acétique et le produit extrait en une ou plusieurs fois par un solvant organique non miscible à l'eau comme par exemple un hydrocarbure aliphatique ou aromatique, un hydrocarbure halogéné, un éther ou un ester d'alcool inférieur d'un acide carboxylique inférieur. Après évaporation du solvant le produit est purifié. Le composé intermédiaire de formule (IV) est alors transformé en composé aminé (II) en le mettant en suspension dans 5 à 100 volumes d'un solvant organique comme par exemple un alcool aliphatique inférieur ou un ester d'alcool aliphatique inférieur d'un acide carboxylique inférieur en présence d'un catalyseur d'hydrogénation comme par exemple du nickel de Raney, du rhodium sur charbon ou du ruthénium sur charbon qui est le catalyseur généralement préféré. La suspension est agitée sous une atmosphère d'hydrogène à une pression comprise entre la pression atmosphérique et trente atmosphères durant une période comprise entre une et cinquante heures à une température comprise entre 0 ° C et 80 ° C ; une pression légèrement supérieure à la pression atmosphérique, une température d'environ 70 ° C et une période d'agitation de deux heures étant en général suffisantes pour assurer une réaction complète. Le milieu réactionnel est alors filtré et le catalyseur lavé plusieurs fois avec un solvant du même type que celui cité ci-dessus. Après évaporation du solvant le produit est purifié.

Il est également possible d'effectuer cette réduction non pas catalytiquement mais chimiquement (de manière stoechiométrique) en opposant le composé (IV) à un agent réducteur comme par exemple du zinc dans de l'acide acétique ou du chlorure stanneux dans de l'acide chlorhydrique ou encore du borure de nickel (réactif préparé par action de borohydrure de sodium sur du chlorure de nickel bivalent).

Pour préparer un composé (II) en deux étapes à partir d'un composé (III) en isolant un composé intermédiaire (V) comportant une fonction azide on opère de la manière suivante :

Un composé (III) est dissous dans 5 à 100 volumes d'un solvant organique anhydre, comme par exemple un hydrocarbure aromatique ou un éther linéaire ou cyclique comme par exemple le tétrahydrofurane ou dans un poly éther (solvant de la série dite des glymes) ou dans un solvant polaire aprotique tel que le dimethylformamide, l'hexaméthylphosphorotriamide, le diméthylsulfoxyde, la N-méthylpyrrolidinone ou le sulfolane ou encore dans un mélange de ces solvants. La solution est maintenue à une température comprise entre -50 ° C et la température ambiante et on ajoute 1 à 4 équivalents d'un agent basique susceptible de déplacer le proton en alpha du carbonyle d'un composé (III) comme par exemple un alcoolate inférieur de métal alcalin de préférence du tertio-butylate ou du tertio-amylate de potassium, du butyllithium, de l'hydrure de sodium ou de l'amidure de sodium. Le mélange est agité durant une période comprise entre 10 minutes et 6 heures et on y ajoute 1 à 5 équivalents d'un réactif susceptible d'introduire une fonction azide sur un carbanion, le réactif préféré étant l'azidure de tosyle et on laisse éventuellement le mélange revenir à la température ambiante puis on agite durant une période comprise entre 10 minutes et 5 heures à une température comprise entre la température ambiante et la température de reflux du mélange. Les sels qui sont présents dans le milieu réactionnel sont alors éventuellement filtrés, puis le milieu réactionnel neutralisé par addition d'une solution au dixième d'acide acétique et le produit extrait en une ou plusieurs fois par un solvant organique non miscible à l'eau comme par exemple un hydrocarbure aliphatique ou aromatique, un hydrocarbure halogéné, un éther ou un ester d'alcool inférieur d'un acide carboxylique inférieur, la phase organique est alors éventuellement lavée avec une solution diluée d'un acide ou d'une base minérale puis par de l'eau. Après évaporation du solvant le produit est purifié. Le composé intermédiaire (V) est alors transformé en composé aminé (II) de la manière suivante :

Un composé (V) est mis en suspension dans 5 à 100 volumes d'un solvant organique comme par exemple un alcool aliphatique inférieur, un ester d'alcool aliphatique inférieur d'un acide carboxylique inférieur, un solvant aromatique comme par exemple du benzène du toluène ou de la pyridine, de l'eau ou

encore un mélange de ces solvants et on y ajoute 1 à 10 équivalents d'un agent réducteur comme par exemple du chlorure de vanadium II (VCl2) en solution aqueuse, du borohydrure de sodium (en présence de méthanol), de l'hydrogéne sulfuré ou du nickel de Raney. Le milieu réactionnel est agité à une température comprise entre la température ambiante et la température de reflux du mélange durant une période suffisamment longue pour assurer la complètion de la réaction (selon la nature de l'agent réducteur utilisé cette période qui est déterminée par chromatographie sur couche mince peut varier de 10 minutes à plusieurs heures) puis éventuellement refroidi, filtré pour éliminer le précipité qui peut être présent, neutralisé,dilué, le solvant est éliminé par distillation sous pression réduite puis le produit est purifié.

Il est également possible d'effectuer cette réduction non pas stoechiométriquement mais d'une manière catalytique en opposant le composé (V) à un agent réducteur en présence d'un catalyseur de réduction comme par exemple du formiate d'ammonium en presence de palladium sur charbon ou de l'hydrogène (sous une pression comprise entre 1 et 5 atmosphères) en présence de palladium déposé sur du carbonate de calcium (catalyseur dit de Lindlar). Après filtration du catalyseur le produit aminé de formule (II) est isolé d'une manière analogue à celle décrite plus haut.

ii) Lorsque R11 est alkyle inférieur (formule (IIa) :

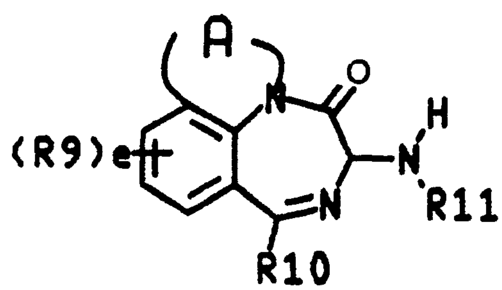

(IIa)

R11 different de l'hydrogene

Pour préparer un composé de formule (IIa) à partir d'un composé (II), on opère de la manière décrite plus haut lors de la préparation de la benzodiazépine de formule (Ic) (schéma 1), mais en utilisant selon le cas :

- un réactif de formule (XIII) pour effectuer une alkylation directe de l'amine,
- ou un réactif de formule (XIIIa) dans le cas d'une amination réductrice à la place des réactifs (XII) ou (XIIa).

## PREPARATION DES COMPOSES DE FORMULE III

La préparation des composés (III) a été réalisée à partir de méthodes décrites dans la littérature, elle est brièvement résumée ci-dessous.

Elle consiste essentiellement à traiter une indoline ou une tétrahydroquinoléine substituée de manière appropriée par un équivalent de trichlorure de bore puis par un benzonitrile substitué de manière appropriée, puis par du chlorure d'aluminium et à chauffer le mélange éventuellement dissout dans un solvant organique jusqu'à une température comprise entre 80 et 150° C.

On obtient après hydrolyse une aminocétone. Cette méthode est décrite en détail par : Adachi M. et coll., Chem. Pharm. Bull., 33, 1826 (1985), ainsi que par Kazuyuki S. et coll., Chem. Pharm. Bull., 33, 1836 (1985).

L'aminocétone est ensuite cyclisée en benzodiazépine (III) selon la méthode décrite par Hester J.B. et coll. (J. Med. Chem., 13, 827, 1970). Elle consiste à traiter une amino-cétone par du chlorure de bromacétyle ou du chlorure d'iodoacétyle dans un solvant organique en présence d'un équivalent d'une base organique pour obtenir un haloacétamide qui est mis à réagir avec une solution d'ammoniac dans un solvant organique pour donner une benzodiazépine de formule (III).

**PREPARATION D'UN COMPOSE DE FORMULE (II) ou (IIa) OPTIQUEMENT ACTIF**

La préparation des composés (II) sous formes d'énantiomères purs est illustrée au schéma 4 et détaillée ci-dessous .

Une benzodiazépine (II)ou (IIa), racémique, est condensée avec une molécule dérivant d'un amino-acide optiquement actif, naturel ou non, appartenant à la série D ou à la série L, de formule (VII) :
R31 étant
- un groupement alkyle ayant de 1 à 6 atomes de carbone éventuellement substitué par un hydroxyle, un groupe thioalkyle ayant de un à six atomes de carbone dans le groupe alkyl ou un groupe carboxyle ou carbonylamino,
- un groupe aryle comportant un ou deux cycles éventuellement substitué par un hydroxyle, et qui est notamment phényle ou benzyle,
- un groupe aralkyle ayant un ou deux cycles aromatiques et dont la portion alkylique comporte de un à six atomes de carbone, éventuellement substitué sur le cycle par un ou plusieurs groupes halogènes, hydroxy ou méthoxy,
- un hétérocycle ayant cinq ou six chainons, et un ou deux hétéroatomes choisis parmi l'azote, l'oxygène et le soufre,
- un groupe indolyl-3-méthyle,
- un groupe imidazolyl-4-méthyle.

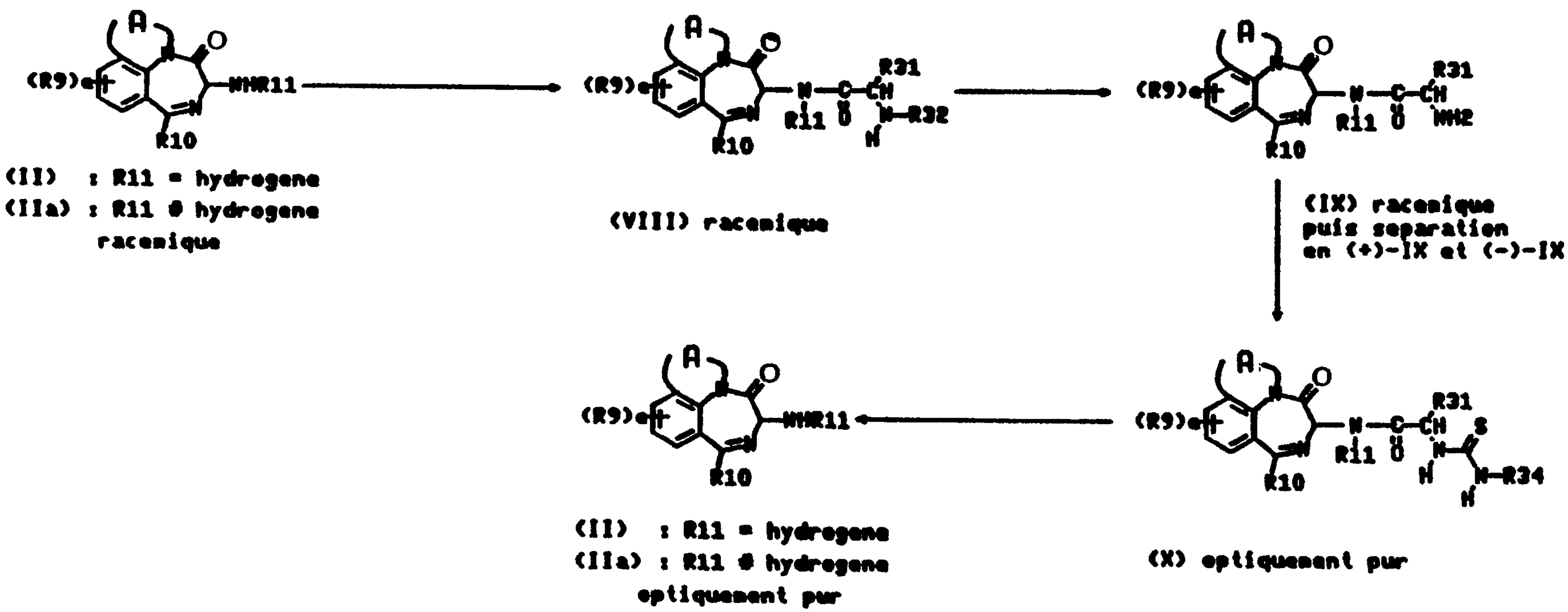


(II) : R11 = hydrogene
(IIa) : R11 ≠ hydrogene
racemique

(VIII) racemique

(II) : R11 = hydrogene
(IIa) : R11 ≠ hydrogene
optiquement pur

(X) optiquement pur

SCHEMA 4 : SEPARATION DES ISOMERES DE (II)

De préférence R31 sera le groupe isobutyle et l'amino-acide appartiendra à la série L,

R32 étant un groupement pouvant être éliminé facilement pour régénérer l'amine libre et peut être

- un radical oxycarbonyle de structure R38-O-CO-dans lequel R38 est un groupe alkyle comportant de un à six atomes de carbone, ou un groupe aryle, éventuellement substitué par un ou plusieurs groupes méthoxy, halogène ou nitro, par exemple benzyle, p-chlorobenzyle, p-bromobenzyle p-nitrobenzyle, fluorènyl-9-méthyle, p-méthoxybenzyle, dichloro-2,4-benzyle, dichloro-2,6-benzyle, t-amyle, isopropyle, adamantyle,
- un groupe alkanoyle ou alcènoyle comportant de un à six atomes de carbone ou acyle tel que formyle, trifluoroacétyle, phtalyle
- p-toluènesulfonyle,
- p-nitrosulfényle,

de préférence R38 sera le groupement tertio-butyle.

R33 étant un groupe hydroxy, un groupe azido (-N3), un groupe imidazol-1-yle, un groupe -O-CO-O-R36, R36 pouvant être un radical alkyle encombré comportant de trois à six atomes de carbone, ou un groupe -OR37, R37 étant un groupe aromatique comportant un ou deux cycles éventuellement substitué par un ou plusieurs radicaux nitro ou halogènes. De préférence R37 est un groupe benzotriazolyle-1.

La condensation est effectuée dans des conditions appropriées pour transformer un composé de formule (II) en un composé de formule (I). On obtient un composé possédant la formule générale (VIII) dans laquelle A, R9, R10, R31, R32 et e ont les significations définies plus haut, sous forme racémique. Le groupement protecteur sur l'azote (R32) est alors enlevé et l'on obtient ainsi l'amine libre de formule (IX) sous forme racémique. Cette déprotection peut être réalisée par hydrolyse :

- soit en milieu acide, en présence d'un acide minéral ou organique fort comme l'acide chlorhydrique, l'acide sulfurique l'acide fluorhydrique, l'acide bromhydrique ou un acide sulfonique comme par exemple l'acide para-toluènesulfonique ou l'acide méthanesulfonique, ou encore l'acide acétique éventuellement substitué par un à trois atomes de chlore ou de fluor, l'acide formique ou tout autre acide convenable dans un solvant ou un mélange de solvants aqueux ou organique comme un acide carboxylique, un hydrocarbure aliphatique halogéné, un hydrocarbure aromatique éventuellement substitué par un groupe halogène ou hydroxy, un alcool aliphatique éventuellement substitué par un ou plusieurs atomes d'halogènes comme par exemple l'éthanol ou le trifluoroéthanol ou encore un éther aliphatique linéaire ou cyclique comme par exemple le diméthoxy-1,2-éthane, le dioxane ou le tétrahydrofurane. La méthode d'hydrolyse acide préférée consiste à dissoudre le composé dans une solution à environ 10 % d'acide trifluoro-acétique dans le chlorure de méthylène, et à agiter durant une période de quelques minutes à quelques heures à une température comprise entre zéro degré centigrade et la température de reflux du mélange réactionnel.
- soit en milieu basique dans certains cas comme par exemple lorsque le groupe protecteur R32 est C-(=O)-O-R38 et R38 est le groupe fluorényl-9-méthyle, le solvant utilisé étant un solvant ou un mélange de solvants aqueux ou organique comme un hydrocarbure aliphatique halogéné, un solvant dipolaire protique ou aprotique comme par exemple le diméthyl-formamide, le diméthylsulfoxyde, la tétraméthylènesulfone (sulfolane), la N-méthylpyrrolidone, l'acétonitrile ou le N,N-diméthylacétamide ; un alcool aliphatique, un ester d'alcool aliphatique d'acide carboxylique, un éther linéaire ou cyclique ; l'agent basique pouvant être une base minérale comme un hydroxyde de métal alcalin ou une base organique comme une amine tertiaire aliphatique telle que par exemple la triéthylamine, la diisopropyléthylamine, la N-méthylpyrrolidine ou encore la N-méthylmorpholine.
- soit encore par hydrogènation catalytique, le catalyseur utilisé pouvant être un métal noble comme par exemple du palladium ou encore un oxyde de l'un de ces métaux déposé sur un support ; la nature du catalyseur convenable variant avec la nature du groupe R32 ; quand R32 est R38-O-CO- et R38 est benzyle le catalyseur peut être du palladium sur charbon.

Le dérivé d'amino-acide obtenu (IX) est séparé en ses diastéréoisomères par chromatographie, pour donner les deux isomères de l'amine (IX).

Puis par dégradation d'Edman effectuée sur chacun des composés (IX) diastéréoisomères, pour obtenir les deux énantiomères (R) et (S) de l'amine (II).

La dégradation d'Edman consiste

- à faire réagir un arylisothiocyanate de formule R34-N=C=S sur la fonction amine libre d'un composé de formule (IX) optiquement actif pour obtenir une thiourée de formule (X) ; le groupe R34 étant un radical aryle tel que phényle ou naphtyle éventuellement substitué par un ou plusieurs substituants choisis parmi les halogènes, le groupe méthoxy ou un groupement alkyle inférieur ayant de 1 à 6 atomes de carbone, le groupe phényle étant généralement utilisé. La réaction est effectuée dans un solvant inerte à la fonction isothiocyanate tel qu'un hydrocarbure aliphatique halogéné, un hydrocarbure aromatique éventuellement substitué par un groupe halogène un ester aliphatique d'un alcool aliphatique un solvant dipolaire aprotique

comme par exemple le diméthylformamide, le diméthylsulfoxyde, la tétraméthylènesulfone (sulfolane) la N-méthylpyrrolidone, l'acétonitrile ou le N,N-diméthylacétamide ou encore un éther aliphatique linéaire ou cyclique comme par exemple le dioxane ou le tétrahydrofurane. Habituellement on préfère effectuer cette réaction dans du chlorure de méthylène, à une température comprise entre zéro degré et la température de reflux du mélange réactionnel, la réaction étant complète au bout d'une période de temps comprise entre quelques minutes et huit heures.

- puis à cycliser et couper la thiourée obtenue (X) pour donner l'amine (II) optiquement active. Ceci peut se faire en une ou deux étapes. On préfère habituellement réaliser les deux étapes sans isoler le composé cyclique intermédiaire ce qui évite une purification. On opère généralement en dissolvant la thiourée dans cinq à cent volumes d'une solution de concentration comprise entre cinq et cent pour cent d'un acide fort comme par exemple l'acide trifluoroacétique dans un solvant organique comme par exemple un hydrocarbure halogéné, de préférence du chlorure de méthylène et en agitant cette solution à une température comprise entre zéro degré et la température de reflux du mélange réactionnel durant une période comprise entre quelques minutes et seize heures.

La séparation des isomères de l'amine (II) peut aussi se faire par salification, cristallisation, puis filtration du sel obtenu. Le procédé consiste à dissoudre l'amine racémique (II) dans une solution d'un acide optiquement actif, connu pour séparer des bases organiques, comme par exemple l'acide mandèlique, l'acide dibenzoyltartrique, l'acide di-p-toluoyltartrique, l'acide camphosulfonique, l'acide p-nitrobenzoylglutamique, l'acide tartrique, l'acide binaphtylphosphorique, dans un solvant aqueux ou organique comme par exemple un alcool aliphatique inférieur, l'acétone, l'acétonitrile ou tout autre solvant approprié à la cristallisation sélective d'un des deux sels diastéréoisomères. L'acide L(+)- tartrique ou l'acide (+)-binaphtylphosphorique sont préférés en solution dans l'acétone ou l'acétonitrile.

L'invention vise également un médicament pour lutter contre le syndrome de Zollinger-Ellison, les troubles gastro-intestinaux, du pancréas et de la vésicule biliaire, les troubles du système nerveux central et la douleur, caractérisé en ce qu'il comprend une benzodiazépine suivant l'invention.

## EXEMPLES

Les exemples suivants illustrent l'invention.

Sauf mention contraire, les méthodes utilisées lors des synthèses et des analyses sont résumées dans ce qui suit.

Les points de fusion ont été repérés en tube capillaire sur un appareil Mettler et n'ont pas été corrigés. Les spectres de résonance magnétique nucléaire ont été enregistrés sur un spectromètre JEOL FX-90Q (90 Mhz) le tétraméthylsilane étant utilisé comme référence interne. Les spectres sont décrits de la manière suivante : déplacement chimique (exprimé en ppm par rapport à la référence interne), multiplicité, intensité de l'intégration, éventuellement constante de couplage et attribution. Les spectres infrarouge ont été enregistrés en pastille de bromure de potassium sur un spectrophotomètre Shimadzu IR-435. Les chromatographies flash ont été effectuées telles que décrites par Still sur gel de silice (article E.MERCK 4063) (Still W.C., Kahn M., Mitra A., J. Org. Chem. (1978), 43, 2923). Les chromatographies sur couche mince ont été effectuées sur plaque de silice 60F-254 de 0.25 mm d'épaisseur (article E. Merck 5714). Les plaques sont examinées sous lumière ultraviolette ou révélées par de l'iode, du réactif de Dragendorff ou du réactif à la toluidine. Les chromatographies sous haute pression (HPLC) sont effectuées sur un appareil Jobin-Yvon. Le pouvoir rotatoire a été mesuré sur un appareil Polartronic dans une cuve de 10 cm de long à température ordinaire.

## PREPARATION DES COMPOSES DE L'INVENTION

### EXEMPLE 1 : (4R-S)-N-(céto-3-phényl-6-tétrahydro-1,2,3,4-pyrrolo[3.2,1-j,k]benzodiazépin[1,4]-yl-4)-N′-(méthyl-3-phényl-)-urée.

Formule (I) ; A = -(CH2)2- ; R9 = H ; R10 = phényle ; R11 = H ; R12 = O ; R13 = NH ; Ar=méthyl-3-phényle.

**Stade 1a :** Transformation d'un composé de formule (III) en un composé de formule (IV).

Dans un ballon tricol sous atmosphère d'azote, on introduit une solution de 26,3 g de phényl-6-tétrahydro-1,2,3,4-pyrrolo[3,2,1-j,k]benzodiazépin-[1,4]-one-3 (100 mmoles) dans un mélange de 500 ml de toluène anhydre et de 250 ml de tétrahydrofurane anhydre. La solution est refroidie à -20° C et on on y ajoute 37,1 g de tertiobutylate de potassium anhydre (330 mmoles), la solution devient brun-rouge sombre. Après 30 minutes d'agitation à -20° C on ajoute 20,1 ml de nitrite d'isoamyle (150 mmoles) et on laisse le mélange revenir à la température ambiante à laquelle on l'agite durant 30 minutes. On ajoute alors 50 ml d'acide acétique puis 500 ml d'eau. La phase organique est décantée et la phase aqueuse est réextraite par du chlorure de méthylène (3 fois 200 ml). Les extraits organiques sont évaporés sous pression réduite et le résidu purifié par chromatographie flash sur silice, l'éluant utilisé étant un mélange de polarité croissante d'acétone dans du chlorure de méthylène.

On obtient ainsi 23.3 g d'hydroxyimino-4-phényl-6-tétrahydro-1,2,3,4-pyrrolo[3,2,1-j,k]benzodiazépin-[1,4]-one-3 sous forme d'un solide jaune (80 %).
F = 215° C (décomposition).

| R.M.N. : | | | | |
|---|---|---|---|---|
| 11.20 ppm | s | 1 proton | | OH |
| 7.80-7.00 ppm | m | 8 protons | | aromatiques |
| 4.35 ppm | t | 2 protons | J = 8Hz | CH2-N |
| 3.20 ppm | t | 2 protons | J = 8Hz | CH2 |

benzylique I.R. : 3300, 3050, 2800, 1665, 1620, 1600, 1570, 1525, 1515, 1340, 1215, 1150 , 1000 cm-1.

**Stade 1b :** Transformation d'un composé de formule (IV) en un composé de formule (II): préparation de l'amino-4-phényl-6-tétrahydro-1,2,3,4-pyrrolo[3,2,1-j,k]benzodiazépin-[1,4]-one-3. Formule (II), A = -(CH2)2- ; R9 = H ; R10 = phényle (Ia).

**1ère méthode :**

Dans un réacteur à hydrogèner, on introduit une suspension de 100 g de nickel de Raney et de 23,3 g d'hydroxyimino-4-phényl-6-tétrahydro-1,2,3,4-pyrrolo [3,2,1-j,k]benzodiazépin[1,4]one-3 dans 1 l de méthanol . La suspension est agitée à la température ambiante jusqu'à ce que l'absorption d'hydrogène soit terminée (environ 20 heures). La suspension est filtrée et lavée avec cinq fois 200 ml de méthanol. Le méthanol est évaporé sous pression réduite pour donner un résidu huileux visqueux qui est purifié par chromatographie flash sur une colonne de silice, l'éluant utilisé étant un mélange de polarité croissante de méthanol dans du chlorure de méthylène. On obtient ainsi 11,0 g d'amino-4-phényl-6-tétrahydro-1,2,3,4-pyrrolo [3,2,1-j,k]-benzodiazépin-[1,4-]one-3 (58 %) sous forme d'huile qui est engagée immédiatement dans la réaction suivante.
C.C.M. : CH2Cl2/MeOH 95/5 Rf = 0,20.

| R.M.N. : | | | |
|---|---|---|---|
| 7.65-7.00 ppm | m | 8 protons | aromatiques |
| 4.80-4.20 ppm | m | 2 protons | CH2-N |
| 4.20-3.60 ppm | m | 3 protons | CH-NH2 |
| 3.40-2.80 ppm | m | 2 protons | CH2Ar |

I.R. : 3350, 1675, 1600, 1560, 1440, 1340, 1240, 1100, 730, 690 cm-1.

**2ème méthode :**

Dans un réacteur pouvant supporter une pression de 12 bars, on introduit sous azote 9,6 g de ruthénium sur charbon à 5 %. On hydrogène le ruthénium pendant 2 heures à 20° C sous 10 bars d'hydrogène. On ajoute sous atmosphère d'azote 32 g (110 mmoles) d'hydroximino-4-phényl-6-tétrahydro-1,2,3,4-pyrrolo[3,2,1-j,k] benzodiazépin-[1,4]-one-3. On porte à nouveau le réacteur à une pression de 8 bars d'hydrogène et chauffe peu à peu jusqu'à 72° C en deux heures. On maintient cette température pendant encore une heure. On refroidit, filtre sur silice et rince par du méthanol. Après chromatographie sur silice dans du chlorure de méthylène enrichi en méthanol on obtient 29,0 g (94 %).

**Stade 1c :** Transformation d'un compose de formule (II) en un compose de formule (I). Cas où R13 = NH.

Dans un ballon sec et maintenu à l'abri de l'humidité par une atmosphère d'azote et une garde à chlorure de calcium, on introduit 5,54 g (20 mmoles) de (4R,S)-amino-4-phényl-6-tétrahydro-1,2,3,4-pyrrolo-[3,2,1-j,k]benzodiazépin-[1,4]-one-3 obtenue au stade précédent et 100 ml de chlorure de méthylène. On refroidit par un bain de glace et ajoute 2,66 g d'isocyanate de méthyl-3-phényle (20 mmoles). Après 1 heure à température ambiante on évapore le solvant puis on chromatographie le résidu sur une colonne de silice dans un mélange de chlorure de méthylène contenant 10 % en volume d'acétone. On obtient ainsi 6,06 g (Rdt 74 %) d'un solide blanc.
F : 180° C.
C.C.M. : CH2Cl2/Acétone 95/5 Rf = 0,25.

| R.M.N. : | | | |
|---|---|---|---|
| 8,95 ppm | s | 1 proton | CO-NH-Ar |
| 7,70-6,70 ppm | m | 13 protons | ArH,NH |
| 5,17 ppm | d | 1 proton | CO-CH-N |
| 4,50 ppm | m | 1 proton | CH2N |
| 3,95 ppm | m | 1 proton | CH2N |
| 3,30 ppm | m | 2 protons | CH2Ar |
| 2,17 ppm | s | 3 protons | CH3Ar |

I.R. : 3350, 1670, 1640, 1610, 1550, 1440, 1380, 1290, 1210 $cm^1$

EXEMPLE 2 : (4R)-N-(céto-3-phényl-6-tétrahydro-1,2,3,4-pyrrolo[3,2,1-j,k]benzodiazépin-[1.4]-yl-4)-N'-méthyl-3-phényl-)-urée.

Formule (I) ; A = -(CH2)2- ; R9 = H ; R10 = phényle ; R11 = H ; R12 = O ; R13 = NH ; Ar = méthyl-3-phényle ; isomère dextrogyre .

**Stade 2a :** Préparation de la Phényl-6-[N-(N-tertiobutyloxy-carbonyl-L-leucyl-)-amino]-4-tétrahydro-1,2,3,4-pyrrolo [3,2,1-j,k]benzodiazépine-[1,4]-one-3.

Formule (VIII) ; A = -(CH2)2- ; R9 = H ; R10 = phényle ; R11 = H ; R12 = O ; R13 = NH ; R31 = isobutyl ; R32 = tertiobutyloxycarbonyle.

Dans un ballon tricol équipé d'une garde à chlorure de calcium, d'un thermomètre plongeant et d'une arrivée d'azote, on dissout à 5° C 19,0 g (68,5 mmoles) de l'amine préparée dans l'exemple (1) stade (b) dans du chlorure de méthylène (500 ml). On ajoute 17,1 g d'hydrate de Boc-L-Leucine (68,5 mmoles) et 10,5 g d'hydrate d'hydroxy-1-benzotriazole (68,5 mmoles). On ajoute ensuite goutte à goutte une solution de 500 ml de chlorure de méthylène contenant 14,1 g de N,N'dicyclohexylcarbodiimide (68,5 mmoles). On agite pendant 2 heures à 5° C et on laisse revenir à la température ambiante. Après 20 h on filtre l'insoluble et le rince abondamment au chlorure de méthylène. On évapore et chromatographie le résidu sur une colonne de silice en éluant par du chlorure de méthylène enrichi en acétone. Obtenu 32,5 g (97 %) de

produit purifié.
C.C.M. : CH2Cl2/Acétone 5 % Rf = 0,8.

| R.M.N. : | | | | |
|---|---|---|---|---|
| 7,75 ppm | d | 1 proton | J = 8Hz | CH-NH-CO |
| 7,60-6,90 ppm | m | 8 protons | | aromatiques |
| 5,42 ppm | d | 1 proton | J = 8Hz | CH-NH-CO |
| 5,10 ppm | d | 1 proton | | NH-CH-CO |
| 4,60 ppm | m | 1 proton | | CH2N |
| 3,95 ppm | m | 1 proton | | CH2N |
| 3,70-3,25 ppm | m | 3 protons | | CH2Ar,NH-CH-CO |
| 1,90-1,20 ppm | m | 3 protons | | CHCH2 |
| 1,45 ppm | s | 9 protons | | C(CH3)3 |
| 1,0 ppm | d | 6 protons | | (CH3)2 |

I.R. : 3300, 2900, 1710-1650, 1500, 1440, 1160, 1020 cm-1.

**Stade 2b** : Préparation de la (4R,S)-N-(-L-leucyl-)amino-4-phényl-6-tétrahydro-1,2,3,4-pyrrolo[3,2,1-j,k]-benzodiazépine-[1,4]-one-3.

Formule (IX) ; A = -(CH2)2- ; R9 = H ; R10 = phényle ; R11 = H ; R13 = NH ; R31 = isobutyl .

32,5 g (66 mmoles) du produit du stade précédent sont dissous dans 300 ml de chlorure de méthylène. On refroidit dans de la glace et ajoute en une minute mais sans laisser monter la température au dessus de 5 °C, 300 ml d'acide trifluoroacétique. Après 1/2 h d'agitation, on évapore à sec. On reprend par de l'acétate d'éthyle et de la soude normale, puis on lave par de l'eau saturée de chlorure de sodium, on sèche et évapore. On chromatographie sur silice, en éluant par un mélange de polarité croissante de méthanol dans du chlorure de méthylène. On obtient 24,0 g (72 %) de produit sous forme de son sel trifluoroacètique.

Pour obtenir la base du composé 25,0 g du sel trifluoroacétique (50 mmoles) sont agités violemment avec 200 ml de soude normale et 200 ml d'acétate d'éthyle. On décante, extrait la soude par 50 ml d'acétate d'éthyle, lave l'acétate d'éthyle par 50 ml de solution aqueuse saturée de chlorure de sodium. On sèche, évapore pour obtenir 19,0 g (97 %)du produit.
CCM. : AcOEt/MeOH 90/10 Rf = 0,30 et 0,40 (2 taches qui correspondent aux 2 isomères).

| R.M.N. : | | | |
|---|---|---|---|
| 8,72 ppm | d | 1 proton | CH-NH-CO |
| 7,60-6,90 ppm | m | 8 protons | aromatiques |
| 5,42 ppm | d | 1 proton | CH-NH-CO |
| 4,40 ppm | m | 1 proton | CH2N |
| 3,95 ppm | m | 1 proton | CH2N |
| 3,60-3,0 ppm | m | 3 protons | CO-CH-NH, CH2Ar |
| 2,0-1,20 ppm | m | 3 protons | CHCH2 |
| 1,70 ppm | s | 2 protons | NH2 |
| 0,95 ppm | m | 6 protons | (CH3)2 |

I.R. : 3350, 2950, 1660, 1600, 1440, 1380, 1240 cm-1.

**Stade 2c** : Séparation des isomères optiques de la N-(-L-leucyl-)-amino-4-phényl-6-tétrahydro-1,2,3,4-pyrrolo[3,2,1-j,k] benzodiazépine-[1,4]-one-3.

On monte un dispositif de chromatographie flash avec 400 g de silice, dans une colonne de 5 cm de diamètre. On dissout 19,0 g de la base obtenue au stade précédent dans de l'acétate d'éthyle et élue successivement par de l'acétate d'éthyle pur (2,5 l), de l'acétate d'éthyle contenant 5 % de méthanol (2,5 l), de l'acétate d'éthyle contenant 10 % de méthanol (2,5 l), de l'acétate d'éthyle contenant 20 % de méthanol (2,5 l) ; on examine sur plaque de silice, réunit puis évapore les fractions de qualité identique pour obtenir :
isomère A : 11,5 g
mélange d'isomères : 0,4 g
isomère B : 11,5 g
Rendement de la séparation : 96 % (48 % de chaque isomère)
Isomère A : C.C.M. : AcOEt/MeOH 90/10 Rf = 0,5
[α]D = -91°5 (c = 1,0 ; CH2Cl2)
Isomère B : C.C.M. : AcOEt/MeOH 90/10 Rf = 0,3
[α]D = +56°3 (c = 1,0 ; CH2Cl2)

**Stade 2d :** Préparation de la phényl-6-N-[-(N-phényl-thiouréido-)-L-leucyl-]-amino-4(R)-tétrahydro-1,2,3,4-pyrrolo[3,2,1-j,k]benzodiazépine[1,4]-one-3.

Formule (X) : A = -(CH2)2- ; R9 = H ; R10 = R34 = phényle; R11 = H ; R31 = isobutyl ; isomère B.

A une solution agitée de 11,7 g de l'isomère B du stade précédent (30 mmoles) dans du chlorure de méthylène (200 ml) on ajoute goutte à goutte 4,05 g d'isothiocyanate de phényle (30 mmoles). On suit sur plaque l'avancement de la réaction. Après une heure on évapore le solvant. On reprend le résidu (15,5 g) par du chlorure de méthylène contenant 10 % d'acétate d'éthyle et chromatographie sur silice dans le chlorure de méthylène enrichi en acétate d'éthyle. On évapore et obtient 15 g (95 %)de produit. C.C.M. : CH2Cl2/Acétate d'éthyle 85/15 Rf = 0,4 .

**Stade 2e :** (+)-(4R)-Amino-4-phényl-6-tétrahydro-1,2,3,4-pyrrolo[3,2,1-j,k]benzodiazépin-[1,4]-one-3.

Formule II ; A = -(CH2)2- ; R9 = H ; R10 = phényle ; isomère dextrogyre.

On dissout 5,8 g (30 mmoles) de phényl-6-N-(N-phényl-thiouréido-)-L-leucyl-)-amino-4-tétrahydro-1,2,3,4-pyrrolo[3,2,1-j,k] benzodiazépine-[1,4]-one-3 obtenue au stade 2d ci-dessus dans 200 ml d'acide trifluoroacétique en agitant à température ordinaire. On chauffe une heure à 40°C et évapore. On chasse plusieurs fois du chlorure de méthylène pour éliminer l'excés d'acide trifluoroacétique. On chromatographie sur silice en éluant par de l'acétate d'éthyle enrichi progres- sivement en méthanol. On obtient 8 g (68 %) d'aiguilles blanches de trifluoroacétate de (+)-(4R)-amino-4-phényl- 6-tétrahydro-1,2,3,4-pyrrolo[3,2,1-j,k] benzodiazépin-[1,4] -one-3.
C.C.M. : CH2CCl2/Acétate d'éthyle 85/15 Rf = 0,4 .

| R.M.N. : | | | |
|---|---|---|---|
| 7,60-6,90 ppm | m | 8 protons | aromatiques |
| 4,65 ppm | m | 1 proton | CH2N |
| 4,45 ppm | s | 1 proton | CHNH3 |
| 3,90 ppm | m | 1 proton | CH2N |
| 3,50-2,90 ppm | m | 5 protons | NH3, CH2Ar |

**Stade 2f : (produit de l'exemple 2)**

(4R)-N-(céto-3-phényl-6-tétrahydro-1,2,3,4-pyrrolo[3,2,1-j-k]benzodiazépin-[1,4]-yl-4)-N′-(méthyl-3-phényl-)-urée.

Ce composé est préparé à partir de la (4R)-amino-4-phényl-6-tétrahydro-1,2,3,4-pyrrolo[3,2,1-j,k]-benzodiazépin-[1,4]-one-3 optiquement active, de la même manière que pour la préparation du composé de l'exemple 1 au stade (1c) à partir de 5,54 g d'amine (20 mmoles) et de 2,66 g de méthyl-3-phénylisocyanate (20 mmoles ). Après purification par chromatographie flash sur une colonne de silice, en éluant par un mélange de polarité croissante d'acétone dans du chlorure de méthylène on obtient 6,5 g (79 %) du composé .
F = 182° C . [α]D = + 76° (c = 1,0 ; éthanol).
C.C.M. : $CH_2Cl_2$/Acétone 95/5 Rf = 0,25 .

EXEMPLE 3 : N-[céto-3-(fluoro-2-phényl)-6-tétrahydro-1,2,3,4-pyrrolo[3,2,1-j,k]benzodiazépin-[1,4]-yl-4-]-N'-[(méthyl-3-)-phényl-]-urée .

Formule (I) ; A = $-(CH_2)_2-$ ; R9 = H ; R10 = fluroro-2-phényle ; R11 = H ; R12 = O ; R13 = NH ; Ar = méthyl-3-phényle.

**Stade 3a :** (Fluoro-2-benzoyl)-7-indoline.

Dans un réacteur muni d'une agitation centrale d'un réfrigérant équipé d'une garde à chlorure de calcium, d'un thermomètre plongeant, d'une arrivée d'azote, d'une ampoule à brome à rétablissement de pression, on dissout 43,03 g (361 mmoles) d'indoline dans 360 ml de tétra chloroéthane. On refroidit dans un bain de glace et ajoute goutte à goutte 46,75 g (399 mmoles) de trichlorure de bore en solution dans 180 ml de tétrachloroéthane. On ajoute 84,0 g (694 mmoles) de fluoro-2-benzonitrile puis 52,2 g (399 mmoles) de trichlorure d'aluminium. On chauffe 8 h à 150° C. Après refroidissement on hydrolyse avec 325 ml d'acide chlorhydrique 4N. On chauffe encore 20 minutes à 80° C pour terminer l'hydrolyse. On laisse refroidir et filtre l'insoluble. On rince par de l'éther, sèche. On reprend le précipité par du chlorure de méthylène et alcalinise par de la soude. On lave la solution organique par une solution concentrée de chlorure de sodium, on sèche sur sulfate de sodium. Après filtration et évaporation, on obtient 63,0 g de résine jaune (72 %).
C.C.M. : $CH_2Cl_2$ Rf = 0,5.

| R.M.N. : | | | | |
|---|---|---|---|---|
| 7,50-7,0 ppm | m | 7 protons | | aromatiques |
| 6,40 ppm | m | 1 proton | | NH |
| 3,80 ppm | t | 2 protons | J = 9Hz | $CH_2N$ |
| 3,0 ppm | t | 2 protons | J = 9Hz | $CH_2Ar$ |

**Stade 3b :** N-bromoacétyl-(fluoro-2-benzoyl-)-7-indoline.

Dans un réacteur muni d'une agitation centrale d'un réfrigérant équipé d'une garde à chlorure de calcium, d'un thermomètre plongeant, d'une arrivée d'azote, d'une ampoule à brome à rétablissement de pression, 117,0 g (485 mmoles) de produit du stade-(a) sont placés en suspension dans 2,5 l d'éther. On refroidit à -20° C et ajoute 41 ml (485 mmoles) de pyridine puis 91,6 g (582 mmoles) de chlorure de bromacétyle goutte à goutte. On laisse revenir à la température ambiante et agite 20 h. On ajoute 2 l d'eau sous agitation et filtre l'insoluble. On rince par de l'eau puis de l'hexane, on sèche. On recristallise dans de l'acétate d'éthyle. On obtient 120,0 g (61 %) de N-bromoacétyl-(fluoro-2-benzoyl-)-7- indoline. F = 136° C.
C.C.M. ; $CH_2Cl_2$/Acétone 95/5 Rf = 0,25.

| R.M.N. : | | | |
|---|---|---|---|
| 7,90-6,90 ppm | m | 7 protons | aromatiques |
| 4,20 ppm | t | 2 protons | CH2 N |
| 3,76 ppm | s | 2 protons | COCH2Br |
| 3,20 ppm | t | 2 protons | CH2Ar |

I.R. : 1660, 1610, 1450, 1390, 1210, 1100, 1050, 745 cm-1.

**Stade 3c :** (Fluoro-2-phényl)-6-tétrahydro 1,2,3,4-pyrrolo[3,2,1-j,k]benzodiazépin-[1,4]-one-3.

Formule (III) ; A = -(CH2)2- ; R9 = H ; R10 = Fluoro-2-phényle .

Dans un réacteur muni d'une agitation centrale d'un réfrigérant équipé d'une garde à potasse, d'un thermomètre plongeant, d'une arrivée d'azote, d'une ampoule à brome à rétablissement de pression, on verse 1 l de tétrahydrofurane et 570 ml de méthanol anhydre. Après avoir refroidi à -30° C on ajoute 320 ml d'ammoniac liquide. On ajoute ensuite, peu à peu 114,0 g (314 mmoles) de produit du stade précédent et laisse sous agitation jusqu'au lendemain, sans refoidir extérieurement. Tout se dissout lentement. Après 20 h on évapore à sec. On lave à l'eau. On obtient 140,0 g de produit brut que l'on chromatographie sur silice en éluant par du chlorure de méthylène progressivement enrichi en acétone. On obtient 70,0 g de (fluoro-2-phényl)-6-tétrahydro-1,2,3,4-pyrrolo[3,2,1-j,k]benzodiazépin-[1,4]-one-3 (79 %).
F = 148° C.
C.C.M. : CH2Cl2/Acétone 95/5 Rf = 0,6.

| R.M.N. : | | | |
|---|---|---|---|
| 7,60-6,90 ppm | m | 7 protons | aromatiques |
| 4,40 ppm | s | 2 protons | COCH2N |
| 4,30 ppm | t | 2 protons | CH2N |
| 3,20 ppm | t | 2 protons | CH2Ar |

I.R. : 1665, 1600, 1445, 1395, 1345, 1210, 740 cm-1.

**Stade 3d :** Hydroximino-4-(fluoro-2-phényl)-6-tétrahydro-1,2,3,4-pyrrolo[3,2,1-j,k]benzodiazépin-[1,4]-one-3.

Formule (IV) ; A = -(CH2)2- ; R9 = H ; R10 = fluoro-2-phényle.

De la même façon que dans l'exemple-1 stade 1a, à partir de 70,0 g (249 mmoles) du produit du stade précédent on obtient 67,0 g (87 %) d'un solide jaune F = 218° C (décomposition)
C.C.M. : CH2Cl2/Acétone 95/5 Rf = 0,29.

| R.M.N. : (DMSO hexadeuterié) : | | | |
|---|---|---|---|
| 7,80-6,95 ppm | m | 7 protons | aromatiques |
| 4,25 ppm | t | 2 protons | CH2N |
| 3,40 ppm | s | 3 protons | NOH, H2O |
| 3,20 ppm | t | 2 protons | CH2Ar |

I.R. : 3450, 3120, 3000, 2700, 1620, 1600, 1340, 1300, 1000, 740 cm-1.

**Stade 3e :** amino-4-(fluoro-2-phényl)-6-tétrahydro-1,2,3,4-pyrrolo[3,2,1-j,k]benzodiazépin-[1,4]-one-3.

Formule (II) ; A = -(CH2)2- ; R9 = H ; R10 = fluoro-2-phényle.

**1ére méthode**

De la même façon qu'à dans l'exemple 1 stade 1b, 2ème méthode et à partir de 30 g (969 mmoles) du produit du stade précédent on obtient 28 g (98 %) d'une mousse qui est employée directement dans les réactions suivantes.
C.C.M. : CH2Cl2/MeOH 95/5 Rf = 0,54.

| R.M.N. : | | | |
|---|---|---|---|
| 7,70-6,90 ppm | m | 7 protons | aromatiques |
| 4,45 ppm | m | 1 proton | CH2N |
| 4,3 ppm | s | 1 proton | CHNH2 |
| 3,90 ppm | m | 1 proton | CH2N |
| 3,50-3,10 ppm | m | 2 protons | CH2Ar |
| 3,20 ppm | s | 2 protons | NH2 |

I.R. : 3400, 1680, 1580, 1440, 1200, 740 cm-1.

**2ème méthode**

5,8 g (168 mmoles) de produit du stade-(d) sont placés en suspension dans du méthanol, sous atmosphère d'azote, et on ajoute 3,99 g (168 mmoles) de chlorure de nickel hexahydraté. On refroidit à -20 ° C et ajoute par petites quantités 1,2 g (336 mmoles) de borohydrure de sodium. La température monte immédiatement et il se forme un précipité noir. On agite à -20 ° C pendant 30 minutes et évapore à sec. On reprend par de l'acide chlorhydrique concentré, filtre, et alcalinise par une solution d'ammoniaque concentrée, en présence de chlorure de méthylène. On évapore et obtient 6,8 g de produit brut que l'on chromatographie sur silice en éluant par du chlorure de méthylène enrichi progressivement en acétone. On obtient 2,8 g (48 %) d'une mousse identique au produit obtenu par la première méthode.

**Stade 3f :**

Ce composé est préparé à partir de l'amino-4-(fluoro-2-phényl)-6-tétrahydro-1,2,3,4-pyrrolo[3,2,1-j,k]-benzodiazépin-[1,4]-one-3 de la même façon qu'à l'exemple 1, stade 1c. A partir de 5,0g (17mmoles) d'amine et de 2,26g (17 mmoles) de méthyl-3-phényl-isocyanate, après purification par chromatograhie flash sur une colonne de silice, l'éluant utilisé étant un mélange de polarité croissante d'acétone dans du chlorure de méthylène, on obtient 4,0g (55%) de composé sous forme d'un solide blanc. F = 190 ° C.
C.C.M. : CH2Cl2/Acétone 90/10 Rf = 0,30.

| R.M.N. : | | | |
|---|---|---|---|
| 9,0 ppm | d | 1 proton | Ar-NH-C0 |
| 7,70-6,70 ppm | m | 12 protons | ArH, NH |
| 5,20 ppm | d | 1 proton | CO-CH-N |
| 4,70-4,30 ppm | m | 1 proton | CH2-N |
| 4,20-3,70 ppm | m | 1 proton | CH2-N |
| 3,60-2,80 ppm | m | 2 protons | CH2Ar |
| 2,25 ppm | s | 3 protons | CH3Ar |

I.R. : 3200, 3050, 3000, 1685, 1640, 1600, 1560, 1520, 1465, 1440, 1430, 1380, 1340, 1285, 1210 et 1190 cm-1.

EXEMPLE 4 : N-[céto-3-chloro-8-(fluoro-2-phényl-)-6-tétrahydro-1,2,3,4- pyrrolo[3,2,1-j,k]benzodiazépin-[1,4]-yl-4-]-N'-[(méthyl-3-)-phényl-]-urée.

Formule I ; A = -(CH2)2- ; R9 = chloro-8 ; R10 = fluoro-2-phényle ; R11 = H ; R12 = O ; R13 = NH ; Ar = méthyl-3-phényle.

**Stade 4a :** Chloro-5-(fluoro-2-benzoyl-)-7-indoline.

Dans un réacteur muni d'une agitation centrale d'un réfrigérant équipé d'une garde à chlorure de calcium, d'un thermomètre plongeant, d'une arrivée d'azote, d'une ampoule à brome à rétablissement de pression, on verse 44,0 g (182 mmoles) de (fluoro-2-benzoyl)-7-indoline telle que préparée au stade (3a) Ex.1 et 800 ml de chlorure de méthylène. On ajoute peu à peu 27,94 g (182 mmoles) de N-chlorosuccinimide et agite une nuit à température ambiante. On lave avec une solution saturée de bicarbonate de sodium, puis à l'eau, on sèche et évapore. On chromatographie sur silice en éluant par du chlorure de méthylène. On obtient 42,0 g (84 %) de produit sous la forme d'une mousse qui n'a pas de point de fusion défini.
C.C.M. : CH2Cl2 Rf = 0,7.

| R.M.N. : | | | |
|---|---|---|---|
| 7,60-7,00 ppm | m | 6 protons | aromatiques |
| 6,30 ppm | m | 1 proton | NH, |
| 3,85 ppm | m | 2 protons | CH2N |
| 3,10 ppm | m | 2 protons | CH2Ar |

**Stade 4b :** N-bromoacétyl-chloro-5-(fluoro-2-benzoyl)-7-indoline.

55,0 g (199 mmoles) de produit du stade précédent sont traités comme dans au stade 3b Ex.1 avec 34,46 g (219 mmoles) de bromure de bromacètyle. On obtient 60,0 g (76 %) de produit.
C.C.M. : CH2Cl2 Rf = 0,25.

R.M.N. :

| | | | | |
|---|---|---|---|---|
| 7,90-6,80 | ppm | m | 6 protons | aromatiques |
| 4,25 | ppm | t | 2 protons | CH2N |
| 3,80 | ppm | s | 2 protons | CH2Br |
| 3,20 | ppm | t | 2 protons | CH2Ar |

**Stade 4c :** Chloro-8-(fluoro-2-phényl)-6-tétrahydro-1,2,3,4-pyrrolo[3,2,1-j,k]benzodiazépin-[1,4]-one-3.

Formule (III) ; A = -(CH2)2- ; R9 = chloro-8 ; R10 = fluoro-2-phényle .

De la même manière qu'à l'exemple 3 stade 3c et à partir de 90,0 g (226 mmoles) de produit du stade

précèdent, on obtient après chromatographie dans un gradient de chlorure de méthylène enrichi en acétone 46,3 g (65 %) de produit. F = 148° C.
C.C.M. : CH2Cl2/Acétone 95/5 Rf = 0,3.

R.M.N. :

| | | | | |
|---|---|---|---|---|
| 7,60-7,00 | ppm | m | 6 protons | aromatiques |
| 4,45 | ppm | s | 2 protons | CO-CH2-N |
| 4,32 | ppm | t | 2 protons | CH2N |
| 4,20 | ppm | t | 2 protons | CH2Ar |

I.R. : 1660, 1440, 1370, 1340, 1225, 880, 800, 745 cm-1.

**Stade 4d** : Chloro-8-(fluoro-2-phényl)-6-hydroximino-4-tétrahydro-1,2,3,4-pyrrolo[3,2,1-j,k]benzodiazépin-[1,4]-one-3. Formule (IV); A = -(CH2)2- ; R9 = chloro-8 ; R10 = fluoro- 2-phényle .

De la même façon que dans l'exemple-1 stade 1a, à partir de 45 g (143 mmoles) du produit du stade précédent on obtient 33 g (67 %) d'un solide jaune F = 264° C (dec.)
C.C.M. : CH2Cl2/Acétone 90/10 Rf = 0,27.

R.M.N. :

| | | | | |
|---|---|---|---|---|
| 7,85-7,0 | ppm | m | 6 protons | aromatiques |
| 4,32 | ppm | t | 2 protons | CH2N |
| 3,48 | ppm | s | 1 proton | NOH |
| 3,28 | ppm | t | 2 protons | CH2Ar |

I.R. : 3300, 1655, 1615, 1580, 1445, 1370, 1340, 1220, 1160, 1020, 850, 740 cm-1.

**Stade 4e** : Amino-4-chloro-8-(fluoro-2-phenyl)-6-tétrahydro-1,2,3,4-pyrrolo[3,2,1-j,k]benzodiazépin-[1,4]-one-3.

Formule (II) ; A = -(CH2)2- ; R9 = chloro-8 ; R10 = fluoro-2-phényle .

De la même façon que dans l'exemple-1 stade 1b, 2° méthode, à partir de 30 g (870 mmoles) du produit du stade précédent on obtient 28 g (rendement 98 %) d'une mousse qui est employée directement dans les réactions suivantes.
C.C.M. : CH2Cl2/MeOH 95/5 Rf = 0,55.

| R.M.N. : | | | |
|---|---|---|---|
| 7,70-6,95 ppm | m | 6 protons | aromatiques |
| 4,60 ppm | m | 1 proton | CH2N |
| 4,40 ppm | s | 1 proton | CH-NH2 |
| 4,0 ppm | m | 1 proton | CH2N |
| 3,23 ppm | m | 2 protons | CH2Ar |
| 2,50 ppm | s | 2 protons | NH2 |

I.R. : 3350, 1670, 1610, 1580, 1440, 1335, 1210, 860, 790, 750 cm-1.

**Stade 4f : (produit de l'exemple)**

Ce composé est préparé à partir de l'amino-4-chloro-8-(fluoro-2-phényl)-6-tétrahydro-1,2,3,4-pyrrolo-[3,2,1-j,k]benzodiazépin-[1,4]-one-3 de la même façon qu'à l'exemple 1, à partir de 4,0 g (12 mmoles) d'amine obtenue au stade 4e précédent et de 1,88 g (12 mmoles) deméthyl-3-phénylisocyanate. Après purification par chromatograhie flash sur une colonne de silice, l'éluant utilisé étant un mélange de polarité croissante de méthanol dans du chlorure de méthylène, on obtient 3,5 g (62 %) du composé sous forme solide.

F = 256 °C (décomposition).

C.C.M. : CH2Cl2/Acétone 90/10 Rf = 0,25 .

| R.M.N. : | | | |
|---|---|---|---|
| 9,0 ppm | d | 1 proton | Ar-NH-C0 |
| 7,70-6,70 ppm | m | 11 protons | ArH, NH |
| 5,25 ppm | d | 1 proton | CO-CH-N |
| 4,70-4,30 ppm | m | 1 proton | CH2-N |
| 4,20-3,80 ppm | m | 1 proton | CH2-N |
| 3,60-2,80 ppm | m | 2 protons | CH2Ar |
| 2,25 ppm | s | 3 protons | CH3Ar |

I.R. : 3320, 1690, 1640, 1590, 1560, 1480, 1340, 1230, 1130, 750 cm-1.

EXEMPLE 5 : (5R,S)-N-(céto-4-phényl-7-hexahydro-1,2,3,3a, 4,5-pyrido[3,2,1-j,k]benzodiazépin-[1,4]-yl-5)-N′-(méthyl-3-phényl-)-urée.

Formule (I) ; A = -(CH2)3- ; R9 = H ; R10 = phényle ; R11 = H ; R12 = O ; R13 = NH ; Ar = méthyl-3-phényle.

**Stade 5a :** Hydroximino-5-phényl-7-hexahydro-1,2,3,3a,4,5-pyrido[3,2,1-j,k]benzodiazépine-[1,4]-one-4.

Formule (IV) ; A = -(CH2)3- ; R9 = H ; R10 = phényle.

Le composé est préparé de la même façon qu'à l'exemple 1, stade 1a, et à partir de 26,9 g de phényl-7-hexahydro-1,2,3,3a,4,5-pyrido[3,2,1-j,k]benzodiazépine-1,4-one-4 (97 mmoles). Après chromatographie dans du chlorure de méthylène progressivement enrichi en acétone on isole 24,3 g d'un composé solide. F = 195 °C

C.C.M. : CH2Cl2/Acétone 90/10 Rf = 0,3

CH2Cl2/Méthanol 95/5 Rf = 0.35

| R.M.N. : | | | |
|---|---|---|---|
| 9.20 ppm | s | 1 proton | NOH |
| 7.60-6.80 ppm | m | 8 protons | aromatiques |
| 4.60 ppm | m | 1 proton | CH2N |
| 3.20 ppm | m | 1 proton | CH2N |
| 2.95 ppm | m | 2 protons | CH2Ar |
| 2.OO ppm | m | 2 protons | CH2-CH2-CH2 |

I.R. : 3200, 1685, 1650, 1600, 1320, 1260, 1130, 1115cm-1.

**Stade 5b :** Amino-5-phényl-7-hexahydro-1,2,3,3a,4,5-pyrido[3,2,1-j,k]benzodiazepine-1,4-one-4.

Formule (II) ; A = -(CH2)3- ; R9 = H ; R10 = phényle.

Préparé tel qu'à l'exemple 1, stade 1b, 2ème méthode à partir de 28,8 g du composé préparé au stade ci-dessus (94 mmoles). Après chromatographie dans du chlorure de méthylène progressivement enrichi en méthanol on isole une mousse (22,0 g) n'ayant pas de point de fusion défini qui est employée directement dans les réactions suivantes.
CCM : CH2Cl2/Acétone 90/10 Rf = 0.10
CH2Cl2/Méthanol 95/5 Rf = 0.25

| R.M.N. : | | | |
|---|---|---|---|
| 7.70-7.00 ppm | m | 8 protons | aromatiques |
| 5.00 ppm | m | 2 protons | CH2N,CHNH2 |
| 3.30 ppm | m | 1 proton | CH2N |
| 2.90 ppm | m | 2 protons | CH2Ar |
| 2.62 ppm | s | 2 protons | NH2 |
| 2.40-1.70 ppm | m | 2 protons | CH2-CH2-CH2 |

I.R. : 1670, 1590, 1440, 1300, 1270 cm-1.

**Stade 5c :** (5R,S)-N-(céto-4-phényl-7-hexahydro-1,2,3,3a,4,5-pyrido[3,2,1-j,k]benzodiazépin-[1,4]-yl-5)-N'-(méthyl-3-phényl-)-urée

Ce composé est préparé à partir de la (5R,S)-Amino-5-phényl-7-hexahydro-1,2,3,3a,4,5-pyrido[3,2,1-j,k]-benzodiazépin-[1,4]-one-4 racémique préparée au stade 5b ci-dessus, de la même manière que le composé au stade 1c Ex.1 à partir de 4,37 g d'amine (15 mmoles) et de 1,99 g de méthyl-3-phénylisocyanate (15 mmoles). Après filtration du précipité et lavage par du chlorure de méthylène on obtient 5,15 g (81 %) du composé. F = 195 °C.
C.C.M. : CH2Cl2/ Acétone 95/5 Rf = 0,25.
CH2Cl2/ MeOH 95/5 Rf = 0,75 .

| R.M.N. : | | | |
|---|---|---|---|
| 9,03 ppm | s | 1 proton | CO-NH-Ar |
| 7,70-6,60 ppm | m | 13 protons | ArH, NH |
| 5,30 ppm | d | 1 proton | CO-CH-N |
| 4,60-4,15 ppm | m | 1 proton | CH2N |
| 3,40-3,05 ppm | m | 1 proton | CH2N |
| 3,05-2,6 ppm | m | 2 protons | CCH2Ar |
| 2,3 ppm | s | 3 protons | CH3Ar |
| 2,30-1,60 ppm | m | 2 protons | CH2CH2CH2 |

I.R. : 3300, 1670, 1640, 1610, 1590, 1550, 1490, 1440, 1370, 1290, 1200 cm-1.

EXEMPLE 6 : (5S)-N-(céto-4-phényl-7-hexahydro-1,2,3,3a,4,5-pyrido[3,2,1-j,k]benzodiazépin[1,4]-yl-5)-N'-(méthyl-3-phényl-)-urée.

Formule (I) ; A = -(CH2)3- ; R9 = H ; R10 = phényle ; R11 = H ; R12 = O ; R13 = NH ; Ar = méthyl-3-phényle ; isomère lévogyre .

**Stade 6a :** Phényl-7-[N-(N-tertiobutyloxy-carbonyl-L-leucyl-)-amino]-5-hexahydro-1,2,3,3a,4,5-pyrido[3,2,1-j,k]benzodiazépine-1,4-one-4.

Formule (VIII) ; A = -(CH2)3- ; R9 = H ; R10 = phényle ; R31 = isobutyl ; R32 = tertiobutyloxycarbonyle.

On dissout 47,77 g de tertiobutyloxycarbonyl-L-leucine hydratée (192 mmoles) puis 21,32 g de N-méthyl morpholine (23,2 ml soit 211 mmoles) dans 600 ml de dichlorométhane et 120 ml de DMF. On laisse sous agitation 5 minutes à température ambiante, puis refroidit à -20° C. On ajoute alors 27,4 ml de chloroformiate d'isobutyle (211 mmoles) et laisse agiter une demi-heure à -20° C. On ajoute ensuite 67,0 g d'amino-5-phényl-7-hexahydro-1,2,3,3a,4,5-pyrido[3,2,1-j,k]benzodiazépine-1,4-one-4 (230 mmoles) en solution dans 600 ml de dichlorométhane. On laisse 2 heures à -20° C et 48 heures à température ambiante. On évapore, le résidu est chromatographié sur silice dans du dichlorométhane contenant 5 % d'acétone. On obtient 99,6 g (94 %)de produit purifié.
C.C.M. : CH2Cl2/acétone 95/5 Rf = 0,60.

| R.M.N. : | | | |
|---|---|---|---|
| 8,00-7,8 ppm | d | 1 proton | NH |
| 7,7O-7,O ppm | m | 9 protons | ArH, NH |
| 5,6O-5,4 ppm | d | 1 proton | COCHN |
| 5,3O-5,1 ppm | m | 1 proton | COCHN |
| 4,70-4,15 ppm | m | 1 proton | CH2-N |
| 3,40-3,0 ppm | m | 1 proton | CH2-N |
| 3,00-2,7 ppm | m | 2 protons | CH2Ar |
| 2,50-1,6 ppm | m | 5 protons | CCH2C, CHCH2 |
| 1,50-1,3 ppm | s | 9 protons | CO-0C(CH3)3 |
| 1,00-0,8 ppm | d | 6 protons | CH(CH3 )2 |

**Stade 6b :** (N-L-leucyl-)-amino-5-phényl-7-hexahydro-1,2,3,3a,4,5-pyrido[3,2,1-j,k]benzodiazépine-1,4-one-4.

Formule (IX) ; A = -(CH2)3- ; R9 = H ; R10 = phényle ; R31 = isobutyl.

Le groupe tertiobutyloxycarbonyle est éliminé de la même façon que dans l'exemple 2 stade 2b. A partir de 100,0 g de produit du stade précédent (198 mmoles), on obtient après neutralisation du sel et chromatographie de la base 76,2 g (95 %) d'amine pure.

**Stade 6c :** Enantiomères de la (N-L-leucyl-)-amino-5-phényl-7-hexahydro-1,2,3,3a,4,5-pyrido[3,2,1-j,k]-benzodiazépine-1,4-one-4.

A partir du produit précédent, comme pour l'exemple 2 stade c, par chromatographie sur 3 kg de silice dans de l'acétate d'éthyle, et en éluant par de l'acétate d'éthyle puis par de l'acétate d'éthyle contenant 5 % de méthanol puis 10 % de méthanol. On obtient 36,4 g (90 mmoles) de dérivé lévogyre [α]D = -155°7 (c = 1 %, éthanol) et 30,8 g (0,076 mole) du dérivé dextrogyre [α]D = + 119°7 (c = 1 %, éthanol).

**Stade 6d :** [N-(N-phényl-thiouréido-)-L-leucyl-]-amino-5-phényl-7-hexahydro-1,2,3,3a,4,5-pyrido [3,2,1-j,k]-benzodiazépine-1,4-one-4.

Formule (X) ; A = -(CH2)3- ; R9 = H ; R10 = R34 = phényle; R31 = isobutyle.

On opère de la même façon qu'à l'exemple 2 stade 2d, avec 36,4 g d'amine lévogyre (90 mmoles) du stade précédent, on obtient 39,2 g de produit (81 %).

| R.M.N. : | | | |
|---|---|---|---|
| 8,45-8,35 ppm | s | 1 proton | CH-NH-CO |
| 7,90-7,70 ppm | d | 1 proton | NH |
| 7,60-7,00 ppm | m | 13 protons | ArH |
| 7,00-6,80 ppm | d | 1 proton | NH |
| 5,50-5,20 ppm | d+m | 2 protons | NCHCO,NCHCO |
| 4,65-4,30 ppm | m | 1 proton | CH2N |
| 3,40-3,00 ppm | m | 1 proton | CH2N |
| 3,00-2,70 ppm | m | 2 protons | CH2Ar |
| 2,20-1,40 ppm | m | 5 protons | CCH2C,CHCH2 |
| 1,05-0,80 ppm | m | 6 protons | (CH3)2 |

**Stade 6e :** (-)-Amino-(5S)-phényl-7-hexahydro-1,2,3,3a,4,5-pyrido[3,2,1-j,k]benzodiazépine-1,4-one-4.

Formule (II);A=-(CH2)3- ; R9=H ; R10=phényle ; isomère lévogyre.

On opère de la même façon qu'à l'exemple 2 stade 2e, à partir de 40 g (74 mmoles) de produit du stade 6d. On obtient après purification et neutralisation 16,8 g (78 %)de composé. [α]D = - 271° ( c = 1%, éthanol).

| R.M.N. : | | | |
|---|---|---|---|
| 7,70-6,90 ppm | m | 8 protons | ArH |
| 4,70-4,30 ppm | m | 1 proton | CH2N |
| 4,45 ppm | s | 1 proton | NCHCO |
| 3,40-3,00 ppm | m | 1 proton | CH2N |
| 3,00-2,70 ppm | m | 2 protons | CH2Ar |
| 2,70-2,50 ppm | s | 2 protons | NH2 |
| 2,40-1,70 ppm | m | 2 protons | CH2CH2 |
| | | | CH2 |

**Stade 6f : (produit de l'exemple)** (5S)-N-(céto-4-phényl-7-hexahydro-1,2,3,3a,4,5-pyrido[3,2,1-j,k]-benzodiazépin[1,4]-yl-5)-N′-(méthyl-3-phényl-)-urée.

Dans un ballon de 250 ml équipé d'une arrivée d'azote, d'une garde à chlorure de calcium et d'un agitateur magnétique on introduit 150 ml de dichlorométhane et 8,8 g de l'amine lévogyre préparée ci-dessus (23,3 mmoles). On ajoute rapidement 3,1 g d'isocyanate de méthyl-3-phényle (23,3 mmoles) à température ambiante. On laisse une heure sous agitation. On évapore puis chromatographie sur silice en éluant par du dichlorométhane enrichi en acétone (95/5) pour obtenir 9,0 g de solide. F = 190° .
C.C.M. : CH2Cl2/acétone 95/5 Rf = 0,35 . [α]D = - 98° (c = 1 %, éthanol).

| R.M.N. : | | | |
|---|---|---|---|
| 7,95 ppm | s | 1 proton | CO-NH-Ar |
| 7,70-6,70 ppm | m | 13 protons | ArH,NH |
| 5,60-5,80 ppm | d | 1 proton | CO-CH-N |
| 4,70-4,30 ppm | m | 1 proton | CH2-N |
| 3,45-3,05 ppm | m | 1 proton | CH2-N |
| 3,0-2,80 ppm | m | 2 protons | C-CH2-Ar |
| 2,30 ppm | s | 3 protons | CH3 |
| 2,30-1,70 ppm | m | 2 protons | CH2CH2CH2 |

EXEMPLE 7 : (+)-(5R)-N-(céto-4-phényl-7-hexahydro-1,2,3,3a,4,5-pyrido[3,2,1-j,k]benzodiazépin[1,4]-yl-5)-N′-(méthyl-3-phényl-)-urée.

Formule I ; A = -(CH2)3- ; R9 = H ; R10 = phényle ; R11 = H ; R12 = O ; R13 = NH ; Ar = méthyl-3-phényle ; isomère dextrogyre .

**Stade 7a :** [N-(N-phényl-thiouréido-)-L-leucyl-]-amino-5-phényl-7-hexahydro-1,2,3,3a,4,5-pyrido[3,2,1-j,k]-benzodiazépine-1,4-one-4.

Formule (X) ; A = -(CH2)3- ; R9 = H ; R10 = R34 = phényle; R31 = isobutyle.

On opère de la même façon que pour l'exemple 2 stade 2d, sur 30,8 g d'amine dextrogyre de l'exemple 6 stade 6c (76 mmoles). On obtient 39,0 g de produit (95 %).

| R.M.N. : | | | |
|---|---|---|---|
| 8,45-8,35 ppm | s | 1 proton | CH-NH-CO |
| 7,90-7,70 ppm | d | 1 proton | NH |
| 7,60-7,00 ppm | m | 14 protons | ArH, NH |
| 5,50-5,20 ppm | d+m | 2 protons | NCHCO,NCHCO |
| 4,60-4,20 ppm | m | 1 proton | CH2N |
| 3,40-2,95 ppm | m | 1 proton | CH2N |
| 3,00-2,70 ppm | m | 2 protons | CH2Ar |
| 2,20-1,40 ppm | m | 5 protons | CCH2C,CHCH2 |
| 1,05-0,80 ppm | m | 6 protons | (CH3)2 |

**Stade 7b** : Préparation de la(+)-amino-(5R)-phényl-7-hexahydro-1,2,3,3a,4,5-pyrido[3,2,1-j,k]-benzodiazépine-1,4-one-4.

Formule (II) ; A = -(CH2)3- ; R9 = H ; R10 = phényle ; R14 = O ; isomère dextrogyre .

On opère de la même façon qu'au stade 2, exemple 2 à partir de 41,0 g (76 mmoles) de produit du stade 7a. On obtient après purification et neutralisation 14,2 g (68 %) [αD] = + 257° (c = 1 %, éthanol).

| R.M.N. : | | | |
|---|---|---|---|
| 7,70-6,90 ppm | m | 8 protons | ArH |
| 4,70-4,30 ppm | m | 1 proton | CH2N |
| 4,45 ppm | s | 1 proton | NCHCO |
| 3,40-3,00 ppm | m | 1 proton | CH2N |
| 3,00-2,70 ppm | m | 2 protons | CH2Ar |
| 2,70-2,50 ppm | s | 2 protons | NH2 |
| 2,40-1,70 ppm | m | 2 protons | CH2CH2<br>CH2 |

**Stade 7c** : (+)-(5R)-N-(céto-4-phényl-7-hexahydro-1,2,3,3a,4,5-pyrido[3,2,1-j,k]benzodiazépin[1,4]-yl-5)-N'-(méthyl-3-phényl-)-urée.

On opère de la même façon qu'à l'exemple 2 stade 2f, à partir de 8,8 g (30,2 mmoles) de produit du stade 7b. On obtient après purification 11,5 g (90 %) d'un solide fondant à 180° C.
[α]D = + 94° (c = 1 % , éthanol).
C.C.M. : CH2Cl2/Acétone 95/5 Rf = 0,35 .

| R.M.N. : | | | |
|---|---|---|---|
| 7,90 ppm | s | 1 proton | CO-NH-Ar |
| 7,70-6,70 ppm | m | 13 protons | ArH,NH |
| 5,80-5,60 ppm | d | 1 proton | CO-CH-N |
| 4,70-4,30 ppm | m | 1 proton | CH2-N |
| 3,45-3,05 ppm | m | 1 proton | CH2-N |
| 3,0-2,70 ppm | m | 2 protons | C-CH2-Ar |
| 2,30 ppm | s | 3 protons | CH3 |
| 2,30-1,70 ppm | m | 2 protons | CH2CH2CH2 |

EXEMPLE 8 : (5R,S)-N-(céto-4-phényl-7-hexahydro-1,2,3,3a,4,5-pyrido[3,2,1-j,k]benzodiazépin-[1,4]-yl-5)-N'-(nitro-4-phényl-)-urée.

Formule (I) ; A = -(CH2)3- ; R9 = H ; R10 = phényle ; R11 = H ; R12 = O ; R13 = NH ; Ar = nitro-4-phényle.

Ce composé est préparé à partir de la (5R,S)-amino-5-phényl-7-hexahydro-1,2,3,3a,4,5-pyrido[3,2,1-j,k]-benzodiazépin-[1,4]-one-4 racémique, de la même manière que pour la préparation du composé de l'Ex.1.

A partir de 3,60 g d'amine (12 mmoles) et de 2,02 g de nitro-4-phénylisocyanate (12 mmoles), on obtient 3,7 g (70 %) du composé. F > 290° C.

C.C.M. : CH2Cl2/Acétone 90/10 Rf = 0,55 .

| R.M.N. : | | | |
|---|---|---|---|
| 9,85 ppm | s | 1 proton | CONHAr |
| 8,2 ppm | d | 1 proton | NH |
| 8,00-7,00 ppm | m | 12 protons | ArH |
| 5,30 ppm | d | 1 proton | COCHN |
| 4,60-4,20 ppm | m | 1 proton | CH2N |
| 3,50-3,10 ppm | m | 1 proton | CH2N |
| 3,10-2,60 ppm | m | 2 protons | CCH2Ar |
| 2,40-1,60 ppm | m | 2 protons | CCH2C |

I.R. : 3300, 2280, 1660, 1610, 1560, 1490, 1440, 1380, 1320, 1200, 1160, 820 cm-1.

EXEMPLE 9 : (5R,S)-N-(céto-4-phényl-7-hexahydro-1,2,3,3a,4,5-pyrido[3,2,1-j,k]benzodiazépin-[1,4]-yl-5)-N'-(fluoro-4-nitro-3-phényl-)-urée.

Formule I ; A = -(CH2)3- ; R9 = H ; R10 = phényle ; R11 = H ; R12 = O ; R13 = NH ; Ar = fluro-4-nitro-3-phényle .

Ce composé est préparé à partir de la (5R,S)-amino-5-phényl-7-hexahydro-1,2,3,3a,4,5-pyrido[3,2,1-j,k]-benzodiazépin-[1,4]-one-4 racémique, de la même manière qu'à l'exemple 1.

A partir de 4,0 g d'amine (13,7 mmoles) et de 2,50 g de fluoro-4-nitro-3-phénylisocyanate (13,7 mmoles), on obtient 6,0 g (92 %) du composé sous forme d'un solide blanc. F > 290° C.

C.C.M. : CH2Cl2/Acétone 95/5 Rf = 0,15 .

| R.M.N. : | | | |
|---|---|---|---|
| 8,20 ppm | d | 1 proton | CONHAr |
| 7,90 ppm | s | 1 proton | CNHCO |
| 7,70-7,00 ppm | m | 11 protons | ArH |
| 5,55 ppm | d | 1 proton | COCHN |
| 4,50-4,30 ppm | m | 1 proton | CH2N |
| 3,30-3,10 ppm | m | 1 proton | CH2N |
| 3,00-2,65 ppm | m | 2 protons | CCH2Ar |
| 2,20-1,70 ppm | m | 2 protons | CCH2C |

I.R. : 3300, 3050, 1640, 1600, 1500, 1440, 1300, 1260, 1200, 1160, 810 , 800 cm-1.

EXEMPLE 10 : (5R,S)-N-(céto-4-phényl-7-hexahydro-1,2,3,3a,4,5-pyrido[3,2,1-j,k]benzodiazépin-[1,4]-yl-5)-N'-(bromo-4-phényl-)-thiourée.

Formule (I) ; A = -(CH2)3- ; R9 = H ; R10 = phényle ; R11 = H ; R12 = S ; R13 = NH ; Ar = bromo-4-phényle .

On dissout 5,1 g de (4R,S)-amino-5-phényl-7-hexahydro-1,2,3,3a,4,5-pyrido[3,2,1-j,k]benzodiazépin-[1,4]-one-4 (17.5 mmoles) dans 50 ml de chlorure de méthylène puis on ajoute 3,75 g d'isothiocyanate de bromo-4-phényle (17.5 mmoles). Après 45 minutes d'agitation à la température ambiante on évapore le solvant. On chromatographie sur une colonne de silice dans un mélange de chlorure de méthylène contenant 5 % en volume d'acétone. On évapore et le résidu est repris dans l'éthanol. La solution est filtrée puis évaporée. On obtient 4,3 g (50 %)du composé. F = 215° C.
C.C.M. : CH2Cl2/Acétone 95/5 Rf = 0,40.

R.M.N. :

| | | | | | |
|---|---|---|---|---|---|
| 9,2-8,5 | ppm | s | 1 | proton | CO-NH-Ar |
| 7,8-7,1 | ppm | m | 13 | protons | ArH,NH |
| 5,85 | ppm | | 1 | proton | CSCHN |
| 4,6-4,2 | ppm | m | 1 | proton | CH2N |
| 3,4-3,1 | ppm | m | 1 | proton | CH2N |
| 3-2,7 | ppm | m | 2 | protons | CH2Ar |
| 2,2-1,6 | ppm | s | 2 | protons | CH2CH2CH2 |

I.R. :3350, 1660, 1560, 1480, 1380, 1230, 1170 cm-1.

EXEMPLE 11 : (5R,S)-N-(céto-4-phényl-7-hexahydro-1,2,3,3a,4,5-pyrido[3,2,1-j,k]benzodiazépin[1,4]-yl-5)-N'-(méthyl-3-phényl-)-thiourée.

Formule (I) ; A = -(CH2)3- ; R9 = H ; R10 = phényle ; R11 = H ; R12 = S ; R13 = NH ; Ar = méthyl-3-phényle .

Ce produit est préparé de la même manière que dans l'exemple précédent à partir de 4,0 g de (4R,S)-amino-5-phényl-7-hexahydro-1,2,3,3a,4,5-pyrido[3,2,1-j,k]benzodiazépin[1,4]-one-4 (13,7 mmoles) et de 2,05 g d'isothiocyanate de méthyl-3-phényle (13,7 mmoles). Le produit est purifié par chromatoflash, l'éluant étant du dichlorométhane contenant 2,5 % d'acétone. On obtient ainsi 4,7 g du produit désiré. (78,5 %). F = 175° C .
C.C.M. : CH2Cl2/Acétone 95/5 Rf = 0,35

| R.M.N. : | | | |
|---|---|---|---|
| 10,2 ppm | s | 1 proton | NH |
| 8,8 ppm | d | 1 proton | NH |
| 7,7-6,7 ppm | m | 12 protons | ArH |
| 6-5,9 ppm | d | 1 proton | COCHN |
| 4,6-4,2 ppm | m | 1 proton | CH2N |
| 3,4-3,1 ppm | m | 1 proton | CH2N |
| 3,1-2,7 ppm | m | 2 protons | CCH2Ar |
| 2,3 ppm | s | 3 protons | CH3 |
| 2,2-1,7 ppm | m | 2 protons | CH2CH2CH2 |

I.R. : 3350, 3150, 2950, 1660, 1580, 1540, 1440, 1280, 1250, 1180 cm-1.

EXEMPLE 12 : (5R,S)-N-(céto-4-phényl-7-hexahydro-1,2,3,3a,4,5-pyrido[3,2,1-j,k]benzodiazépin-[1,4]-yl-5)-N'-(méthyl-2-phényl-)-urée.

Formule (I) ; A = -(CH2)3- ; R9 = H ; R10 = phényle ; R11 = H ; R12 = O ; R13 = NH ; Ar = méthyl-2-phényle .

Ce composé est préparé à partir de la (5R,S)-amino-5-phényl-7-hexahydro-1,2,3,3a,4,5-pyrido[3,2,1-j,k]-benzodiazépin-[1,4]-one-4 racémique, de la même manière que pour la préparation du composé de l'exemple 1.

A partir de 2,91 g d'amine (10 mmoles) et de 1,33 g de méthyl-2-phénylisocyanate (10 moles). On obtient 3,00 g (75 %) du composé sous forme d'un solide. F = 190° C
C.C.M. : CH2Cl2/Acétone 90/10 Rf = 0,50.
CH2Cl2/MeOH 95/5 Rf = 0,45.

| R.M.N. : | | | |
|---|---|---|---|
| 8,70-7,00 ppm | m | 14 protons | ArH,NHCONH |
| 5,53 ppm | d | 1 proton | COCHN |
| 4,60-4,20 ppm | m | 1 proton | CH2N |
| 3,30-2,90 ppm | m | 1 proton | CH2N |
| 2,90-2,60 ppm | m | 2 protons | CCH2Ar |
| 2,20-1,60 ppm | m | 2 protons | CCH2C |
| 2,10 ppm | s | 3 protons | CH3Ar |

I.R. : 3300, 2920, 1670, 1640, 1580, 1515, 1480, 1470, 1290, 1160, 740 cm-1.

EXEMPLE 13 : (5R,S)-N-(céto-4-phényl-7-hexahydro-1,2,3,3a,4,5-pyrido[3,2,1-j,k]benzodiazépin-[1,4]-yl-5)-N'-(méthyl-4-phényl-)-urée.

Formule (I) ; A = -(CH2)3- ; R9 = H ; R10 = phényle ; R11 = H ; R12 = O ; R13 = NH ; Ar = méthyl-4-phényle.

Ce composé est préparé à partir de la (5R,S)-amino-5-phényl-7-hexahydro-1,2,3,3a,4,5-pyrido[3,2,1-j,k]-benzodiazépin-[1,4]-one-4 racémique, de la même manière que pour la préparation du composé de l'exemple 1.

A partir de 2,91 g d'amine (10 mmoles) et de 1,33 g de méthyl-4-phénylisocyanate (10 mmoles). On obtient 3,10 g (73 %) du composé sous forme d'un solide. F = 280° C.
C.C.M. : CH2Cl2/Acétone 90/10 Rf = 0,46 .
CH2Cl2/MeOH 95/5 Rf = 0,65 .

| R.M.N. : | | | |
|---|---|---|---|
| 9,00 ppm | s | 1 proton | CONHAr |
| 7,60-6,90 ppm | m | 13 protons | ArH,NH |
| 5,30 ppm | d | 1 proton | COCHN |
| 4,60-4,10 ppm | m | 1 proton | CH2N |
| 3,40-3,05 ppm | m | 1 proton | CH2N |
| 3,05-2,60 ppm | m | 2 protons | CCH2Ar |
| 2,40-1,60 ppm | m | 2 protons | CCH2C |
| 2,25 ppm | s | 3 protons | ArCH3 |

I.R. : 3400, 3050, 2700, 2500, 1600, 1540, 1490, 1440, 1365, 1300, 1180, 800 cm-1.

EXEMPLE 14 : (5R,S)-N-(céto-4-phényl-7-hexahydro-1,2,3,3a,4,5-pyrido[3,2,1-j,k]benzodiazépin-[1,4]-yl-5)-N′-(trifluoro-méthyl-2-phényl-)-urée.

Formule (I) ; A = -(CH2)3- ; R9 = H ; R10 = phényle ; R11 = H ; R12 = O ; R13 = NH ; Ar = trifluoro-méthyl-2-phényle.

Ce composé est préparé à partir de la (5R,S)-amino-5-phényl-7-hexahydro-1,2,3,3a,4,5-pyrido[3,2,1-j,k]-benzodiazépin-[1,4]-one-4 racémique, de la même manière que pour la préparation du composé de l'exemple 1.

A partir de 2,91 g d'amine (10 mmoles) et de 1,87 g de trifluorométhyl-2-phénylisocyanate (10 mmoles). On obtient 3,6 g (75 %) du composé . F > 290° C.

C.C.M. : CH2Cl2/Acétone 95/5 Rf = 0,30 .

CH2Cl2/MeOH 95/5 Rf = 0,50 .

| R.M.N. : | | | |
|---|---|---|---|
| 7,95-7,05 ppm | m | 14 protons | ArH,NHCONH |
| 5,27 ppm | d | 1 proton | COCHN |
| 4,55-4,15 ppm | m | 1 proton | CH2N |
| 3,55-3,05 ppm | m | 1 proton | CH2N |
| 3,05-2,70 ppm | m | 2 protons | CCH2Ar |
| 2,30-1,60 ppm | m | 2 protons | CCH2C |

I.R. : 3350, 3050, 1660, 1600, 1520, 1440, 1370, 1310, 1200, 1150, 1100, 740 cm-1 .

EXEMPLE 15 : (5R,S)-N-(céto-4-phényl-7-hexahydro-1,2,3,3a,4,5-pyrido[3,2,1-j,k]benzodiazépin-[1,4]-yl-5)-N′-(naphtyl-1-phényl-)-urée.

Formule (I) ; A = -(CH2)3- ; R9 = H ; R10 = phényle ; R11 = H ; R12 = O ; R13 = NH ; Ar = naphtyle-1-.

Ce composé est préparé à partir de la (5R,S)-amino-5-phényl-7hexahydro-1,2,3,3a,4,5-pyrido[3,2,1-j,k]-benzodiazépin-[1,4]-one-4 racémique, de la même manière que pour la préparation de l'exemple 1.

A partir de 2,91 g d'amine (10 mmoles) et de 1,69 g de naphtyl-1-isocyanate (10 mmoles). On obtient 3,9 g (85 %) du composé . F = 190° C.

C.C.M. : CH2Cl2/Acétone 95/5 Rf = 0,15.

CH2Cl2/MeOH 95/5 Rf = 0,35 .

| R.M.N. : | | | |
|---|---|---|---|
| 9,20 ppm | s | 2 protons | CONHAr |
| 8,40-7,10 ppm | m | 15 protons | ArH,NH |
| 5,40 ppm | d | 1 proton | COCHN |
| 4,60-4,10 ppm | m | 1 proton | CH2N |
| 3,40-3,05 ppm | m | 1 proton | CH2N |
| 3,05-2,60 ppm | m | 2 protons | CCH2Ar |
| 2,40-1,60 ppm | m | 2 protons | CCH2C |

I.R. : 3300, 3030, 3000, 1670, 1640, 1530, 1440, 1380, 1200, 760 cm-1.

EXEMPLE 16 : (5R,S)-N-(céto-4-phényl-7-hexahydro-1,2,3,3a,4,5-pyrido[3,2,1-j,k]benzodiazépin[1,4]-yl-5)-N -(bromo-2-phényl-)-thiourée.

Formule (I) ; A = -(CH2)3- ; R9 = H ; R10 = phényle ; R11 = H ; R12 = S ; R13 = NH ; Ar = bromo-2-phényle.

Ce produit est préparé de la même manière que dans l'exemple 23 à partir de 3,00 g de (4R,S)-amino -5-phényl-7-hexahydro-1,2,3,3a,4,5-pyrido[3,2,1-j,k]benzodiazépin-[1,4]-one-4 (10,3 mmoles) et de 2,20 g d'isothiocyanate de bromo-2-phényle (10,3 mmoles). Le produit est purifié par chromatoflash, l'éluant étant du dichlorométhane contenant 2,5 % d'acétone. On obtient ainsi 4,07 g du produit désiré (77 %). F = 180 °C .
C.C.M. : CH2Cl2/Acétone 95/5 Rf = 0,5 .
CH2Cl2/MeOH 95/5 Rf = 0,85 .

| R.M.N. : | | | |
|---|---|---|---|
| 8,20-6,90 ppm | m | 14 protons | ArH,NHCSNH |
| 6,05 ppm | d | 1 proton | COCHN |
| 4,70-4,30 ppm | m | 1 proton | CH2N |
| 3,40-3,00 ppm | m | 1 proton | CH2N |
| 3,05-2,70 ppm | m | 2 protons | CCH2Ar |
| 2,40-1,70 ppm | m | 2 protons | CH2CH2CH2 |

I.R. : 3300, 2900, 1660, 1580, 1520, 1440, 1280, cm-1.

EXEMPLE 17 : (4R,S)-N-(céto-3-phényl-6-tetrahydro-1,2,3,4-pyrrolo[3,2,1-j,k]benzodiazépin[1,4]-yl-4)-N′-(bromo-4-phényl-)-thiourée.

Formule (I) ; A = -(CH2)2- ; R9 = H ; R10 = phényle ; R11 = H ; R12 = S ; R13 = NH ; Ar = bromo-4-phényle .

Ce composé est préparé à partir de la (4R,S)-amino-4-phényl-6-tetrahydro-1,2,3,4-pyrrolo[3,2,1-j,k]-benzodiazépin-[1,4]-one-3 préparée au stade 1b de l'Ex.1 (2ème méthode) et en opérant de la même manière que pour le composé au stade Ic. A partir de 4,0 g d'amine (14,4 mmoles) et de 3,4 g de chloro-4-phénylisothiocyanate (14,4 mmoles). Après purification par chromatographie sur silice dans le chlorure de méthylène enrichi en acétone on obtient 5,0 g (83 %) du composé sous forme solide.
F = 160 °C
C.C.M. : CH2Cl2/Acétone 80/20 Rf = 0,6 .

| R.M.N. : | | | |
|---|---|---|---|
| 8,70 ppm | m | 1 proton | NH |
| 8,50 ppm | d | 1 proton | NH |
| 7,40-7,00 ppm | m | 12 protons | ArH |
| 5,95 ppm | d | 1 proton | CHN |
| 4,80-4,30 ppm | m | 1 proton | CH2 |
| 4,30-3,65 ppm | m | 1 proton | CH2 |
| 3,60-2,90 ppm | m | 2 protons | CCH2 |

I.R. : 3300, 1675, 1600, 1505, 1490, 1470, 1395, 1240 cm-1

EXEMPLE 18 : (4R,S)-N-(céto-3-phényl-6-tetrahydro-1,2,3,4-pyrrolo[3,2,1-j,k]benzodiazépin[1,4]-yl-4)-N′-(bromo-3-phényl-)-thiourée.

Formule (I) ; A = -(CH2)2- ; R9 = H ; R10 = phényle ; R11 = H ; R12 = S ; R13 = NH ; Ar = bromo-3-phényle .

Ce composé est préparé à partir de la (4R,S)-amino-4-phényl-6-tetrahydro-1,2,3,4-pyrrolo[3,2,1-j,k]-benzodiazépin-[1,4]-one-3. A partir de 6,0 g d'amine (21,6 mmoles) et de 4,69 g de bromo-3-phénylisothiocyanate (14,4 mmoles). On obtient après chromatographie sur silice en éluant par le chlorure de méthylène enrichi en acétone 7,3 g (69 %) de solide. F = 135° C
C.C.M. : CH2Cl2/Acétone 97/3 Rf = 0,30 .

| R.M.N. : | | | |
|---|---|---|---|
| 8,70 ppm | m | 1 proton | NH |
| 8,45 ppm | d | 1 proton | NH |
| 7,70-7,00 ppm | m | 12 protons | ArH |
| 6,0 ppm | d | 1 proton | CHN |
| 4,70-4,40 ppm | m | 1 proton | CH2 |
| 4,10-3,60 ppm | m | 1 proton | CH2 |
| 3,60-2,80 ppm | m | 2 protons | CCH2 |

I.R. : 3300, 1670, 1510, 1485, 1470, 1240, 1170, 700 cm-1.

EXEMPLE 19 : (4R,S)-N-(céto-3-(méthyl-4-phényl)-6-tetrahydro-1,2,3,4-pyrrolo[3,2,1-j,k]benzodiazépin[1,4]-yl-4)-N′-(bromo-4-phényl-)-thiourée.

Formule (I) ; A = -(CH2)2- ; R9 = H ; R10 = CH34-C6H4 ; R11 = H ; R12 = S ; R13 = NH ; Ar = bromo-4-phényle.

Stade 19a : Transformation d'un composé (III) en composé (IV).

De la même manière qu'au stade 3a de l'exemple 3 le composé est préparé à partir de 134 g (1,125 mole) d'indoline et de 253 g (2,16 mole) de méthyl-4-benzonitrile. On obtient 249 g (93 %) de méthyl-4-benzoyl-7-indoline. F = 106° C
C.C.M. : CH2Cl2 Rf = 0,56 .

| R.M.N. : | | | |
|---|---|---|---|
| 7,70-7,10 ppm | m | 7 protons | ArH |
| 3,70 ppm | t | 2 protons | CH2 |
| 3,10 ppm | t | 2 protons | CH2 |
| 2,40 ppm | s | 3 protons | ArCH3 |

I.R. : 3350, 1620, 1600,1500, 1460, 1390, 1290, 1260, 1010, 740 cm-1.

Stade 19b : N-bromoacétyl-(méthyl-4-benzoyl)-7-indoline qui est préparée à partir de 249 g (1,05 mole) du produit préparé au stade 34a ci-dessus en opérant de la même manière qu'au stade 3b, on obtient 265 g (70 %) de solide.

C.C.M. : CH2Cl2/Acétone 95/5 Rf = 0,70 .

| R.M.N. : | | | |
|---|---|---|---|
| 7,85-7,10 ppm | m | 7 protons | ArH |
| 4,45-4,15 ppm | t | 2 protons | CH2 |
| 3,75 ppm | s | 2 protons | BrCH2CO |
| 3,35-3,00 ppm | t | 2 protons | CH2 |
| 2,40 ppm | s | 3 protons | ArCH3 |

I.R. : 1660, 1600,1580, 1450, 1400, 1280, 990, 870, 830, 740 cm-1.

Stade 19c : (méthyle-4-phényl-)-6-tetrahydro-1,2,3,4-pyrrolo[3,2,1-j,k]benzodiazépin[1,4]-one-3.

(III) ; A = -(CH2)2- ; R9 = H ; R10 = CH34-C6H4.

A partir de 266 g (743 mmoles) de produit du stade ci-dessus, en opérant de la même manière qu'a l'exemple 3 on obtient 143,5 g (70 %) de produit solide. F = 203° C

C.C.M. : CH2Cl2/Acétone 95/5 Rf = 0,3.

| R.M.N. : | | | |
|---|---|---|---|
| 7,50-6,90 ppm | m | 7 protons | ArH |
| 4,40-4,15 ppm | t | 2 protons | CH2 |
| 4.35 ppm | s | 2 protons | COCH2N |
| 3.30-3.05 ppm | t | 2 protons | CH2 |
| 3.40 ppm | s | 3 protons | ArCH3 |

I.R. : 1670, 1600, 1450, 1385, 1345, 1305, 1040, 820, 755 cm-1.

Stade 19d : Hydroxymino-4-(méthyl-4-phényl-)-6-tetrahydro-1,2,3,4-pyrrolo[3,2,1-j,k]benzodiazépin[1,4]-one-3

(IV) ; A = -(CH2)2- ; R9 = H ; R10 = CH34-C6H4.

A partir de 143 g (517 mmoles) de produit du stade ci-dessus on obtient 117 g (82 %) de produit. F = 202° C (décomposition)

C.C.M. : CH2Cl2/Acétone 95/5 Rf = 0,24 .

| R.M.N. : | | | |
|---|---|---|---|
| 7,70-7,00 ppm | m | 7 protons | ArH |
| 4,40-4,10 ppm | m | 2 protons | CH2 |
| 3,85 ppm | s | 1 proton | |
| 3,40-3,00 ppm | m | 2 protons | CH2 |
| 2,40 ppm | s | 3 protons | ArCH3 |

I.R. : 3200, 1680, 1600, 1440, 1385, 1345, 1300, 1230, 1010, 920, 800, 740 cm-1.

Stade 19e : amino-4-(méthyl-4-phényl-)-6-tetrahydro-1,2,3,4-pyrrolo[3,2,1-j,k]benzodiazépin [1,4]-one-3.

FORMULE (II) ; A = -(CH2)2- ; R9 = H ; R10 = CH34-C6H4 ;

A partir de 35 g (l2 mmoles) du composé du stade 34d et en opérant de la même façon qu'au stade 1b 2ème méthode, après chromatographie on obtient 24,5 g (70 %) du composé souhaité sous forme solide.
F = 115 °C
C.C.M. : CH2Cl2/Acétone 80/20 Rf = 0,20 .

| R.M.N. : | | | |
|---|---|---|---|
| 7,60-7,00 ppm | m | 7 protons | ArH |
| 4,80-4,50 ppm | m | 2 protons | CH2 |
| 4,35 ppm | s | 1 proton | CHN |
| 4,20-3,70 ppm | m | 2 protons | CH2 |
| 3,60-3,00 ppm | m | 2 protons | CH2 |
| 2,75 ppm | s | 2 protons | |
| 2,38 ppm | s | 3 protons | CH3 |

I.R. : 3350, 2900, 1680, 1600, 1445, 1390, 1240, 820, 760, 735 cm-1.

Stade 19f : à partir de 6 g (20,6 mmoles) de produit du stade précédent, et de 44 g (20,6 mmoles) de bromo-4-phényl isocyanate, en opèrant de de la même manière qu'à l'exemple-1 stade 1c. Après purification par chromatographie sur silice dans le chlorure de méthylène enrichi en acétone, on obtient 7 g de solide (67 %).
F = 150 °C.
C.C.M. : CH2Cl2/Acétone 97/30 Rf = 0,46 .

| R.M.N. : | | | |
|---|---|---|---|
| 8,71 ppm | s | 1 proton | NH |
| 8,15 ppm | d | 1 proton | NH |
| 7,60-7,00 ppm | m | 11 protons | ArH |
| 5,95 ppm | d | 1 proton | NCHCO |
| 4,80-4,40 ppm | m | 1 proton | CH2 |
| 4,10-3,60 ppm | m | 1 proton | CH2 |
| 3,60-2,90 ppm | m | 2 protons | CH2 |
| 2,38 ppm | s | 3 protons | CH3 |

I.R. : 3300, 1675, 1600, 1510, 1490,1300, 1240, 1060, 1000, 820, 740 cm-1.

EXEMPLE 20 : (5R,S)-N-(céto-4-phényl-7-hexahydro-1,2,3,3a, 4,5-pyrido[3,2,1-j,k]benzodiazépin[1,4]-yl-5)-N'-(pyridyl-3-)-urée.

Formule (I) ; A = -(CH2)3- ; R9 = H ; R10 = C6H5 ; R11 = H ; R12 = O ; R13 = NH ; Ar = pyridyl-3.

1,5 g (16 mmoles) d'amino-3-pyridine sont dissous dans 80 ml de chlorure de méthylène. On ajoute 2,6 g (16 mmoles) de N,N'-carbonyldiimidazole et agite 3 heures à température ambiante. On refoidit à 0° C et ajoute une solution de 4,6 g (16 mmoles) d'amine obtenue au stade 5b Ex.5, dans 40 ml de chlorure de méthylène. On filtre le précipité qui s'est formé et le chromatographie sur silice dans du chlorure de méthylène enrichi en méthanol. On obtient 4,25 g (65 %) de solide . F = 280° C.
C.C.M. : CH2Cl2/Méthanol 90/10 Rf = 0,5 .

| R.M.N. : | | | |
|---|---|---|---|
| 9,3 ppm | s | 1 proton | NH |
| 8,6-7,0 ppm | m | 13 protons | ArH,NH |
| 5,3 ppm | d | 1 proton | COCHN |
| 4,55-4,10 ppm | m | 1 proton | CH2 |
| 3,40-3,0 ppm | m | 1 proton | CH2 |
| 3,0-2,15 ppm | m | 2 protons | CH2 |
| 2,3-1,5 ppm | m | 2 protons | CH2 |

I.R. : 3320, 1670, 1640,1595, 1540, 1475, 1300, 1200, 810, 690 cm-1.

EXEMPLE 21 : (5R,S)-N-(céto-4-phényl-7-hexahydro-1,2,3,3a, 4,5-pyrido[3,2,1-j,k]benzodiazépin[1,4]-yl-5)-N'-(pyridyl-4-)-urée.

Formule (I) ; A = -(CH2)3- ; R9 = H ; R10 = C6H5 ; R11 = H ; R12 = O ; R13 = NH ; Ar = pyridyl-4.

On opère de la même façon que dans l'exemple ci-dessus, à partir de 1,7 g (13,7 mmoles) d'amino-4-pyridine de 4,6 g (13,7 mmoles) d'amine obtenue au stade 5b Ex.5, et de 2,43 g (15 mmoles) de N,N'-carbonyldiimidazole. Après chromatographie sur silice dans du chlorure de méthylène enrichi en acétone, on isole 4,9 g (87 %) de solide fondant au dessus de 280° C avec décomposition.
C.C.M. : CH2Cl2/Acétone 95/5 Rf = 0,35 .

| R.M.N. : | | | |
|---|---|---|---|
| 9,52 ppm | s | 1 proton | NH |
| 8,32 ppm | d | 1 proton | NH |
| 7,90-6,80 ppm | m | 12 protons | ArH |
| 5,3 ppm | d | 1 proton | COCHN |
| 4,55-4,10 ppm | m | 1 proton | CH2 |
| 3,50-3,05 ppm | m | 1 proton | CH2 |
| 3,0-2,5 ppm | m | 2 protons | CH2 |
| 2,4-1,5 ppm | m | 2 protons | CH2 |

I.R. : 3300, 1660, 1590,1520, 1380, 1200, 820, 740, 690 cm-1.

EXEMPLE 22 : (5R,S)-N-(céto-4-phényl-7-hexahydro-1,2,3,3a,4,5-pyrido[3,2,1-j,k]benzodiazépin[1,4]-yl-5)-N'-(bromo-3-phényl-)-thiourée.

Formule (I) ; A = -(CH2)3- ; R9 = H ; R10 = C6H5 ; R11 = H ; R12 = S ; R13 = NH ; Ar = bromo-3-phényle .

On opère de la même manière qu'à l'exemple 5, en partant de 5,8 g (20 mmoles) de l'amine obtenue au stade 5b, et de 4,28 g (20 mmoles) de bromo-3 phényle isothio cyanate. Après chromatographie sur silice dans du chlorure de méthylène enrichi en acétone. On obtient 5,0 g (50 %) de solide. F = 185° C.
C.C.M. : CH2Cl2/Acétone 95/5 Rf = 0,60 .

| R.M.N. : | | | |
|---|---|---|---|
| 10,4 ppm | s | 1 proton | NH |
| 9,0 ppm | s | 1 proton | NH |
| 8,2-7,0 ppm | m | 12 protons | ArH |
| 5,9 ppm | d | 1 proton | COCHN |
| 4,60-4,20 ppm | m | 1 proton | CH2 |
| 3,50-3,10 ppm | m | 1 proton | CH2 |
| 3,0-2,60 ppm | m | 2 protons | CH2 |
| 2,3-1,6 ppm | m | 2 protons | CH2 |

I.R. : 3300, 1670, 1645, 1600, 1540, 1505, 1475, 1370, 1300, 1200, 1160, 790, 680 cm-1.

EXEMPLE 23 : (5R,S)-N-(céto-4-phényl-7-hexahydro-1,2,3,3a,4,5-pyrido[3,2,1-j,k]benzodiazépin[1,4]-yl-5)-N′-(quinoléinyl-3-)-urée.

Formule (I) ; A = -(CH2)3- ; R9 = H ; R10 = C6H5 ; R11 = H ; R12 = O ; R13 = NH ; Ar = quinoléinyl-3 .

On opère de la même façon que dans l'exemple ci-dessus, à partir de 1,73 g (12 mmoles) d'amino-3-quinoléine, de 3,5 g (12 mmoles) d'amine obtenue au stade 5b Ex.5, et de 1,944 g (12 mmoles) de N,N′-carbonyldiimidazole. Après chromatographie sur silice dans du chlorure de méthylène enrichi en acétone, on isole 4,4 g (80 %) de solide fondant au dessus de 280° C avec décomposition.
C.C.M. : CH2Cl2/Méthanol 90/10 Rf = 0,55 .

| R.M.N. : | | | |
|---|---|---|---|
| 9,65 ppm | s | 1 proton | NH |
| 9,0-7,0 ppm | m | 15 protons | ArH,NH |
| 5,4 ppm | d | 1 proton | COCHN |
| 4,65-4,20 ppm | m | 1 proton | CH2 |
| 3,50-3,10 ppm | m | 1 proton | CH2 |
| 3,10-2,60 ppm | m | 2 protons | CH2 |
| 2,4-1,6 ppm | m | 2 protons | CH2 |

I.R. : 3350, 3050, 1660, 1580,1500, 1480, 1340, 1250, 1200, 880, 780, 740, 695 cm-1.

EXEMPLE 24 : (5S)-N-(céto-4-phényl-7-hexahydro-1,2,3,3a,4,5-pyrido[3,2,1-j,k]benzodiazépin[1,4]-yl-5)-N′-(bromo-4-phényl-)-thiourée.

Formule (I) ; A = -(CH2)3- ; R9 = H ; R10 = C6H5 ; R11 = H ; R12 = S ; R13 = NH ; R14 = O ; Ar = bromo-4-phényle ; isomère lévogyre (Iaq).

A partir de 5,2 g (17,8 mmoles) de l'amine obtenue au stade 6e, et de 3,8 g (17,8 mmoles) de bromo-4-phényle isocyanate, en opérant de la même manière qu'à l'exemple 10, après chromatographie sur silice dans du chlorure de méthylène enrichi en acétone. On obtient 7,7 g (86 %) de cristaux. F = 200 °C. [α]D = -93 °C (EtOH c = 1 %).
C.C.M. : CH2Cl2/Acétone 95/5 Rf = 0,40 .

| R.M.N. : | | | |
|---|---|---|---|
| 8,9 ppm | s | 1 proton | NH |
| 8,2 ppm | d | 1 proton | NH |
| 7,70-7,0 ppm | m | 12 protons | ArH |
| 6,0 ppm | d | 1 proton | COCHN |
| 4,55-4,20 ppm | m | 1 proton | CH2 |
| 3,40-3,0 ppm | m | 1 proton | CH2 |
| 3,0-2,50 ppm | m | 2 protons | CH2 |
| 2,3-1,6 ppm | m | 2 protons | CH2 |

I.R. : 3300, 1660, 1580,1500, 1440, 1380, 1160 cm-1.

EXEMPLE 25 : (5S)-N-(céto-4-phényl-7-hexahydro-1,2,3,3a,4,5-pyrido[3,2,1-j,k]benzodiazépin[1,4]-yl-5)-N′-(méthyl-4-phényl-)-urée.

Formule (I) ; A = -(CH2)3- ; R9 = H ; R10 = C6H5 ; R11 = H ; R12 = O ; R13 = NH ; Ar = méthyl-4-phényle ; isomère lévogyre .

A partir de 2,6 g (9 mmoles) de l'amine obtenue au stade 6e, et de 1,19 g (9 mmoles) de méthyl-4-phényle isocyanate, en opérant de la même manière qu'à l'exemple 13, et après chromatographie sur silice dans du chlorure de méthylène enrichi en acétone, on obtient 2,7 g (71 %) de cristaux . F = 190 °C. [α]D = -155° (CH2Cl2 c = 1 %).
C.C.M. : CH2Cl2/Acétone 90/10 Rf = 0.46 .

| R.M.N. : | | | |
|---|---|---|---|
| 9,0 ppm | s | 1 proton | NH |
| 7,60-6,90 ppm | m | 13 protons | ArH,NH |
| 5,30 ppm | d | 1 proton | COCHN |
| 4,60-4,10 ppm | m | 1 proton | CH2 |
| 3,40-3,05 ppm | m | 1 proton | CH2 |
| 3,05-2,60 ppm | m | 2 protons | CH2 |
| 2,35 ppm | s | 3 protons | CH3 |
| 2,4-1,6 ppm | m | 2 protons | CH2 |

I.R. : 3300, 2900, 1675, 1640, 1600, 1540, 1500, 1440, 1380, 1200, 1160, 790, 740, 700 cm-1.

EXEMPLE 26 : (5R)-N-(céto-4-phényl-7-hexahydro-1,2,3,3a,4,5-pyrido[3,2,1-j,k]benzodiazépin[1,4]-yl-5)-N′-(bromo-4-phényl-)-thiourée.

Formule (I) ; A = -(CH2)3- ; R9 = H ; R10 = C6H5 ; R11 = H ; R12 = S ; R13 = NH ; Ar = bromo-4-phényle ; Isomère dextrogyre .

A partir de 4,75 g (16 mmoles) de l'amine obtenue au stade 7b, et de 3,49 g (16 mmoles) de bromo-4-phényle isocyanate, en opérant de la même manière qu'à l'exemple 10, après chromatographie sur silice dans du chlorure de méthylène enrichi en acétone. On obtient 7,0 g (86,5 %) de cristaux. F = 200° C. [α]D = + 95° (EtOH c = 1%).
C.C.M. : CH2Cl2/Acétone 95/5 Rf = 0,40 .

| R.M.N. : | | | |
|---|---|---|---|
| 8,85 ppm | s | 1 proton | NH |
| 8,15 ppm | d | 1 proton | NH |
| 7,65-6,90 ppm | m | 12 protons | ArH |
| 6,0 ppm | d | 1 proton | COCHN |
| 4,50-4,25 ppm | m | 1 proton | CH2 |
| 3,35-3,0 ppm | m | 1 proton | CH2 |
| 3,0-2,60 ppm | m | 2 protons | CH2 |
| 2,4-1,6 ppm | m | 2 protons | CH2 |

I.R. : 3300, 1660, 1580,1500, 1440, 1380, 1160 cm-1.

EXEMPLE 27 : (5R)-N-(céto-4-phényl-7-hexahydro-1,2,3,3a,4,5-pyrido[3,2,1-j,k]benzodiazépin[1,4]-yl-5)-N'-(méthyl-4-phényl-)-urée.

Formule (I) ; A = -(CH2)3- ; R9 = H ; R10 = C6H5 ; R11 = H ; R12 = O ; R13 = NH ; Ar = méthyl-4-phényle ; isomère dextrogyre .

A partir de 2,9 g (10 mmoles) de l'amine obtenue au stade 7b, et de 1,33 g (10 mmoles) de méthyl-4-phényle isocyanate, en opérant de la même manière qu'à l'exemple 13, et après chromatographie sur silice dans du chlorure de méthylène enrichi en acétone, on obtient 2,1 g (50 %) de cristaux . F = 190° C. [α]D = + 157° C (CH2Cl2 c = 1 %).
C.C.M. : CH2Cl2/Acétone 90/10 Rf = 0,46 .

| R.M.N. : | | | |
|---|---|---|---|
| 7,80-6,85 ppm | m | 14 protons | ArH,2NH |
| 5,60 ppm | d | 1 proton | COCHN |
| 4,70-4,25 ppm | m | 1 proton | CH2 |
| 3,40-3,0 ppm | m | 1 proton | CH2 |
| 3,0-2,50 ppm | m | 2 protons | CH2 |
| 2,25 ppm | s | 3 protons | CH3 |
| 2,35-1,60 ppm | m | 2 protons | CH2 |

I.R. ; 3300, 2900, 1660, 1600, 1540,1500, 1440, 1370, 1200, 1160, 810, 740, 700 cm-1.

EXEMPLE 28 : (5R,S)-N-(céto-4-(méthyl-4-phényl-)-7-hexahydro-1,2,3,3a,4,5-pyrido[3,2,1-j,k]-benzodiazépin-[1,4]-yl-5)-N'-(bromo-3-phényl-)-thiourée.

Formule (I) ; A = -(CH2)3- ; R9 = H ; R10 = méthyl-4-phényl ; R11 = H ; R12 = S ; R13 = NH ; Ar = bromo-3-phényle .

Stade 28a. Transformation d'un composé de formule (III) en un composé de formule (IV).

Ce composé est préparé à partir de 25,8 g (194 mmoles) de tétrahydro-1,2,3,4-quinoléine et de 45,5 g (388 mmoles) de méthyl-4-benzonitrile, en opérant de la même manière qu'au stade 3a. On obtient 25,4 g (52 %) de (méthyl-4-benzoyl-8-tétrahydro 1,2,3,4-quinoléine.
F = 94° C.
C.C.M. : CH2Cl2 Rf = 0,70.
Cyclohexane/AcOEt 90/10 Rf = 0,30.

| R.M.N. : | | | |
|---|---|---|---|
| 8,7 ppm | s | 1 proton | NH |
| 7,7-6,95 ppm | m | 7 protons | ArH |
| 3,65-3,30 ppm | m | 2 protons | CH2N |
| 2,70-3,0 ppm | m | 2 protons | CH2 |
| 2,45 ppm | s | 3 protons | CH3 |
| 2,20-1,60 ppm | m | 2 protons | CH2 |

I.R. : 3300, 2900, 1660, 1580, 1470, 1380, 1240, 1160, 820, 750 cm-1.

Stade 28b. N-bromoacétyl-(méthyl-4-benzoyl-)-8-tétrahydro-1,2,3,4-quinoléine.

A partir de 12,5 g (50 mmoles) de produit du stade 28a ci-dessus, en opérant de la même manière qu'au stade 3b Ex.3, on obtient 8,5 g (46 %) de cristaux.

Le produit très instable est utilisé immédiatement dans la réaction suivante.

Stade 28c. Céto-4-hexahydro-1,2,3,3a,4,5-(méthyl-4-phényl-)-7-pyrido[3,2,1-j,k]benzodiazepine[1,4].

A partir de 10 g (26,8 mmoles) de produit du stade 28b ci-dessus, en opérant de la même manière qu'au stade 3c Ex.3, on obtient 4 g (50 %) de cristaux.
C.C.M. : CH2Cl2/Acétone 95/5 Rf = 0,1.
CH2Cl2/MeOH 90/10 Rf = 0,7.

| R.M.N. : | | | |
|---|---|---|---|
| 7,5-6,8 ppm | m | 7 protons | ArH |
| 4,78-4,65-3,78-3,65 | AB | 2 protons | CO-CH2-N |
| 4,60-4,25 ppm | m | 1 proton | CH2N |
| 3,25-2,90 ppm | m | 1 proton | CH2N |
| 2,90-2,15 ppm | m | 2 protons | CH2 |
| 2,30 ppm | s | 3 protons | CH3Ar |
| 2,25-1,60 ppm | m | 2 protons | CH2 |

Stade 28d. Hydroximino-5-hexahydro-1,2,3,3a,4,5-(méthyl-4-phenyl-)-7-pyrido[3,2,1-j,k]benzodiazepine[1,4].

Formule(IV) ; A = -(CH2)3- ; R9 = H ; R10 = méthyl-4-phényle.

De la même façon que dans l'exemple 1, stade 1a, à partir de 138 g (476 mmoles) de produit du stade ci-dessus, aprés chromatographie dans du chlorure de méthylène enrichi en acétone, on isole 118 g (80 %) de composé solide. F = 170° C.
C.C.M. : CH2Cl2/MeOH 90/10 Rf = 0,70.

| R.M.N. : | | | |
|---|---|---|---|
| 7,80-6,10 ppm | m | 7 protons | ArH |
| 4,5 ppm | m | 1 proton | OH |
| 3,0-2,70 ppm | m | 2 protons | CH2 |
| 2,70-2,20 ppm | m | 2 protons | CH2 |
| 2,38 ppm | s | 3 protons | CH3Ar |
| 2,20-1,70 ppm | m | 2 protons | CH2 |

Stade 28e. Amino-5-céto-4-hexahydro-1,2,3,3a,4,5-(méthyl-4-phényl-)-7-pyrido[3,2,1-j,k]benzodiazépine[1,4].

Formule(II) ; A = -(CH2)3- ; R9 = H ; R10 = méthyl-4-phényle.

De la même façon que dans l'exemple 1, stade 1b, 2ème méthode à partir de 35 g (109 mmoles) du composé obtenu au stade ci-dessus, et après chromatographie sur silice dans du chlorure de méthylène enrichi en méthanol, on isole une mousse 24,0 g (80 %) n'ayant pas de point de fusion net et qui est employée directement dans les réactions suivantes. F environ 110 °C.
C.C.M. : CH2Cl2/MeOH 90/10 Rf = 0,60.

| R.M.N. : | | | |
|---|---|---|---|
| 7,80-6,90 ppm | m | 7 protons | ArH |
| 4,70-4,25 ppm | m | 2 protons | CHN,CH2N |
| 3,45-3,0 ppm | m | 1 proton | CH2N |
| 3,0-2,45 ppm | m | 2 protons | CH2 |
| 2,35 ppm | s | 3 protons | CH3Ar |
| 2,95-1,50 ppm | m | 2 protons | CH2 |

Stade 28f : (produit de l'exemple)

A partir de 3,07 g (10 mmoles) de l'amine obtenue au stade précédent, et de 2,14 g (10 mmoles) de bromo-3-phényle isothiocyanate, en opérant de la même manière qu'à l'exemple 22. On obtient 4 g (77 %) de cristaux.
F = 170 °C.
C.C.M. : CH2Cl2/Acétone 95/5 Rf = 0,55 .

| R.M.N. : | | | |
|---|---|---|---|
| 8,50 ppm | s | 1 proton | NH |
| 8,15 ppm | d | 1 proton | NH |
| 7,60-6,90 ppm | m | 11 protons | ArH |
| 6,0 ppm | d | 1 proton | COCHN |
| 4,65-4,20 ppm | m | 1 proton | CH2 |
| 3,35-3,0 ppm | m | 1 proton | CH2 |
| 3,0-2,60 ppm | m | 2 protons | CH2 |
| 2,35 ppm | s | 3 protons | CH3Ar |
| 2,30-1,50 ppm | m | 2 protons | CH2 |

I.R. : 3300, 2900, 1660, 1580,1500, 1470, 1140, 820, 770, 750 cm-1.

EXEMPLE 29 : (5R,S)-N-(céto-4-(méthyl-4-phényl-)-7-hexahydro-1,2,3,3a,4,5-pyrido[3,2,1-j,k]-benzodiazépin-[1,4]-yl-5)-N'-(bromo-4-phényl-)-thiourée.

Formule (I); A = -(CH2)3-; R9 = H ; R10 = méthyl-4-phényl; R11 = H ; R12 = S ; R13 = NH ; Ar = bromo-4- phényle.

Ce produit est obtenu à partir de 6,14 g (20 mmoles) de l'amine obtenue au stade e de l'exemple précédent et de 4,28 g (20 mmoles) de bromo-4-phényle isothiocyanate, en opérant de la même manière qu'à l'exemple 10. On obtient 4,2 g (45 %) de cristaux.
F = 185° C.
C.C.M. : CH2Cl2/Acétone 95/5 Rf = 0,55 .

| R.M.N. : | | | |
|---|---|---|---|
| 8,65 ppm | s | 1 proton | NH |
| 8,15 ppm | d | 1 proton | NH |
| 7,60-7,0 ppm | m | 11 protons | ArH |
| 6,0 ppm | d | 1 proton | COCHN |
| 4,65-4,20 ppm | m | 1 proton | CH2 |
| 3,35-3,0 ppm | m | 1 proton | CH2 |
| 3,0-2,70 ppm | m | 2 protons | CH2 |
| 2,35 ppm | s | 3 protons | CH3Ar |
| 2,30-1,60 ppm | m | 2 protons | CH2 |

I.R. : 3300, 2900, 1660, 1600, 1580,1510, 1480, 1380, 1160, 1060, 1000, 820, 745 cm-1.

EXEMPLE 30 : (5R,S)-N-(céto-4-(méthyl-4-phényl-)-7-hexahydro-1,2,3,3a,4,5-pyrido[3,2,1-j,k]benzodiazépin-[1,4]-yl-5)-N'-(éthoxycarbonyl-4-phényl-)-urée.

Formule (I) ; A = -(CH2)3- ; R9 = H ; R10 = méthyl-4-phényl ; R11 = H ; R12 = O ; R13 = NH ; Ar = éthoxy carbonyl-4-phényle .

A partir de 3,05 g (10 mmoles) de l'amine (II) précédente et de 1,91 g (10 mmoles) d'éthoxycarbonyl-4-phényle isocyanate, en opérant de la même manière qu'à l'exemple 10. On obtient 1,5 g (30 %) de cristaux .
F > 290° C.
C.C.M. : CH2Cl2/Acétone 95/5 Rf = 0,30.

| R.M.N. : | | | |
|---|---|---|---|
| 9,50 ppm | s | 1 proton | NH |
| 8,0-7,10 ppm | m | 12 protons | ArH,NH |
| 5,3 ppm | d | 1 proton | COCHN |
| 4,60-4,10 ppm | m | 3 protons | OCH2,CH2N |
| 3,50-3,10 ppm | m | 1 proton | CH2N |
| 3,10-2,60 ppm | m | 2 protons | CH2 |
| 2,35 ppm | s | 3 protons | CH3Ar |
| 2,40-1,60 ppm | m | 2 protons | CH2 |
| 1,45-1,20 ppm | t | 3 protons | CH3C |

I.R. : 3400, 2900, 1710, 1660, 1600, 1530, 1500, 1280, 1165, 1150, 1020, 830, 770 cm-1.

EXEMPLE 31 : (5R,S)-N-(céto-4-(méthyl-4-phényl-)-7-hexahydro-1,2,3,3a,4,5-pyrido[3,2,1-j,k] benzodiazépin-[1,4]-yl-5)-N'-(aminosulfonyl-4-phényl-)-urée.

Formule (I) ; A = -(CH2)3- ; R9 = H ; R10 = méthyl-4-phényl ; R11 = H ; R12 = O ; R13 = NH ; Ar = aminosulfonyl-4-phényle .

1,72 g de sulfanilamide (10 mmoles) sont placés en solution dans 25 ml de diméthylformamide et 50 ml de chlorure de méthylène. On ajoute 1,62 g (10 mmoles) de N,N'-carbonyldiimidazole et laisse 4 heures à température ambiante. On refroidit à -5° C et ajoute 3,05 g (10 mmoles) de l'amine (II) des exemples précédents dissous dans 50 ml de chlorure de méthylène. On laisse 14 heures sous agitation à température ambiante. On évapore à sec. Le résidu huileux est repris par du chlorure de méthylène et lavé par de l'eau. Il se produit un abondant précipité que l'on lave à l'eau, puis que l'on sèche. On le dissout dans du méthanol et l'absorbe sur 50 g de silice. Cette silice est placée en tête d'une colonne de 250 g de silice qui est éluée par du chlorure de méthylène enrichi par de l'acétate d'éthyle. On obtient 1,5 g (30 %) de cristaux .
F = 270° C.
C.C.M. : CH2Cl2/AcOEt 50/50 Rf = 0,60.

| R.M.N. : | | | |
|---|---|---|---|
| 9,50 ppm | s | 1 proton | NH |
| 7,85-7,0 ppm | m | 12 protons | ArH,NH |
| 5,3 ppm | d | 1 proton | COCHN |
| 4,60-4,15 ppm | m | 1 proton | CH2N |
| 3,35 ppm | s | 2 protons | SO2NH2 |
| 3,50-3,05 ppm | m | 1 proton | CH2N |
| 3,05-2,60 ppm | m | 2 protons | CH2 |
| 2,35 ppm | s | 3 protons | CH3Ar |
| 2,60-1,50 ppm | m | 2 protons | CH2 |
| 1,45-1,20 ppm | t | 3 protons | CH3C |

I.R. : 3300, 1660, 1570, 1540, 1480, 1340, 1150, 1100, 900, 820, 650, 540 cm-1.

L'étude des composés de l'invention a été réalisée quant à leur toxicité et à la recherche de leur activité par des méthodes d'études "in vitro" et "in vivo".

Ainsi, la toxicité aigüe a été étudiée par voie orale sur la souris mâle. Pour cela, les produits ont été administrés en solution aqueuse à raison de 2 ml/100 g. Les animaux ont été ensuite observés durant les trois heures qui suivent l'administration puis quotidiennement durant quatorze jours, moment auquel ils ont été sacrifiés et autopsiés.

Les DL50 (doses léthales provoquant la mort de 50 % des animaux) ont été calculés selon la méthode de Reed J.L. et Muench H. (Am. J. Hyg. 1939, 27, 493). Leur valeur est supérieure à 1000 mg/Kg-1 ce qui correspond à une faible toxicité des composés.

**A. Etudes "in vitro"**

Ces études ont consisté à déterminer l'affinité de liaison des composés de l'invention aux récepteurs de la cholecystokinine et aux récepteurs de la gastrine.

Il a été déterminé dans ces essais les concentrations des solutions de produits de formule (I) capables d'inhiber pour les récepteurs considérés cinquante pour cent des liaisons de la cholecystokinine marquée à l'iode 125 (ci-dessous abrégée en [125I]-CCK8 sulfatée) ou dans le cas du récepteur de la gastrine marquée à l'iode 125 aux récepteurs :
- de membranes plasmiques de pancréas de rat (mâles SD, IFFA CREDO 200-225 g). (Récepteur périphérique de la cholecystokinine : CCK-A).
- de membranes de cerveau de cobayes (mâles, COB LABO, 325-350 g). (Récepteur central de la cholecystokinine : CCK-B) selon le protocole décrit par INNIS R.B. et SNYDER S.M., Eur. J. Pharmacol., 65, 123-124, 1980.

- de glandes gastriques de cobaye (males, COB LABO, 325-350 g) selon la méthode décrite par PRAISSMAN M., WALDEN M.E. et PELLECHIA C., Journal of Receptor Research **3**, 647-665,1983. (Récepteur de la gastrine).

Les résultats exprimés en CI50 ou concentration inhibitrice 50 % sont reportés au tableau 1 qui suit. L'unité de mesure est la nanomole.

TABLEAU I

| | Liaison aux récepteurs | | |
|---|---|---|---|
| | CCK A | CCK B | GASTRINE |
| Example | nanomoles | nanomoles | nanomoles |
| 1 | 59.6 | 431 | 14.4 |
| 2 | 1025 | 99.9 | 85.9 |
| 5 | 84,2 | 251 | 1,5 |
| 7 | 1726 | 255 | 40.5 |
| 8 | 3078 | 1808 | 60 |
| 10 | 3041 | 88 | 3.2 |
| 23 | 389 | 176 | 298 |
| 26 | >10000 | 137 | 36 |
| XXa | 1419 | 81.4 | 4.7 |

L'examen de ces résultats montre que les produits de l'invention présentent des affinités sélectives pour les récepteurs CCK B et / ou gastrine particulièrement intéressantes. Ainsi leur sélectivité peut être :

- soit particulière pour les récepteurs centraux (CCK B) tel que pour le composé de l'exemple 2 qui, comparé au composé de référence XXa montre une activité du même ordre sur les récepteurs CCK A et CCK B mais se montre près de 20 fois moins actif sur les récepteurs de la gastrine. Il en est de même pour le composé de l'exemple 23, qui, bien qu'environ 2 fois moins actif que le composé XXa sur le récepteur CCKB, est près de 60 fois moins actif que cette même référence sur le récepteur de la gastrine, et en conséquence possède une sélectivité pour le récepteur CCK B près de 30 fois supérieure.
- soit particulière pour les récepteurs de la gastrine tels que les composés des exemples 5 et 10 qui ont une activité semblable au composé XXa de référence mais sont respectivement 3 et 1,5 fois plus actifs sur le récepteur de la gastrine,

Par ailleurs des composés de l'invention présentent une activité sur les récepteurs CCK B aussi bien que sur ceux de la gastrine d'une sélectivité semblable ou améliorée comparativement à celle du composé XXa. A titre illustratif il est présenté les résultats du composé de l'exemple 26 :

**Sélectivité :**
GASTRINE/CCK A XXa : 1419/4,7 = 302
26 : > 10000/36 = > 250
CCK B/CCK A XXa : 1419/81,4 = 17
26 : > 10000/137 = > 70

**B. Etudes in vivo**

**1 : Sécrétion acide induite par la gastrine :** L'activité des produits de l'invention sur la sécrétion acide induite par la gastrine chez le rat a été étudiée selon la méthode décrite par Pascaud X.P. ; Roger A.R. et Genton M.J.H. ; Digestion 16, 57-68, 1977. Elle consiste à recueillir la sécrétion gastrique acide sur deux périodes consécutives de deux heures chez des rats SD mâles (IFFA CREDO 250-300 g) munis d'une fistule gastrique chronique et perfusés en continu par voie intraveineuse avec une solution de Pentagastrine (Peptavlon, ICI, 6 µg/kg/h). L'inhibition de cette sécrétion, provoquée par l'administration per os de 1 mg/kg de produit de l'invention est déterminée. Selon cette technique le composé de l'invention de l'exemple 10 qui est un racémique présente un activité inhibitrice identique à celle de l'énantiomère (XXa) de l'art antérieur. Les résultats sont reportés au tableau II qui suit.

**2: Evacuation gastrique :** L'activité antagoniste de la CCK sur les effets moteurs digestifs de l'hormone est recherchée sur l'évacuation gastrique ralentie par administration sous-cutanée de CCK-8 (80 µg/kg)

chez des souris SWISS mâles (IFFA CREDO 16-20 g) selon la technique de Lotti V.J., Cerino D.J., Kling P.J., et Chang R.S.L. ; Life Sciences 39, 1631-1638, 1986. Dans ce test, l'effet stimulant des produits relève de leur activité sur le récepteur périphérique CCK-A. Les résultats obtenus avec le composé de l'exemple 10 et le produit (XXa) de l'art antérieur sont présentés ci-dessous (tableau II) et exprimés par leur DE50 qui est la dose en mg/kg de composé à l'essai, permettant par voie orale, d'inhiber 50% du ralentissement de l'évacuation gastrique provoquée par la CCK-8 chez la souris.

TABLEAU II

| COMPOSE | SECRETION GASTRIQUE % d'Inhibition | EVACUATION GASTRIQUE DE 50 |
|---|---|---|
| (XXa) | 29 % | 0.15 |
| Exemple 10 | 31 % | 15.5 |

Les résultats du tableau II montrent qu'à dose équivalente (1mg/kg) ces deux composés administrés par voie orale sont capables d'inhiber de manière significative et avec une même intensité d'action les effets stimulants d'une dose sub-maximale de gastrine sur la sécrétion gastrique. Il a fallu par contre 100 fois plus de produit de l'exemple 10 que du produit de l'art antérieur (XXa) pour diminuer de 50 % les effets inhibiteurs de la CCK-8 sur l'évacuation gastrique, ce qui est démonstratif de la très faible activité du composé de l'invention sur les récepteurs périphériques CCK-A impliqués dans ce phénomène. Les composés de l'invention permettent donc d'inhiber la sécrétion gastrique sans provoquer à titre d'effet secondaire d'augmentation de l'évacuation gastrique. D'une manière plus générale il permettent d'agir sur les phénomènes régulés par les récepteurs B de la cholecystokinine ou par les récepteurs de la gastrine sans agir à titre d'effet secondaire sur les phénomènes régulés par les récepteurs A de la cholecystokinine.

Les produits de l'invention sont administrés sous forme de compositions appropriées à la nature et à l'importance de l'affection à traiter. La posologie journalière chez l'homme est habituellement comprise entre 2 milligrammes et 1 gramme de produit qui peut être absorbé en une ou plusieurs prises. Les compositions sont préparées sous des formes compatibles avec la voie d'administration envisagée, comme par exemple les comprimés, dragées, capsules, suppositoires, gels ou suspensions. Ces compositions sont préparées par des méthodes courantes pour l'homme de l'art et comprennent de 1 à 60 % en poids de principe actif (composé de formule I) et 40 à 99 % en poids de véhicule pharmaceutique approprié et compatible avec le principe actif et la forme physique de la composition envisagée. A titre d'exemple, il est présenté ci-dessous la méthode de préparation de comprimés contenant un composé de l'invention.

| PREPARATION DE COMPRIMES | |
|---|---|
| Substance active de formule (I) | 1 à 75 mg |
| Lactose | 124 à 74 mg |
| Cellulose microcristalline | 36 à 60 mg |
| Polyvinylpyrrolidone | 6 mg |
| Carboxyméthylamidon sodique | 8 mg |
| Stéarate de magnésium | 1 mg |

Mélanger la substance active,le lactose,la cellulose microcristalline et le carboxyméthylamidon.
Mouiller et granuler à l'aide d'une solution aqueuse ou alcoolique de polyvinylpyrrolidone de concentration adaptée.
Sécher et calibrer le granulé.
Mélanger de façon homogène le stéarate de magnésium.
Comprimer à raison de 200 mg par comprimé.

**Revendications**

1. Benzodiazépines de formule (I) :

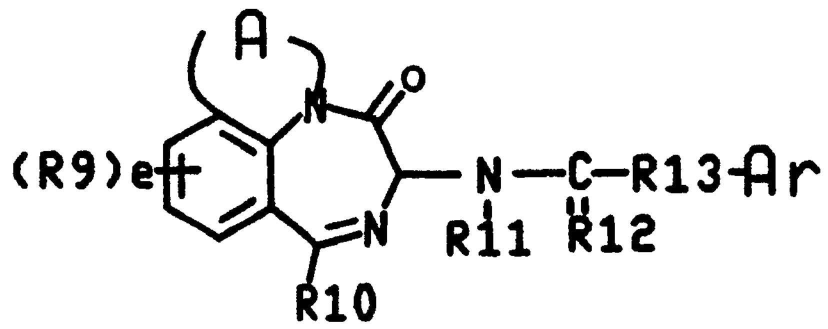

(I)

Dans laquelle,
R9 est hydrogène, nitro, halogène, cyano, trifluorométhyle, hydroxy, alkyle inférieur, alkoxy inférieur, -COOR20, -N(R21)-R22 ;
R10 est un phényle éventuellement mono à tri substitué par des groupes identiques ou différents choisis parmi halogène, alkyle inférieur, alkoxy inférieur, cyano, nitro ou trifluorométhyle ;
R11 est hydrogène ou alkyle inférieur ;
R12 est un atome d'oxygène ou un atome de soufre ;
R13 est un groupe -N(R24)- ;
R20 est hydrogène ou alkyle inférieur ;
R21 et R22 sont identiques ou différents et peuvent être l'hydrogène ou un groupe alkyle ;
R24 est hydrogène ou alkyle inférieur ;
Ar est un groupe aromatique hydrocarboné ou hétéroaromatique comportant un cycle ou deux cycles condensés, chacun des cycles comportant de 5 à 7 atomes parmi lesquels figurent de zéro à deux hétéroatomes d'azote; ce groupe aromatique ou hétéro aromatique étant éventuellement mono ou di substitué par des groupes identiques ou différents choisis parmi alkyle inférieur, carboxyle, alkoxycarbonyle inférieur, sulfonyle, sulfamoyle, cyano, nitro, trifluorométhyle ou, sous réserve que R12 soit le soufre, par des groupes halogène ou alkoxy inférieur ;
A est égal à [-CH(R1)-]a dans lequel R1 est hydrogène ou alkyle inférieur ;
a est égal à deux, trois ou quatre ;
e est égal à un ou deux ;

2. Benzodiazépines suivant la revendication 1 caractérisées en ce que R12 représente un atome de soufre.

3. Benzodiazépines suivant la revendication 1 ou 2 caractérisées en ce que le groupe Ar ne comporte qu'un cycle.

4. Benzodiazépines suivant l'une des revendications 1 ou 3 caractérisées en ce que le groupe Ar est un groupe phényle substitué par un groupe méthyle.

5. Benzodiazépines suivant l'une des revendications 2 ou 3 caractérisées en ce que le groupe Ar est un groupe phényle substitué par un groupe méthyle ou un atome de brome.

6. Benzodiazépines suivant l'une des revendications 1 à 5 caractérisées en ce que le groupe A est un pont éthylène (-CH2-CH2-) ou triméthylène (-CH2-CH2-CH2-).

7. Benzodiazépines suivant l'une des revendications 1 à 6 caractérisées en ce que le groupe R9 est un atome d'hydrogène ou un atome de chlore en position para par rapport à l'atome d'azote commun au cycle diazépine et à l'autre hétérocycle azoté condensé avec le cycle diazépine et en ce que e = 1.

8. Benzodiazépines suivant l'une des revendications 1 à 7 caractérisées en ce que le groupe R10 est un groupe phènyle, un groupe phènyle mono-substitué par un atome de fluor en position ortho ou par un radical méthyle en position para par rapport à l'atome de carbone reliant le cycle phényle au cycle diazépine.

9. Benzodiazépines suivant l'une des revendications 1 à 8 caractérisées en ce que le groupe R11 est un atome d'hydrogène.

10. Benzodiazépines suivant l'une des revendications 1 à 9 caractérisées en ce que le groupe R13 est un groupe -NH-.

11. Benzodiazépines suivant l'une des revendications 1 à 10 caractérisées en ce que l'atome de carbone asymétrique du cycle benzodiazèpine possède la configuration absolue R selon la nomenclature de Cahn, Ingold et Prelog.

12. Benzodiazépines suivant l'une des revendications 1 à 10 caractérisées en ce que l'atome de carbone asymétrique du cycle benzodiazèpine possède la configuration absolue S selon la nomenclature de Cahn, Ingold et Prelog.

13. Benzodiazépines suivant la revendication 1 et qui sont :

La (4R,S)-N-(céto-3-phényl-6-tétrahydro-1,2,3,4-pyrrolo[3,2,1-j,k]benzodiazépin-[1,4]-yl-4)-N'-(méthyl-3-phényl-)-urée et son isomère (5R).

La (5R,S)-N-(céto-4-phényl-7-hexahydro-1,2,3,3a,4,5-pyrido[3,2,1-j,k]benzodiazépin-[1,4]-yl-5)-N'-(méthyl-3-phényl-)-urée et son isomère (5R).

La (5R,S)-N-(céto-4-phényl-7-hexahydro-1,2,3,3a,4,5-pyrido[3,2,1-j,k]benzodiazépin-[1,4]-yl-5)-N'-(nitro-4-phényl-)-urée.

La (5R,S)-N-(céto-4-phényl-7-hexahydro-1,2,3,3a,4,5-pyrido[3,2,1-j,k]benzodiazépin-[1,4]-yl-5)-N'-(bromo-4-phényl-)-thiourée et ses isomères optiques et son isomère (5R).

14. Procédé de préparation des benzodiazépines de formule (I), définie à la revendication 1 qui consiste

a) pour préparer les composés racémiques,

i) à faire réagir une amine racémique de formule (II) :

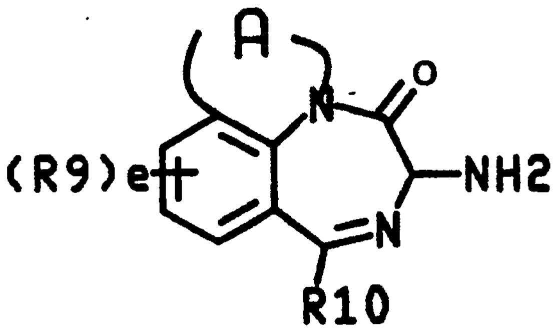

(II)

dans laquelle A, R9, R10 et e ont la signification définie à la revendication 1 avec un arylisocyanate de formule Ar-N=C=O, Ar ayant la signification définie à la revendication 1 pour obtenir une benzodiazépine racémique (Ia), de formule (I) dans laquelle R11 est l'hydrogène, R13 est NH et R12 est l'oxygène,

ii) à faire réagir une amine racémique (II) dans laquelle A, R9, R10 et e ont la signification définie à la revendication 1 avec un agent de protection des amines de formule (XII) : R25-CH2X, dans laquelle R25 est un benzène éventuellement mono di ou tri substitué par des substituants identiques ou différents choisis parmi les halogènes et le groupe nitro et X est un halogène ou un alkyl ou arylsulfonate, ou encore avec un agent (XIIa) R25-CHO en présence dans ce dernier cas de borohydrure de sodium, de cyanoborohydrure de sodium ou d'hydrogène sur catalyseur pour obtenir la benzylamine de formule (XIX)

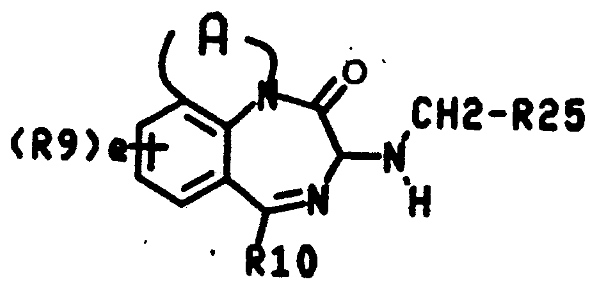

(XIX)

qui est mise à réagir avec un arylisocyanate de formule Ar-N=C=O, Ar ayant la signification définie à la revendication 1 pour obtenir l'urée de formule (XV) :

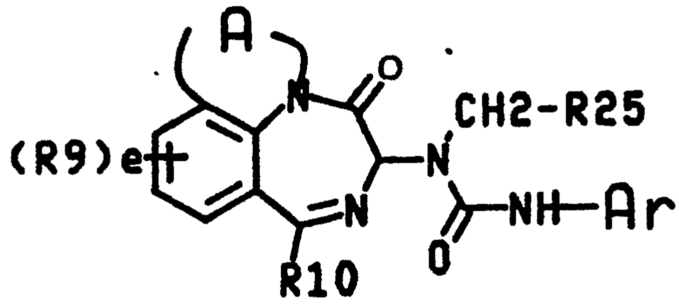

(XV)

qui est mise à réagir avec un réactif d'alkylation de formule (XIV) : R24-R39 dans lequel R24 a la signfication définie à la revendication 1 et R39 est un halogène ou un pseudo halogène pour obtenir une urée tétrasubstituée de formule (XVI),

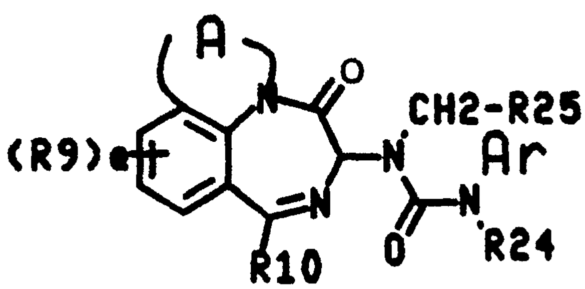

(XVI)

puis à éliminer par hydrogènolyse le groupe protecteur R25-CH2 avec un réactif choisi parmi le sodium dans l'ammoniac liquide ou l'hydrogène en présence d'un catalyseur d'hydrogènation comme par exemple un metal finement divisé sur un support comme du carbone pour obtenir une benzodiazépine racémique (Ib), de formule (I) dans laquelle R11 est l'hydrogène, R13 est N(R24), R24 étant alkyle inférieur et R12 est l'oxygène,

iii) à faire réagir une amine racémique (II) dans laquelle A, R9, R10, e ont la signification définie ci-dessus un agent d'alkylation de formule (XIII) : R11-R36 dans laquelle R11 est alkyle inférieur et R36 est un halogène ou un alkyl ou arylsulfonate, ou avec un aldéhyde de formule (XIIIa) R37-CHO dans laquelle R37 est l'hydrogène ou alkyle inférieur tel que (R37-CH2-) = (R11-) en présence d'un agent de réduction tel qu'un hydrure de bore ou l'hydrogène en présence d'un catalyseur pour obtenir l'amine de formule (IIa) :

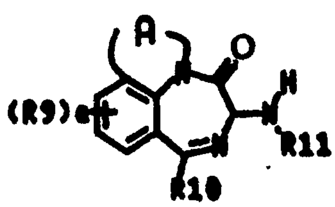

(IIa)

**R11 different de l'hydrogene**

que l'on fait réagir avec un arylisocyanate de formule Ar-N = C = O, pour obtenir une benzodiazépine racémique (Ic), de formule (I) dans laquelle R11 est alkyle inferieur, R13 est NH et R12 est l'oxygène.

iv) à faire réagir une benzodiazépine racémique de formule (Ic) dans laquelle A, R9, R10, R11, Ar et e ont la signification définie à la revendication 1 avec un réactif d'alkylation (XIV) défini en ii) de la présente

revendication pour obtenir une benzodiazépine racémique (Id), de formule (I) dans laquelle R11 est alkyle inférieur, R13 est N-R24, R24 étant alkyle inférieur, et R12 est l'oxygène,

v) à faire réagir une amine racémique (II) dans laquelle A, R9, R10 et e ont la signification à la revendication 1 avec un arylisothiocyanate de formule Ar-N = C = S, Ar ayant la signification définie à la revendication 1 pour obtenir une benzodiazépine racémique (Ie), de formule (I) dans laquelle R11 est l'hydrogène, R13 est NH et R12 est le soufre,

vi) à faire réagir une benzylamine racémique de formule (XIX) précédemment définie avec un arylisothiocyanate de formule Ar-N = C = S, pour obtenir la thiourée de formule (XVII) :

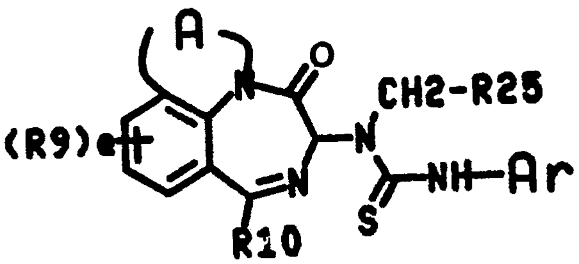
(XVII)

qui est mise à réagir avec un réactif d'alkylation (XIV) pour obtenir une thiourée tétrasubstituée de formule (XVIII) :

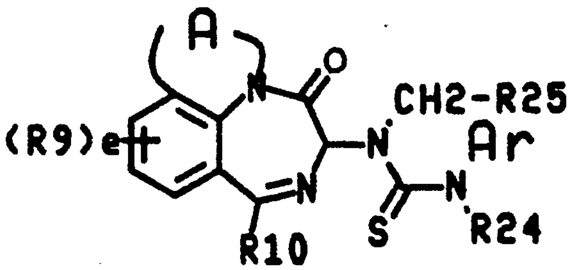
(XVIII)

puis à éliminer par hydrogènolyse le groupe protecteur R25-CH2 par réaction avec le sodium dans l'ammoniac liquide pour obtenir une benzodiazépine racémique (If), de formule (I) dans laquelle R11 est l'hydrogène, R13 est N(R24), R24 étant alkyle inférieur et R12 est le soufre.

vii) à faire réagir une amine racémique (IIa) dans laquelle A, R9, R10, R11 et e ont la signification définie à la revendication 1 avec un arylisothiocyanate de formule Ar-N = C = S, Ar ayant la signification définie à la revendication 1 pour obtenir une benzodiazépine racémique (Ig), de formule (I) dans laquelle R11 est alkyle inférieur, R13 est NH et R12 est le soufre,

viii) à faire reagir une benzodiazépine racémique (Ig) telle que décrite co-dessus avec un réactif d'alkylation (XIV), pour obtenir une benzodiazépine racémique (Ih), de formule (I) dans laquelle R11 est alkyle inférieur, R13 est N-R24, R24 étant alkyle inférieur et R12 est le soufre,

b) pour préparer les formes énantiomères,

à opérer à partir des énantiomères (II) appropriés et de manière identique à celle décrite dans chacun des huit cas précédents pour obtenir les formes énantiomères des benzodiazépines de formules (Ia), (Ib), (Ic), (Id), (Ie), (If), (Ig) et (Ih).

15. Médicament pour lutter principalement contre les troubles provoqués par un déréglement des mécanismes physiologiques controlés par la cholecystokinine ou la gastrine, chez les mammifères ou chez l'homme, caractérisé en ce qu'il comprend une benzodiazépine selon l'une des révendications 1 à 13.

16. Médicament pour lutter contre les troubles provoqués par un déréglement des mécanismes physiologiques contrôlés par les récepteurs B de la cholecystokinine, en particulier, les troubles de l'appétit, la douleur, et éventuellement contre les troubles du système nerveux central, sans provoquer d'effets secondaires dépendants des récepteurs A de la cholecystokinine, caractérisé en ce qu'il comprend une benzodiazépine selon l'une des révendications 1 à 13.

17. Médicament pour lutter principalement contre les troubles provoqués par un déréglement des mécanismes physiologiques contrôlés par la gastrine, en particulier le syndrome de Zollinger-Ellison, les troubles de la sécrétion gastrique, et plus particuliérement les ulcères gastro-duodénaux et les gastro-duodénites, sans provoquer d'effets secondaires dépendants des récepteurs A de la cholecystokinine, caractérisé en ce qu'il comprend une benzodiazépine selon l'une des révendications 1 à 13.

Revendications pour l'Etat contractant suivant: ES

1. Procédé de préparation des benzodiazépines de formule (I),

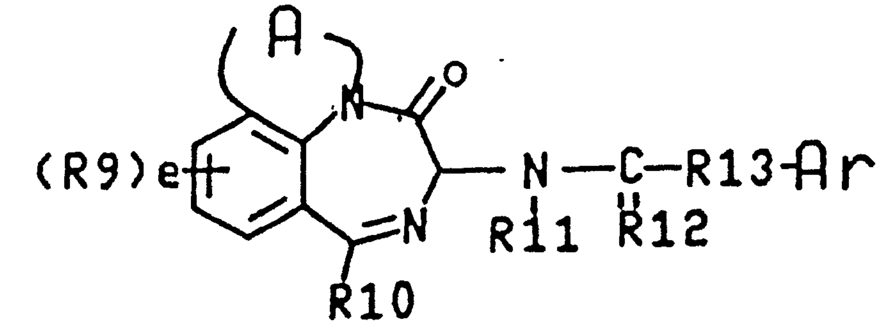

(I)

dans laquelle R9 est hydrogène, nitro, halogène, cyano, trifluorométhyle, hydroxy, alkyle inférieur, alkoxy inférieur, -COOR20, -N (R21) -R22;
R10 est un phényle éventuellement mono à tri substitué par des groupes identiques ou différents choisis parmi halogène, alkyle inférieur, alkoxy inférieur, cyano, nitro ou trifluorométhyle;
R11 est hydrogène ou alkyle inférieur;
R12 est un atome d'oxygène ou un atome de soufre;
R13 est un groupe -N (R24) -;
R20 est hydrogène ou alkyle inférieur;
R21 et R22 sont identiques ou différents et peuvent être l'hydrogène ou un groupe alkyle;
R24 est hydrogène ou alkyle inférieur;
Ar est un groupe aromatique hydrocarboné ou hétéroaromatique comportant un cycle ou deux hétéroatomes d'azote; ce groupe aromatique ou hétéro aromatique étant éventuellement mono ou di substitué par des groupes identiques ou différents choisis parmi alkyle inférieur, carboxyle, alkoxycarbonyle inférieur, sulfonyle, sulfamoyle, cyano, nitro, trifluorométhyle ou, sous réserve que R12 soit le soufre, par des groupes halogène ou alkoxy inférieur;
A est égal à -CH(R1)- a dans lequel R1 est hydrogène ou alkyle inférieur;
a est égal à deux, trois ou quatre;
e est égal à un ou deux;
qui consiste

a) pour préparer les composés racémiques,

i) à faire réagir une amine racémique de formule (II) :

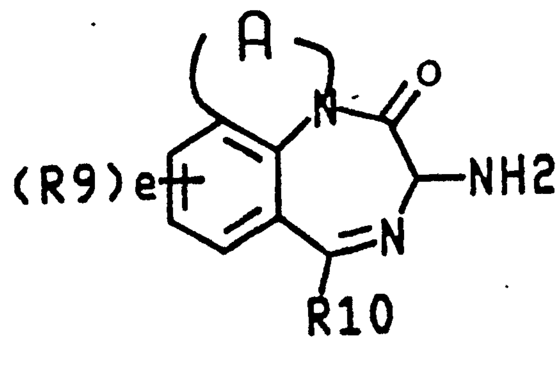

(II)

dans laquelle A, R9, R10 et e ont la signification définie à la revendication 1 avec un arylisocyanate de formule Ar-N = C = O, Ar ayant la signification définie à la revendication 1 pour obtenir une benzodiazépine racémique (Ia), de formule (I) dans laquelle R11 est l'hydrogène, R13 est NH et R12 est l'oxygène,

ii) à faire réagir une amine racémique (II) dans laquelle A, R9, R10 et e ont la signification définie à la revendication 1 avec un agent de protection des amines de formule (XII) : R25-CH2X, dans laquelle R25 est un benzène éventuellement mono di ou tri substitué par des substituants identiques ou différents choisis parmi les halogènes et le groupe nitro et X est un halogène ou un alkyl ou arylsulfonate, ou encore avec un agent (XIIa) R25-CHO en présence dans ce dernier cas de borohydrure de sodium, de cyanoborohydrure de sodium ou d'hydrogène sur catalyseur pour obtenir la benzylamine de formule (XIX)

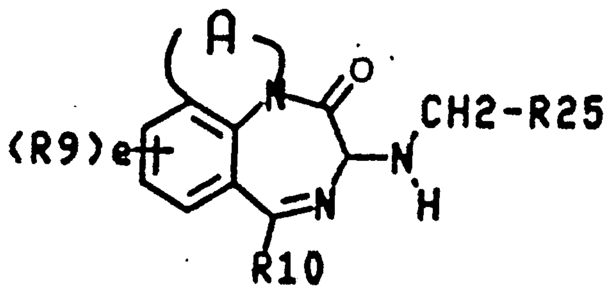

(XIX)

qui est mise à réagir avec un arylisocyanate de formule Ar-N = C = O, Ar ayant la signification définie à la revendication 1 pour obtenir l'urée de formule (XV) :

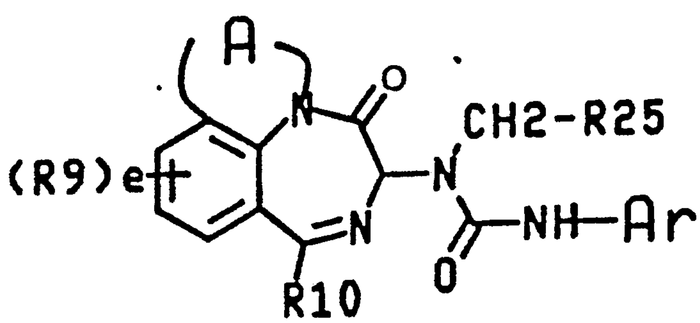

(XV)

qui est mise à réagir avec un réactif d'alkylation de formule (XIV) : R24-R39 dans lequel R24 a la signification définie à la revendication 1 et R39 est un halogène ou un pseudo halogène pour obtenir une urée tétrasubstituée de formule (XVI),

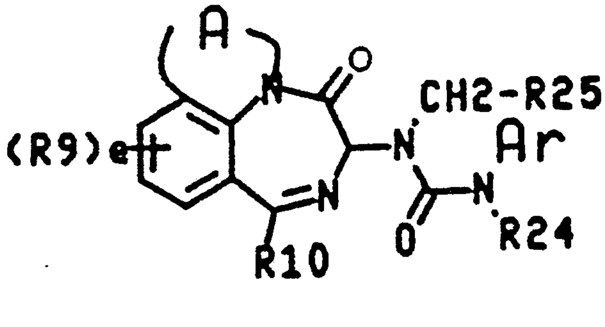

(XVI)

puis à éliminer par hydrogènolyse le groupe protecteur R25-CH2 avec un réactif choisi parmi le sodium dans l'ammoniac liquide ou l'hydrogène en présence d'un catalyseur d'hydrogènation comme par exemple un metal finement divisé sur un support comme du carbone pour obtenir une benzodiazépine racémique (Ib), de formule (I) dans laquelle R11 est l'hydrogène, R13 est N(R24), R24 étant alkyle inférieur et R12 est l'oxygène,

iii) à faire réagir une amine racémique (II) dans laquelle A, R9, R10, e ont la signification définie ci-dessus un agent d'alkylation de formule (XIII) : R11-R36 dans laquelle R11 est alkyle inférieur et R36 est un halogène ou un alkyl ou arylsulfonate, ou avec un aldéhyde de formule (XIIIa) R37-CHO dans laquelle R37 est l'hydrogène ou alkyle inférieur tel que (R37-CH2-) = (R11-) en présence d'un agent de réduction tel qu'un hydrure de bore ou l'hydrogène en présence d'un catalyseur pour obtenir l'amine de formule (IIa) :

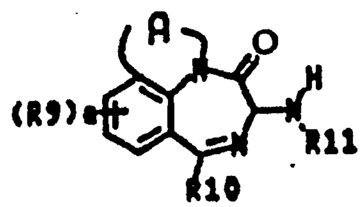

(IIa)

R11 different de l'hydrogene

que l'on fait réagir avec un arylisocyanate de formule Ar-N = C = O, pour obtenir une benzodiazépine racémique (Ic), de formule (I) dans laquelle R11 est alkyle inférieur, R13 est NH et R12 est l'oxygène.

iv) à faire réagir une benzodiazépine racémique de formule (Ic) dans laquelle A, R9, R10, R11, Ar et e ont la signification définie à la revendication 1 avec un réactif d'alkylation (XIV) défini en ii) de la présente revendication pour obtenir une benzodiazépine racémique (Id), de formule (I) dans laquelle R11 est alkyle inférieur, R13 est N-R24, R24 étant alkyle inférieur, et R12 est l'oxygène.

v) à faire réagir une amine racémique (II) dans laquelle A, R9, R10 et e ont la signification à la revendication 1 avec un arylisothiocyanate de formule Ar-N = C = S, Ar ayant la signification définie à la revendication 1 pour obtenir une benzodiazépine racémique (Ie), de formule (I) dans laquelle R11 est l'hydrogène, R13 est NH et R12 est le soufre,

vi) à faire réagir une benzylamine racémique de formule (XIX) précédemment définie avec un arylisothiocyanate de formule Ar-N = C = S, pour obtenir la thiourée de formule (XVII) :

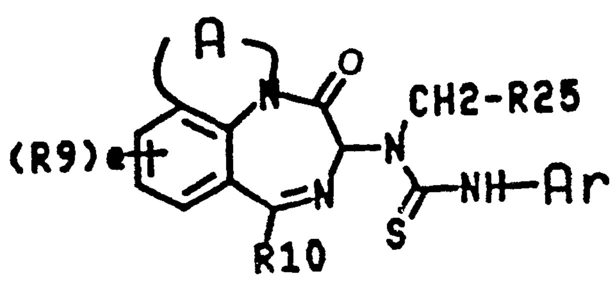

(XVII)

qui est mise à réagir avec un réactif d'alkylation (XIV) pour obtenir une thiourée tétrasubstituée de formule (XVIII) :

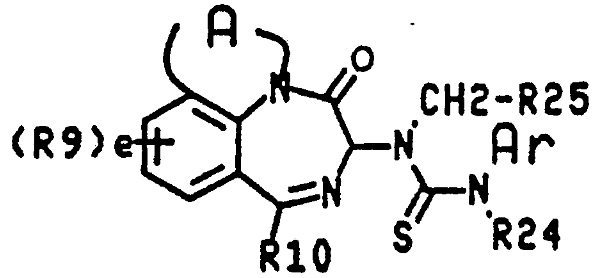

(XVIII)

puis à éliminer par hydrogènolyse le groupe protecteur R25-CH2 par réaction avec le sodium dans l'ammoniac liquide pour obtenir une benzodiazépine racémique (If), de formule (I) dans laquelle R11 est l'hydrogène, R13 est N(R24), R24 étant alkyle inférieur et R12 est le soufre.

vii) à faire réagir une amine racémique (IIa) dans laquelle A, R9, R10, R11 et e ont la signification définie à la revendication 1 avec un arylisothiocyanate de formule Ar-N = C = S, Ar ayant la signification définie à la revendication 1 pour obtenir une benzodiazépine racémique (Ig), de formule (I) dans laquelle R11 est alkyle inférieur, R13 est NH et R12 est le soufre,

viii) à faire réagir une benzodiazépine racémique (Ig) telle que décrite co-dessus avec un réactif d'alkylation (XIV), pour obtenir une benzodiazépine racémique (Ih), de formule (I) dans laquelle R11 est alkyle inférieur, R13 est N-R24, R24 étant alkyle inférieur et R12 est le soufre,

b) pour préparer les formes énantiomères à opérer à partir des énantiomères (II) appropriés et de manière identique à celle décrite dans chacun des huit cas précédents pour obtenir les formes énantiomères des benzodiazépines de formules (Ia), (Ib), (Ic), (Id), (Ie), (If), (Ig) et (Ih).

2. Procédé suivant la revendiation 1 caractérisées en ce que R12 représente un atome de soufre.

3. Procédé suivant la revendication 1 ou 2 caractérisées en ce que le groupe Ar ne comporte qu'un cycle.

4. Procédé suivant l'une des revendications 1 ou 3 caractérisées en ce que le groupe Ar est un groupe phényle substitué par un groupe méthyle.

5. Procédé suivant l'une des revendications 2 ou 3 caractérisées en ce que le groupe Ar est un groupe phényle substitué par un groupe méthyle ou un atome de brome.

6. Procédé suivant l'une des revendications 1 à 5 caractérisées en ce que le groupe A est un pont éthylène (-CH2-CH2-) ou triméthylène (-CH2-CH2-CH2-).

7. Procédé suivant l'une des revendications 1 à 6 caractérisées en ce que le groupe R9 est un atome d'hydrogène ou un atome de chlore en position para par rapport à l'atome d'azote commun au cycle diazépine et à l'autre hétérocycle azoté condensé avec le cycle diazépine et en ce que e = 1.

8. Procédé suivant l'une des revendications 1 à 7 caractérisées en ce sue le groupe R10 est un groupe phènyle, un groupe phènyle mono-substitué par un atome de fluor eb position ortho ou par un radical méthyle en position para par rapport à l'atome de carbone reliant le cycle phényle au cycle diazépine.

9. Procédé suivant l'une des revendications 1 à 8 caractérisées en ce que le groupe R11 est un atome d'hydrogène.

10. Procédé suivant l'une des revendications 1 à 9 caractérisées en ce que le groupe R13 est un groupe -NH-.

11. Procédé suivant l'une des revendications 1 à 10 caractérisées en ce que l'atome de carbone asymétrique du cycle benzodiazépine possède la configuration absolue R selon la nomenclature de Cahn, Ingold et Prelog.

12. Procédé suivant l'une des revendications 1 à 10 caractérisées en ce que l'atome de carbone asymétrique du cycle benzodiazépine possède la configuration absolue S selon la nomenclature de Cahn, Ingold et Prelog.

13. Procédé suivant la revendication 1 en ce qu'il consiste à préparer :

la (4R,S)-N-(céto-3-phényl-6-tétrahydro-1,2,3,4-pyrrolo[3,2,1-j,k]benzodiazépin-[1,4]-yl-4)-N′-(méthyl-3-phényl-)-urée et son isomère (5R).

la (5R,S)-N-(céto-4-phényl-7-hexahydro-1,2,3,3a,4,5-pyrido[3,2,1-j,k]benzodiazépin-[1,4]-yl-5)-N′-méthyl-3-phényl-)-urée et son isomère (5R).

la (5R,S)-N-(céto-4-phényl-7-hexahydro-1,2,3,3a,4,5-pyrido[3,2,1-j,k]benzodiazépin-[1,4]-yl-5)-N′-(nitro-4-phényl-)-urée ou

la (5R,S)-N-(céto-4-phényl-7-hexahydro-1,2,3,3a,4,5-pyrido[3,2,1-j,k]benzodiazépin-[1,4]-yl-5)-N′-(bromo-4-phényl-)-thiourée et ses isomères optiques et son isomère (5R).

14. L'utilisation d'une benzodiazépine suivant les revendications 1 à 13 pour lutter principalement contre les troubles provoqués par un dérèglement des mécanismes psychologiques contrôlés par la cholecystokinine ou la gastrine, chez les mammifères ou chez l'homme.

15. L'utilisation d'une benzodiazépine suivant les revendications 1 à 13 pour lutter principalement contre les troubles provoqués par un dérèglement des mécanismes psychologiques contrôlés par les récepteurs B de la cholecystokinine, en particulier, les troubles de l'appétit, la douleur, et éventuellement contre les troubles du système nerveux central; sans provoquer d'effets secondaires dépendants des récepteurs A de la cholecystokinine.

16. L'utilisation d'une benzodiazépine suivant les revendications 1 à 13 pour lutter principalement contre les troubles provoqués par un dérèglement des mécanismes psychologiques contrôlés par la gastrine, en particulier le syndrome de Zollinger-Ellison, les troubles de la sécrétion gastrique, et plus particulièrement les ulcères gastro-duodénaux et les gastro-duodénites, sans provoquer d'effets secondaires dépendants des récepteurs A de la cholecystokinine.

Revendications pour l'Etat contractant suivant: GR

1. Benzodiazépines de formule (I) :

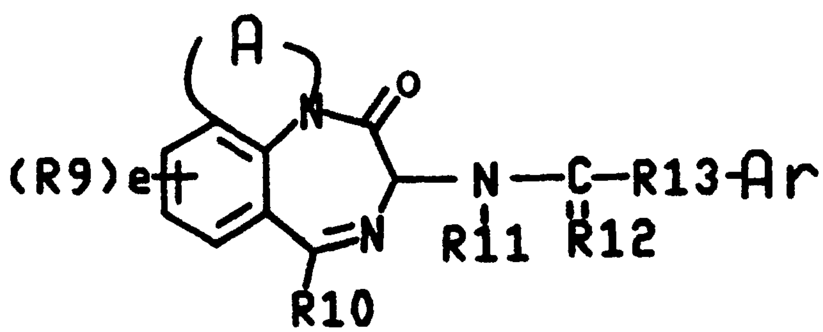

(I)

Dans laquelle,
R9 est hydrogène, nitro, halogène, cyano, trifluorométhyle, hydroxy, alkyle inférieur, alkoxy inférieur, -COOR20, -N(R21)-R22 ;
R10 est un phényle éventuellement mono à tri substitué par des groupes identiques ou différents choisis parmi halogène, alkyle inférieur, alkoxy inférieur, cyano, nitro ou trifluorométhyle ;
R11 est hydrogène ou alkyle inférieur ;
R12 est un atome d'oxygéne ou un atome de soufre ;
R13 est un groupe -N(R24)- ;
R20 est hydrogène ou alkyle inférieur ;
R21 et R22 sont identiques ou différents et peuvent être l'hydrogène ou un groupe alkyle ;
R24 est hydrogène ou alkyle inférieur ;
Ar est un groupe aromatique hydrocarboné ou hétéroaromatique comportant un cycle ou deux cycles condensés, chacun des cycles comportant de 5 à 7 atomes parmi lesquels figurent de zéro à deux hétéroatomes d'azote; ce groupe aromatique ou hétéro aromatique étant éventuellement mono ou di substitué par des groupes identiques ou différents choisis parmi alkyle inférieur, carboxyle, alkoxycarbonyle inférieur, sulfonyle, sulfamoyle, cyano, nitro, trifluorométhyle ou, sous réserve que R12 soit le soufre, par des groupes halogène ou alkoxy inférieur ;
A est égal à [-CH(R1)-]a dans lequel R1 est hydrogène ou alkyle inférieur ;
a est égal à deux, trois ou quatre ;
e est égal à un ou deux ;

2. Benzodiazépines suivant la revendication 1 caractérisées en ce que R12 représente un atome de soufre.

3. Benzodiazépines suivant la revendication 1 ou 2 caractérisées en ce que le groupe Ar ne comporte qu'un cycle.

4. Benzodiazépines suivant l'une des revendications 1 ou 3 caractérisées en ce que le groupe Ar est un

groupe phényle substitué par un groupe méthyle.

5. Benzodiazépines suivant l'une des revendications 2 ou 3 caractérisées en ce que le groupe Ar est un groupe phényle substitué par un groupe méthyle ou un atome de brome.

6. Benzodiazépines suivant l'une des revendications 1 à 5 caractérisées en ce que le groupe A est un pont éthylène (-CH2-CH2-) ou triméthylène (-CH2-CH2-CH2-).

7. Benzodiazépines suivant l'une des revendications 1 à 6 caractérisées en ce que le groupe R9 est un atome d'hydrogène ou un atome de chlore en position para par rapport à l'atome d'azote commun au cycle diazépine et à l'autre hétérocycle azoté condensé avec le cycle diazépine et en ce que e = 1.

8. Benzodiazépines suivant l'une des revendications 1 à 7 caractérisées en ce que le groupe R10 est un groupe phènyle, un groupe phènyle mono-substitué par un atome de fluor en position ortho ou par un radical méthyle en position para par rapport à l'atome de carbone reliant le cycle phényle au cycle diazépine.

9. Benzodiazépines suivant l'une des revendications 1 à 8 caractérisées en ce que le groupe R11 est un atome d'hydrogène.

10. Benzodiazépines suivant l'une des revendications 1 à 9 caractérisées en ce que le groupe R13 est un groupe -NH-.

11. Benzodiazépines suivant l'une des revendications 1 à 10 caractérisées en ce que l'atome de carbone asymétrique du cycle benzodiazèpine possède la configuration absolue R selon la nomenclature de Cahn, Ingold et Prelog.

12. Benzodiazépines suivant l'une des revendications 1 à 10 caractérisées en ce que l'atome de carbone asymétrique du cycle benzodiazèpine possède la configuration absolue S selon la nomenclature de Cahn, Ingold et Prelog.

13. Benzodiazépines suivant la revendication 1 et qui sont :

La (4R,S)-N-(céto-3-phényl-6-tétrahydro-1,2,3,4-pyrrolo[3,2,1-j,k]benzodiazépin-[1,4]-yl-4)-N′-(méthyl-3-phényl-)-urée et son isomère (5R).

La (5R,S)-N-(céto-4-phényl-7-hexahydro-1,2,3,3a,4,5-pyrido[3,2,1-j,k]benzodiazépin-[1,4]-yl-5)-N′-(méthyl-3-phényl-)-urée et son isomère (5R).

La (5R,S)-N-(céto-4-phényl-7-hexahydro-1,2,3,3a,4,5-pyrido[3,2,1-j,k]benzodiazépin-[1,4]-yl-5)-N′-(nitro-4-phényl-)-urée.

La (5R,S)-N-(céto-4-phényl-7-hexahydro-1,2,3,3a,4,5-pyrido[3,2,1,-j,k]benzodiazépin-[1,4]-yl-5)-N′-(bromo-4-phényl-)-thiourée et ses isomères optiques et son isomère (5R).

14. Procédé de préparation des benzodiazépines de formule (I), définie à la revendication 1 qui consiste

a) pour préparer les composés racémiques,

i) à faire réagir une amine racémique de formule (II) :

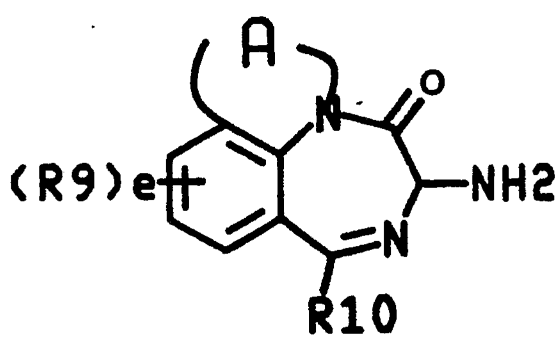

(II)

dans laquelle A, R9, R10 et e ont la signification définie à la revendication 1 avec un arylisocyanate de formule Ar-N=C=O, Ar ayant la signification définie à la revendication 1 pour obtenir une benzodiazépine racémique (Ia), de formule (I) dans laquelle R11 est l'hydrogène, R13 est NH et R12 est l'oxygène,

ii) à faire réagir une amine racémique (II) dans laquelle A, R9, R10 et e ont la signification définie à la revendication 1 avec un agent de protection des amines de formule (XII) : R25-CH2X, dans laquelle R25 est un benzène éventuellement mono di ou tri substitué par des substituants identiques ou différents choisis parmi les halogènes et le groupe nitro et X est un halogène ou un alkyl ou arylsulfonate, ou encore avec un agent (XIIa) R25-CHO en présence dans ce dernier cas de borohydrure de sodium, de cyanoborohydrure de sodium ou d'hydrogène sur catalyseur pour obtenir la benzylamine de formule (XIX)

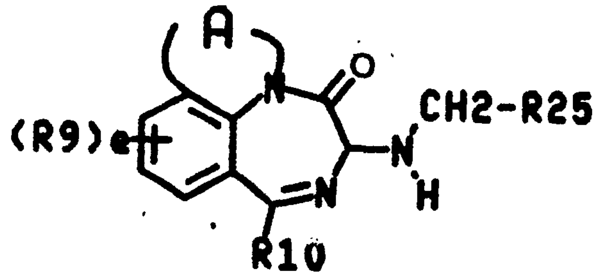

(XIX)

qui est mise à réagir avec un arylisocyanate de formule Ar-N = C = O, Ar ayant la signification définie à la revendication 1 pour obtenir l'urée de formule (XV) :

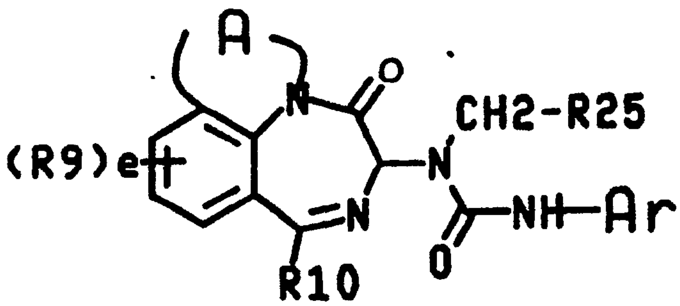

(XV)

qui est mise à réagir avec un réactif d'alkylation de formule (XIV) : R24-R39 dans lequel R24 a la signification définie à la revendication 1 et R39 est un halogène ou un pseudo halogène pour obtenir une urée tétrasubstituée de formule (XVI),

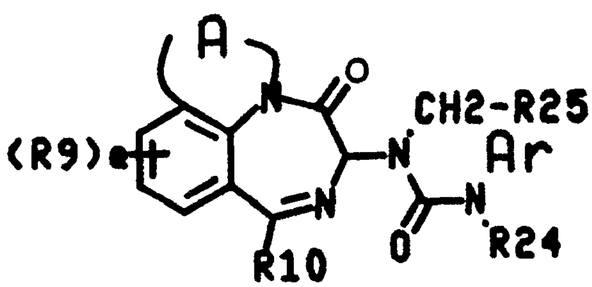

(XVI)

puis à éliminer par hydrogènolyse le groupe protecteur R25-CH2 avec un réactif choisi parmi le sodium dans l'ammoniac liquide ou l'hydrogène en présence d'un catalyseur d'hydrogènation comme par exemple un metal finement divisé sur un support comme du carbone pour obtenir une benzodiazépine racémique (Ib), de formule (I) dans laquelle R11 est l'hydrogène, R13 est N(R24), R24 étant alkyle inférieur et R12 est l'oxygène,

iii) à faire réagir une amine racémique (II) dans laquelle A, R9, R10, e ont la signification définie ci-dessus un agent d'alkylation de formule (XIII) : R11-R36 dans laquelle R11 est alkyle inférieur et R36 est un halogène ou un alkyl ou arylsulfonate, ou avec un aldéhyde de formule (XIIIa) R37-CHO dans laquelle R37 est l'hydrogène ou alkyle inférieur tel que (R37-CH2-) = (R11-) en présence d'un agent de réduction tel qu'un hydrure de bore ou l'hydrogène en présence d'un catalyseur pour obtenir l'amine de formule (IIa) :

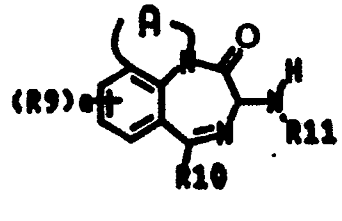

(IIa)

R11 différent de l'hydrogene

que l'on fait réagir avec un arylisocyanate de formule Ar-N = C = O, pour obtenir une benzodiazépine racémique (Ic), de formule (I) dans laquelle R11 est alkyle inférieur, R13 est NH et R12 est l'oxygène.

iv) à faire réagir une benzodiazépine racémique de formule (Ic) dans laquelle A, R9, R10, R11, Ar et e ont la signification définie à la revendication 1 avec un réactif d'alkylation (XIV) défini en ii) de la présente revendication pour obtenir une benzodiazépine racémique (Id), de formule (I) dans laquelle R11 est alkyle inférieur, R13 est N-R24, R24 étant alkyle inférieur, et R12 est l'oxygène,

v) à faire réagir une amine racémique (II) dans laquelle A, R9, R10 et e ont la signification à la revendication 1 avec un arylisothiocyanate de formule Ar-N = C = S, Ar ayant la signification définie à la revendication 1 pour obtenir une benzodiazépine racémique (Ie), de formule (I) dans laquelle R11 est l'hydrogène, R13 est NH et R12 est le soufre,

vi) à faire réagir une benzylamine racémique de formule (XIX) précédemment définie avec un arylisothiocyanate de formule Ar-N = C = S, pour obtenir la thiourée de formule (XVII) :

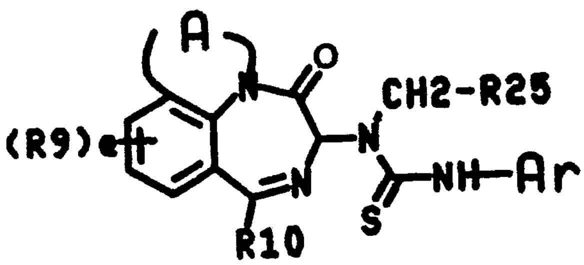

(XVII)

qui est mise à réagir avec un réactif d'alkylation (XIV) pour obtenir une thiourée tétrasubstituée de formule (XVIII) :

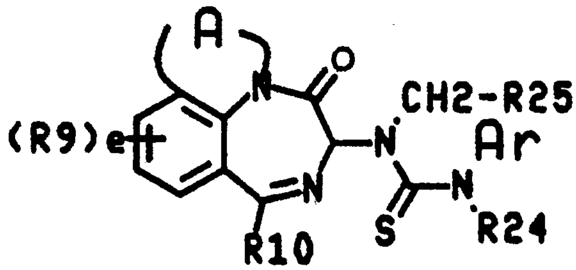

(XVIII)

puis à éliminer par hydrogènolyse le groupe protecteur R25-CH2 par réaction avec le sodium dans l'ammoniac liquide pour obtenir une benzodiazépine racémique (If), de formule (I) dans laquelle R11 est l'hydrogène, R13 est N(R24), R24 étant alkyle inférieur et R12 est le soufre.

vii) à faire réagir une amine racémique (IIa) dans laquelle A, R9, R10, R11 et e ont la signification définie à la revendication 1 avec un arylisothiocyanate de formule Ar-N = C = S, Ar ayant la signification

définie à la revendication 1 pour obtenir une benzodiazépine racémique (Ig), de formule (I) dans laquelle R11 est alkyle inférieur, R13 est NH et R12 est le soufre,

viii) à faire réagir une benzodiazépine racémique (Ig) telle que décrite co-dessus avec un réactif d'alkylation (XIV), pour obtenir une benzodiazépine racémique (Ih), de formule (I) dans laquelle R11 est alkyle inférieur, R13 est N-R24, R24 étant alkyle inférieur et R12 est le soufre,

b) pour préparer les formes énantiomères, à opérer à partir des énantiomères (II) appropriés et de manière identique à celle décrite dans chacun des huit cas précédents pour obtenir les formes énantiomères des benzodiazépines de formules (Ia), (Ib), (Ic), (Id), (Ie), (If), (Ig) et (Ih).

15. L'utilisation d'une benzodiazépine suivant les revendications 1 à 13 pour lutter principalement contre les troubles provoqués par un dérèglement des mécanismes psychologiques contrôlés par la cholecystokinine ou la gastrine, chez les mammifères ou chez l'homme.

16. L'utilisation d'une benzodiazépine suivant les revendications 1 à 13 pour lutter principalement contre les troubles provoqués par un dérèglement des mécanismes psychologiques contrôlés par les récepteurs B de la cholecystokinine, en particulier, les troubles de l'appétit, la douleur, et éventuellement contre les troubles du système nerveux central; sans provoquer d'effets secondaires dépendants des récepteurs A de la cholecystokinine.

17. L'utilisation d'une benzodiazépine suivant les revendications 1 à 13 pour lutter principalement contre les troubles provoqués par un dérèglement des mécanismes psychologiques contrôlés par la gastrine, en particulier le syndrome de Zollinger-Ellison, les troubles de la sécrétion gastrique, et plus particulièrement les ulcères gastro-duodénaux et les gastro-duodénites, sans provoquer d'effets secondaires dépendants des récepteurs A de la cholecystokinine.